# EUROPEAN PATENT APPLICATION

(11) **EP 4 393 959 A1**
(43) Date of publication of application: **03.07.2024**
(21) Application number: 22861413.7
(22) Date of filing: 24.08.2022
(51) Int. Cl.: C07K 19/00, A61K 38/10, A61K 39/395, A61K 47/68, A61P 9/10, A61P 17/02, A61P 25/00, A61P 25/14, A61P 25/16, A61P 25/18, A61P 25/24, A61P 25/28, A61P 31/04, A61P 31/12, A61P 35/00, C07K 7/08, C07K 16/00, C07K 16/18, C12N 15/62

(54) **HUMAN TRANSFERRIN RECEPTOR-BINDING ANTIBODY-PEPTIDE CONJUGATE**

(30) Priority: 24.08.2021 JP 2021136682
(71) Applicant: PeptiDream Inc., Kawasaki-shi, Kanagawa 210-0821 (JP); JCR Pharmaceuticals Co., Ltd., Ashiya-shi, Hyogo 659-0021 (JP)
(72) Inventor: TAKAHASHI Kenichi, Kobe-shi, Hyogo 651-2241 (JP); YODEN Eiji, Kobe-shi, Hyogo 651-2241 (JP); HASHIMOTO Hidehiko, Kobe-shi, Hyogo 651-2241 (JP); FUJIYAMA Saki, Kobe-shi, Hyogo 651-2241 (JP); OHUCHI Masaki, Kawasaki-shi, Kanagawa 210-0821 (JP); SAWAI Naoki, Kawasaki-shi, Kanagawa 210-0821 (JP); INABA Shinnosuke, Kawasaki-shi, Kanagawa 210-0821 (JP)
(74) Representative: van Dam, Vincent
(86) International application number: PCT/JP2022/031933
(87) International publication number: WO 2023/027125

(57) **Abstract**

The technic to pass through the blood-brain barrier is provided. A conjugate comprising:
(1) a peptide that binds to a transferrin receptor, wherein the peptide is:
(i) a peptide comprising 1st to 15th amino acid sequence (Ala-Val-Phe-Val-Trp-Asn-Tyr-Tyr-Ile-Ile-Arg-Arg-Tyr-MeY-Cys) of an amino acid sequence described in SEQ ID NO: 1;
(ii) a peptide comprising an amino acid sequence having substitution, deletion, addition and/or insertion of 1 to 11 amino acid residues in the 1st to 15th amino acid sequence of the amino acid sequence described in SEQ ID NO: 1;
(iii) a peptide comprising 1st to 12th amino acid sequence (Ala-Val-Phe-Val-Trp-Asn-Tyr-Tyr-Ile-Ile-Ser-Cys) of an amino acid sequence described in SEQ ID NO: 14; or
(iv) a peptide comprising an amino acid sequence having substitution, deletion, addition and/or insertion of 1 to 8 amino acid residues in the 1st to 10th amino acid sequence of the amino acid sequence described in SEQ ID NO: 14,
and
(2) a compound comprising an antibody or an antigen-binding fragment thereof.

## Description

### Technical Field

The present invention relates to a conjugate (hereinafter also referred to as a complex) comprising a peptide capable of binding to the human transferrin receptor (hTfR) and an antibody or an antigen-binding fragment thereof. The present invention also relates to a conjugate of a peptide having cell permeability and an antibody or antigen-binding fragment thereof. Furthermore, the invention relates to pharmaceuticals comprising such conjugate.

### Background of the Invention

All over the world, there is a great need for the development of therapeutic drugs for brain-related diseases such as Alzheimer's disease and brain tumors, and research and development are progressing. In addition to conventional small molecule drugs, antibody drugs, which are macromolecules, have recently become increasingly important. However, research and development are fraught with difficulties. One of the reasons for this is the existence of the blood-brain barrier.

In capillaries that supply blood to most tissues of the brain, except for some areas including the circumventricular organs (pineal gland, pituitary gland, area postrema, etc.), unlike capillaries in muscles and other tissues, the endothelial cells that form their endothelium are tightly attached to each other by strong intercellular junctions. Therefore, passive transport of substances from the blood to the brain is prevented, and although there are exceptions, substances other than highly fat-soluble substances or substances with small molecular weight (less than 200 to 500 Daltons) and electrically neutral near physiological pH are difficult to be transported from the capillaries to the brain. Such a mechanism that restricts the exchange of substances between blood and brain tissue fluid via the capillary endothelium in the brain is called the blood-brain barrier (BBB). The blood-brain barrier also restricts the exchange of substances between blood and tissue fluids of the central nervous system, including the brain and spinal cord, as well as the brain. The presence of the blood-brain barrier allows most of the cells of the central nervous system to maintain their biochemical homeostasis without being affected by fluctuations in the concentrations of hormones, lymphokines and other substances in the blood.

As a method to allow macromolecular substances to reach the brain through the blood-brain barrier, various methods have been reported to modify the macromolecular substances so that they have affinity with a transferrin receptor, which are membrane proteins existing on endothelial cells of capillaries in the brain (Patent Documents 1 to 3). For example, as Patent Document 1 describes a blood-brain barrier shuttle that has affinity to the transferrin receptor and can bind to the receptor.

However, there is a need for further methods that allow macromolecular substances, especially antibodies, to pass through the blood-brain barrier.

### Prior Art Documents

### Patent Documents

[Patent Document 1] JP 2015-528452 A (Translation of PCT Application)
[Patent Document 2] JP H06-228199 A
[Patent Document 3] WO2016/208695 A
[Patent Document 4] WO2019/151539 A

### Summary of the Invention

### Problems to be Solved by the Invention

An objective of the present invention is to provide a conjugate of a peptide that binds to the transferrin receptor and an antibody. The conjugate has both transferrin receptor binding ability and binding ability to the antigen to which the antibody constructing the conjugate binds.

Also an object of the present invention is to provide a technique for passing through the blood-brain barrier by conjugating a peptide that binds to a transferrin receptor and has the ability to pass through the blood-brain barrier with an antibody or antigen-binding fragment thereof. More specifically, an object of the present invention is to provide a conjugate of peptide, which bind to transferrin receptor and has the ability to pass through the blood-brain barrier, with antibodies.

Furthermore, an object of the present invention is to provide a pharmaceutical containing the above conjugate.

### Solution to Problem

The invention was completed after discovering that a conjugate with antigen binding ability and transferrin receptor binding ability can be created by combining a peptide having a specific structure with a compound containing an antibody, either directly or via a linker, and a desired antibody can be passed through the blood-brain barrier and introduced into cells by using the conjugate.

A certain invention described in this specification relates to a conjugate containing a peptide that binds to the transferrin receptor and an antibody or an antigen-binding fragment thereof.

The above peptide is a peptide that binds to a transferrin receptor, wherein the peptide is:
(i) a peptide comprising 1st to 15th amino acid sequence (Ala-Val-Phe-Val-Trp-Asn-Tyr-Tyr-Ile-Ile-Arg-Arg-Tyr-MeY-Cys) of an amino acid sequence described in SEQ ID NO: 1;
(ii) a peptide comprising an amino acid sequence having substitution, deletion, addition and/or insertion of 1 to 11 amino acid residues in the 1 st to 15th amino acid sequence of the amino acid sequence described in SEQ ID NO: 1;
(iii) a peptide comprising 1st to 12th amino acid sequence (Ala-Val-Phe-Val-Trp-Asn-Tyr-Tyr-Ile-Ile-Ser-Cys) of an amino acid sequence described in SEQ ID NO: 14; or
(iv) a peptide comprising an amino acid sequence having substitution, deletion, addition and/or insertion of 1 to 8 amino acid residues in the 1 st to 10th amino acid sequence of the amino acid sequence described in SEQ ID NO: 14.

And a compound comprising the antibody or the antigen-binding fragment thereof may be IgG or an IgG-derived antigen-binding fragment, or an antibody or an antigen-binding fragment thereof selected from the group consisting of IgG1, IgG2 and IgG4.

Another example of the above-mentioned peptide is a peptide comprising an amino acid sequence containing one or more substitutions selected from:
(I) substitution of the 1st alanine residue of SEQ ID NO: 1 for an aliphatic amino acid or a methylated aliphatic amino acid;
(II) substitution of the 2nd amino acid residue of SEQ ID NO: 1 for any amino acid residue or any N-methylamino acid;
(III) substitution of the 3rd amino acid residue of SEQ ID NO:1 for an aromatic amino acid residue, a methylated aromatic amino acid residue, or an amino acid residue having an aromatic ring in a side chain;
(IV) substitution of the 5th amino acid residue of SEQ ID NO:1 for an aromatic amino acid residue, a methylated aromatic amino acid residue, or an amino acid residue having an aromatic ring in a side chain;
(V) substitution of the 6th asparagine residue of SEQ ID NO:1 for a hydrophilic amino acid or alanine;
(VI) substitution of the 8th tyrosine residue of SEQ ID NO:1 for an aromatic amino acid residue, a methylated aromatic amino acid residue, or an amino acid residue having an aromatic ring in a side chain;
(VII) substitution of the 10th isoleucine residue of SEQ ID NO:1 for any amino acid;
(VIII) substitution of the 11th arginine residue of SEQ ID NO:1 for any amino acid;
(IX) substitution of the 12th arginine residue of SEQ ID NO:1 for any amino acid; and
(X) substitution of the 13th tyrosine residue of SEQ ID NO:1 for any amino acid;
(XI) substitution of the 14th N-methyltyrosine residue of SEQ ID NO:1 for any amino acid;
or a peptide containing one or more substitutions selected from:
(I) substitution of the 1st alanine residue of SEQ ID NO: 14 for an aliphatic amino acid or a methylated aliphatic amino acid;
(II) substitution of the 2nd amino acid residue of SEQ ID NO: 14 for any amino acid residue or any N-methylamino acid;
(III) substitution of the 3rd amino acid residue of SEQ ID NO: 14 for an aromatic amino acid residue, a methylated aromatic amino acid residue, or an amino acid residue having an aromatic ring in a side chain;
(IV) substitution of the 5th amino acid residue of SEQ ID NO: 14 for an aromatic amino acid residue, a methylated aromatic amino acid residue, or an amino acid residue having an aromatic ring in a side chain;
(V) substitution of the 6th asparagine residue of SEQ ID NO: 14 for a hydrophilic amino acid or alanine;
(VI) substitution of the 8th tyrosine residue of SEQ ID NO: 14 for an aromatic amino acid residue, a methylated aromatic amino acid residue, or an amino acid residue having an aromatic ring in a side chain;
(VII) substitution of the 10th isoleucine residue of SEQ ID NO: 14 for any amino acid; and
(X) substitution of the 11th serine residue of SEQ ID NO: 14 for a hydrophilic amino acid residue.

More specifically, the peptide is a cyclic peptide consisting of any one of the amino acid sequences from the 1st to 15th amino acid sequence of SEQ ID NOs: 1 to 13, 15, 18 to 86, 90 to 110, and from the 1st to 12th amino acid sequence of SEQ ID NOs: 14, 16, 17, 87 to 89.

In the conjugate of the present invention, preferably, the peptide is bound to the antibody via a linker.

And preferably, the above linker is a peptide linker, a chemical linker, or a combination thereof.

The specific linker is a linker comprising a sequence described in any one of SEQ ID NOs: 111 to 161.

Preferably, the conjugate is one in which the peptide is bound to the antibody via a maleimide, hydrazide or NHS part attached to the end of the linker. Preferably, the conjugate is one in which a linker-attached peptide of any one of SEQ ID NOs: 1 to 110 is bound to an antibody or antigen-binding fragment thereof.

This specification also provides a composition containing any one of the above-described conjugates for delivering the conjugate into a cell or passing through the blood-brain barrier. The composition may include various elements described in this specification in addition to the conjugate.

This specification also provides a pharmaceutical composition containing the conjugate or the salt thereof described in any one of the above claims as an active ingredient. The composition may contain various elements described in this specification in addition to the conjugate or salt thereof. The salt thereof means a pharmaceutically acceptable salt of the conjugate.

This specification also discloses a processing method (production method of conjugate) that allows an antibody or an antigen-binding fragment thereof to be delivered into a cell or to passing through the blood-brain barrier. The method includes a step of conjugating a peptide that binds to the transferrin receptor described above with an antibody or an antigen-binding fragment thereof.

This specification also provides methods of treating various diseases, including the step of delivering the above conjugate or composition into a cell or passing through the blood-brain barrier.

### Advantageous Effects of the Invention

The invention described in this specification can provide the conjugate which comprises a peptide, which is the peptide that binds to the human transferrin receptor (hTfR) or the peptide with cell permeability and the like, and the antibody or the antigen-binding fragment thereof and the like, as demonstrated by the examples.

### Brief Description of Drawings

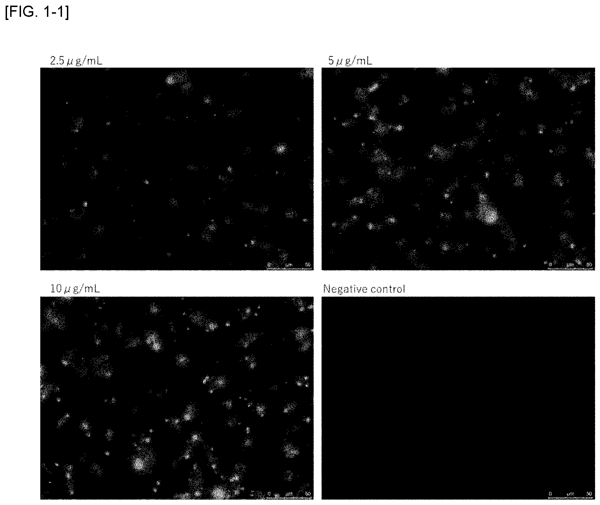

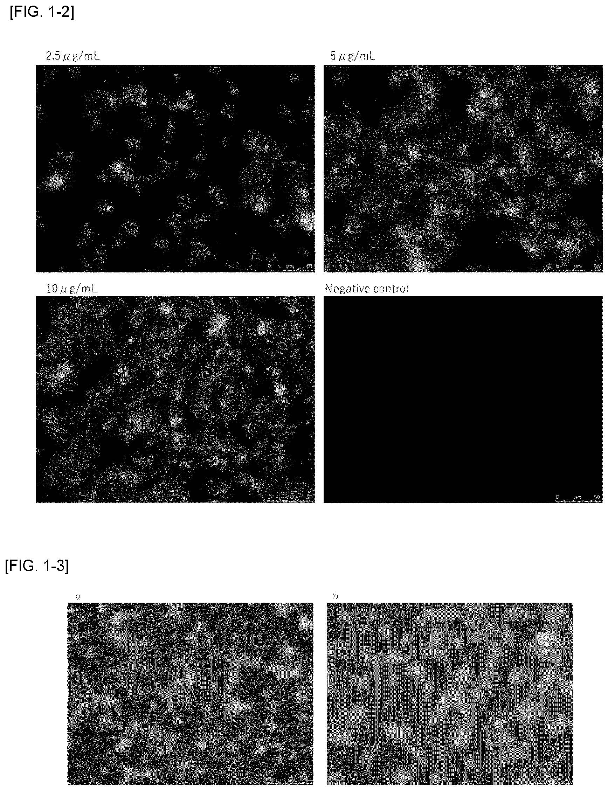

FIG. 1-1 is a photograph in lieu of a drawing showing a fluorescence intensity measurement in a cell treated with a trastuzumab-peptide conjugate.
FIG. 1-2 is a photograph in lieu of a drawing showing a fluorescence intensity measurement in a cell treated with the trastuzumab-peptide conjugate.
FIG. 1-3 is a photograph in lieu of a drawing showing a fluorescence intensity measurement in a cell treated with the trastuzumab-peptide conjugate.
FIG. 2-1 shows a concentration of the trastuzumab-peptide conjugate in plasma.
FIG. 2-2 shows a concentration of the trastuzumab-peptide conjugate in each tissue.

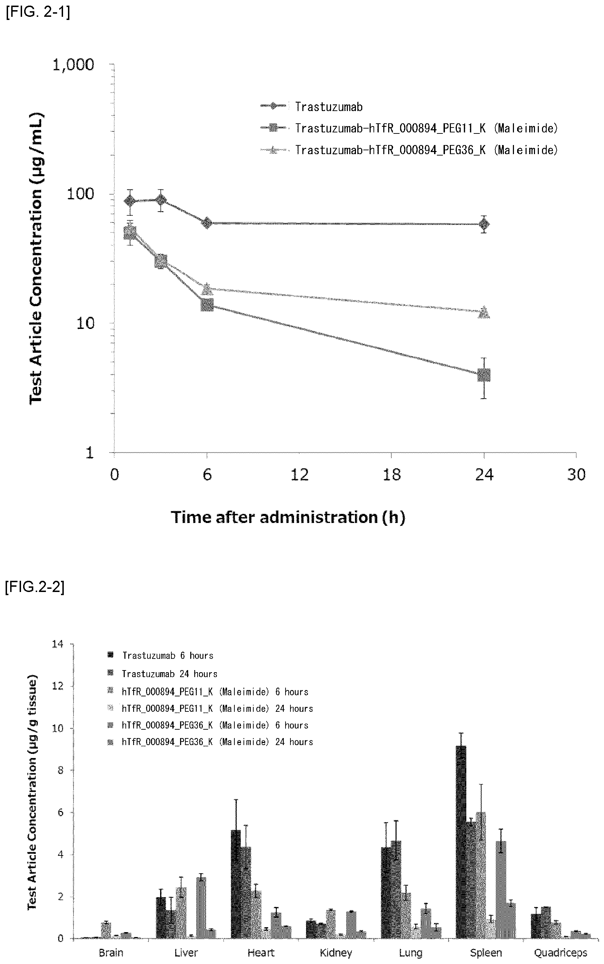

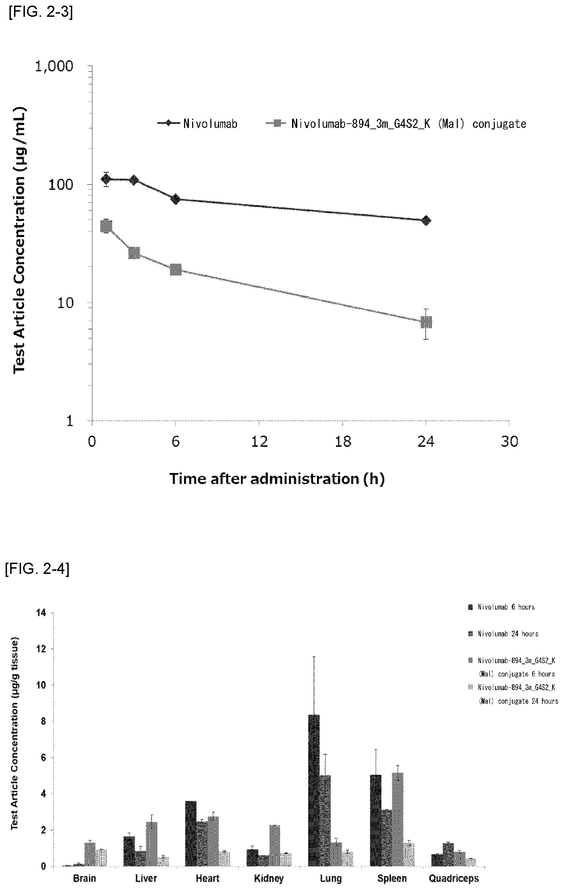

FIG. 2-3 shows a concentration of a nivolumab-peptide conjugate in plasma.
FIG. 2-4 shows a concentration of the nivolumab-peptide conjugate in each tissue.

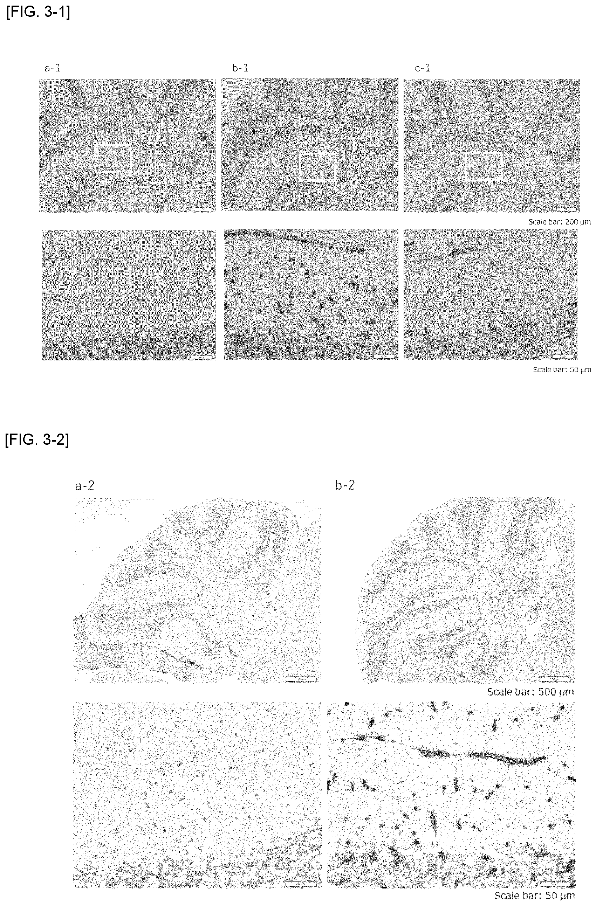

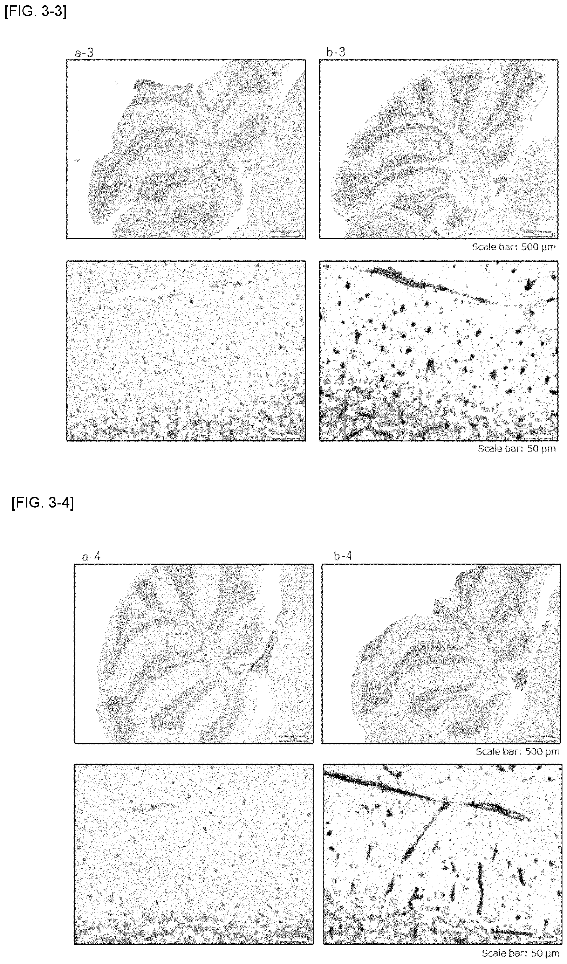

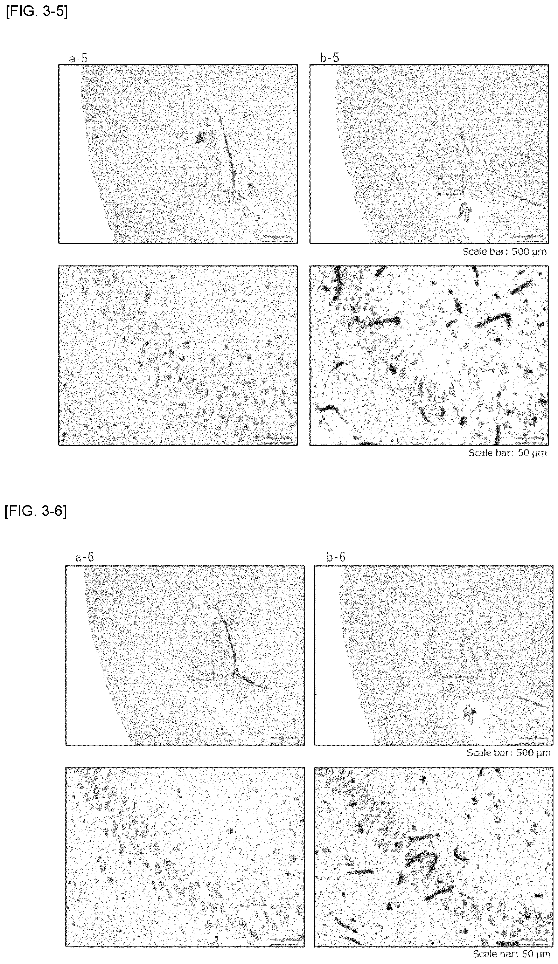

FIG. 3-1 is a photograph in lieu of a drawing showing results of a mouse brain localization confirmation test (cerebellum, 6 hours treatment), treated with trastuzumab-hTfR_000894_PEG11_K(Maleimide) and trastuzumab-hTfR_000894_PEG36_K(Maleimide).
FIG. 3-2 is a photograph in lieu of a drawing showing results of a mouse brain localization confirmation test (cerebellum, 6 hours treatment), treated with nivolumab-894_3m_G4S2_K(Mal).
FIG. 3-3 is a photograph in lieu of a drawing showing results of a mouse brain localization confirmation test (cerebellum, 6 hours treatment), treated with nivolumab-894_3m_GGRGRS_K(Mal).
FIG. 3-4 is a photograph in lieu of a drawing showing results of a mouse brain localization confirmation test (cerebellum, 24 hours treatment), treated with nivolumab-894_3m_GGRGRS_K(Mal).
FIG. 3-5 is a photograph in lieu of a drawing showing results of a mouse brain localization confirmation test (hippocampus, 6 hours treatment), treated with nivolumab-894_3m_GGRGRS_K(Mal).
FIG. 3-6 is a photograph in lieu of a drawing showing results of a mouse brain localization confirmation test (hippocampus, 24 hours treatment), treated with nivolumab-894_3m_GGRGRS_K(Mal).

### Detailed Description of Embodiments

The following is a description of embodiments for carrying out the present invention. The present invention is not limited to the embodiments described below, but also includes modifications made from the following embodiments as appropriate within the range obvious to those skilled in the art.

International patent application PCT/JP2021/006709 (International Publication NO. WO2021-167107 pamphlet; unpublished at the time of filing the basic application), whose applicant is fully identical to one of this application, is incorporated entirety herein by reference.

### Conjugate (Complex)

The conjugate of the present invention is a compound comprising:
(1) a peptide that binds to the transferrin receptor, wherein the peptide is:
   (i) a peptide comprising 1st to 15th amino acid sequence (Ala-Val-Phe-Val-Trp-Asn-Tyr-Tyr-Ile-Ile-Arg-Arg-Tyr-MeY-Cys) of an amino acid sequence described in SEQ ID NO: 1;
   (ii) a peptide comprising an amino acid sequence having substitution, deletion, addition and/or insertion of 1 to 11 amino acid residues in the 1st to 15th amino acid sequence of the amino acid sequence described in SEQ ID NO: 1;
   (iii) a peptide comprising 1st to 12th amino acid sequence (Ala-Val-Phe-Val-Trp-Asn-Tyr-Tyr-Ile-Ile-Ser-Cys) of an amino acid sequence described in SEQ ID NO: 14; or
   (iv) a peptide comprising an amino acid sequence having substitution, deletion, addition and/or insertion of 1 to 8 amino acid residues in the 1st to 10th amino acid sequence of the amino acid sequence described in SEQ ID NO: 14, and
(2) an antibody or antigen-binding fragment thereof.

Here, in the compound comprising the peptide and the antibody or the antigen-binding fragment thereof, they may be bound directly or via a linker, but preferably via a linker.

### Transferrin Receptor

The transferrin receptor is a receptor that binds to transferrin, a protein which is found in plasma and binds to an iron ion, and has the function of taking it into cells. Transferrin receptor is expressed on various cells such as reticulocytes, placental trophoblasts, and lymphocytes, and it has been suggested that transferrin receptors are particularly expressed on tumor cells. Since the transferrin receptor has the property of triggering cellular endocytosis by stimulating binding of iron ion in plasma, research is underway to use antibodies or the like that bind to the transferrin receptor as a drug delivery system to allow desired substances to pass through the BBB. Two types of transferrin receptors, type I and type II, are known, but type I (Gene ID: 7037) is preferred as the transferrin receptor in the present invention. In this specification, the human-type transferrin receptor is referred to as the human TfR, hTfR, or simply TfR, unless otherwise noted.

### Peptides That Bind to Transferrin Receptor

Binding to the transferrin receptor (also referred to as having binding activity or affinity) means binding specifically to the transferrin receptor.

Affinity is expressed by the equilibrium constant (KD) for dissociation between the transferrin receptor and the binding peptide, which is a measure of the binding strength between the transferrin receptor and the binding peptide: and as the value of KD decreases, the binding strength between the transferrin receptor and the binding peptide becomes stronger (alternatively, affinity can be expressed as an affinity constant (KA), which is 1/KD). As will be clear to those skilled in the art (e.g., based on further disclosure herein), affinity can be determined in a manner that is known per se, depending on the type and nature of the substance to be bound. Binding activity is also a measure of the strength of the binding between the transferrin receptor and the binding peptide. Binding activity is related to both the affinity between the transferrin receptor and its binding site on the binding peptide and the number of relevant binding sites present on the binding molecule.

Specific binding of the transferrin receptor and the binding peptide can be determined by any suitable method known per se, including, for example, surface plasmon resonance (SPR) assay, Scatchard analysis and/or competitive binding assays such as radioimmunoassay (RIA), enzyme immunoassay (EIA) and sandwich competition assay described herein, including different variants thereof that are known per se in the art. Preferably, the affinity of the peptide in the invention and the transferrin receptor may be less than 100 nM, preferably less than 50 nM, and, although it is not limited, it may be about 10⁻⁵ M to about 10⁻⁹ M, or in another manner, less than 10⁻⁷ M, for example, 10⁻⁷ M to 10⁻¹³ M, for example, 10⁻⁹ M to 10⁻¹³ M.

The peptide that binds to the transferrin receptor is a peptide that bind specifically to the transferrin receptors as described above, and the peptide is not limited as long as the peptide is:
(i) a peptide comprising 1st to 15th amino acid sequence (Ala-Val-Phe-Val-Trp-Asn-Tyr-Tyr-Ile-Ile-Arg-Arg-Tyr-MeY-Cys) of an amino acid sequence described in SEQ ID NO: 1;
(ii) a peptide comprising an amino acid sequence having substitution, deletion, addition and/or insertion of 1 to 11 amino acid residues in the 1 st to 15th amino acid sequence of the amino acid sequence described in SEQ ID NO: 1;
(iii) a peptide comprising 1st to 12th amino acid sequence (Ala-Val-Phe-Val-Trp-Asn-Tyr-Tyr-Ile-Ile-Ser-Cys) of an amino acid sequence described in SEQ ID NO: 14; or
(iv) a peptide comprising an amino acid sequence having substitution, deletion, addition and/or insertion of 1 to 8 amino acid residues in the 1 st to 10th amino acid sequence of the amino acid sequence described in SEQ ID NO: 14_{∘}

### Passability Through Blood-brain Barrier (BBB)

Passability through BBB means, for example, that the substance can pass through the BBB into the brain, and that the substance or its metabolite can be detected at any site in the brain at some time after administration, or that knowledge which can be inferred to indicate that the substance had an effect in the brain can be obtained.

### Brain-related Disease

Brain-related disease is a disease caused by some abnormality in the brain, for example, central nervous system (CNS) diseases. Examples of the brain-related disease include, but are not limited to, Alzheimer's disease, Parkinson's disease, prion disease, Huntington's disease, lysosomal disease, central nervous system disorders, central nervous system tumors including brain tumors, cerebral ischemia, diseases involving brain damage, traumatic central nervous system disorders, viral and bacterial central nervous system diseases, and diseases affecting the mind such as schizophrenia, depression, and the like.

### Compound Containing Antibody or Antigen-binding Fragment Thereof

Antibody indicates a glycoprotein produced by B cells, a type of lymphocyte, which is a molecule that plays an important role in immune response. It is also called immunoglobulin, γ (gamma)-globulin, or Ig. The antibody is composed of polypeptides called light chains and heavy chains, and can be divided into the Fc region and the Fab region. The Fab region is known to have antigen binding ability.

The compound containing the antigen-binding fragment thereof refers to the compound that contains the Fab region and the Fab region, which are regions of the above antibody that have antigen-binding ability.

Antibody can be further divided into several isotypes. In mammals, for example, five types are known: IgG, IgA, IgM, IgD, and IgE. Among them, IgG (immunoglobulin G) is the antibody most commonly found in humans, especially in blood and tissues, and is mainly involved in secondary immune responses. Almost all antibody drugs currently on the market are IgG.

IgG is further divided into four subclasses, IgG1, IgG2, IgG3, IgG4 and IgG5.

Preferred antibody in the present invention is IgG, and even more preferred antibody is IgG1 or IgG4. The compound containing preferred antigen-binding fragment is those containing the Fab region of IgG, and even more preferably those containing the Fab region of IgG1 or IgG4.

### Peptide with Cell Permeability

Peptides with cell permeability are well known, as described, for example, in Japanese Patent No. 6478632 and Japanese Patent No. 6708770 (Peptide with Cell Permeability). And, as demonstrated by the examples, the peptide in the present invention binds to the transferrin receptor and is taken up into the cell. Therefore, the complex of the present invention makes it possible to deliver the desired active ingredient into the cell, for example, it makes possible to deliver a nucleic acid drug into the cell.

### Peptide

It refers to a structure with multiple consecutive amino acids, and includes polypeptides and proteins. In this application, the term "amino acid" includes not only naturally occurring amino acids (natural amino acids) that are incorporated into peptide chains by translation of mRNA in cells, but also unnaturally occurring amino acids (unnatural amino acids) that can form part of a peptide chain by peptide bonding. Amino acids may be artificially synthesized or naturally occurring.

In the present application, peptides in which cyclic portion is formed by cyclization after synthesis (also called cyclic peptides) and peptides obtained by further chemical modification of such peptides are also included in the peptide.

In this specification, a cyclic peptide means a peptide that is cyclic in whole or in part by the bonding of two amino acids separated by one or more amino acid residues in the amino acid sequence between them. Although there is no restriction on the bonding type between the two amino acids, amide bond between the carboxyl group of one amino acid and the amino group of the other amino acid, thioether bond between the carboxyl group of one amino acid and the thiol group of the other amino acid, thiol bond between the thiol group of one amino acid and the thiol group of the other amino acid, those with a cyclic structure formed by lactam ring formation or macrocyclization reaction, and those with lasso-peptide-like structures are also included in the cyclic peptide. However, when the two amino acids are bonded by the amide bond, the amide bond is not limited to those formed by the bonding of the carboxyl group of one amino acid with the amino group of the other amino acid, but only if the amide bond is formed as a result of a synthetic reaction. The same is true for other bond types.

That is, in the present application, a cyclic peptide may have a linear portion, as long as a portion of the cyclic peptide forms a cyclic structure.

In this specification, a part of amino acid may be modified for cyclization of peptides. Such partially modified amino acid is also included in the amino acid of this application. For example, a chloroacetyl group is added to the amino acid located at the N-terminal end, which is combined with a cysteine residue in the peptide to form a ring, and various (natural/unnatural) amino acids to which a chloroacetyl group is added are also included in the amino acid of the present application.

Non-natural amino acids are compounds other than natural amino acids that have the characteristics of amino acids. Examples include, but not limited to, amino acids that do not constitute proteins *in vivo,* such as β-amino acid, γ-amino acid, L-amino acid, D-amino acid (also called D-type amino acid), amino acid mutant, chemically modified amino acid such as amino acid derivative, norleucine, β-alanine, ornithine, etc. Also, Examples include N-methylamino acid, N-ethylamino acid, D-amino acid, histidine-like amino acid, amino acids having structures such as extra methylene or aromatic ring in the side chain, and amino acid derivatives having structures in which the carboxylic acid functional group in the side chain is replaced by a sulfonic acid group.

Examples of unnatural amino acids and their abbreviations in this specification are given below. CAS reference numbers or company name from which purchased are given in parentheses, and synthetic example numbers are given for newly synthesized compounds. Specialty amino acids are not limited to those listed above and, for example, those with a structure in which one or more of the hydrogen atoms in the molecule is replaced by an alkyl group are also specialty amino acids. When a hydrogen atom is replaced by an alkyl group, the alkyl group is preferably a methyl group or an ethyl group, more preferably a methyl group. In this specification, amino acids with Me or N-Me in front of the amino acid name indicate N-methylamino acids unless otherwise noted. For example, N-methylated amino acid of alanine (Ala or A) is indicated as MeAla, N-MeAla, MeA or N-MeA. In addition, amino acids with a single letter notation and with the letter d in front of it are indicated as D-amino acids. For example, the D-amino acid of alanine (Ala or A) is indicated as da. Those without a CAS number or company name may be purchased as general reagents. The following amino acids can be used in peptide synthesis by Fmoc-protecting the alpha-amino group by known methods.

Yph: (S)-2-Amino-3-(4-phenoxyphenyl) propanoic acid (CAS No.: 150351-64-7)
W7OMe: (S)-2-Amino-3-(7-methoxy-1H-indol-(3-yl) propanoic acid (CAS No.: 25198-03-2)
W7N: (S)-2-Amino-3-(1H-pyrrolo[2,3-β]pyridin-3-yl) propanoic acid (CAS No.: 49758-35-2)
W7F: (S)-2-Amino-3-(7-fluoro-1H-indol-3-yl) propanoic acid (CAS No.: 138514-97-3)
W6N: (S)-2-Amino-3-(1H-pyrrolo[2,3-c]pyridin-3-yl) propanoic acid (Kishida Chemical Inc.)
(CAS No.: 149704-63-2)
W6F: (S)-2-Amino-3-(6-fluoro-1H-indol-3-yl) propanoic acid (CAS No.: 19310-00-0)
W5OMe: 5-Methoxy-L-tryptophan (CAS No.: 25197-96-0)
W5F: (S)-2-Amino-3-(5-fluoro-1H-indol-3-yl) propanoic acid (CAS No.: 16626-02-1)
W4OMe: 4-Methoxy-L-tryptophan (CAS No.: 406938-53-2)
W4N: (S)-2-Amino-3-(1H-pyrrolo[3,2-β]pyridin-3-yl) propanoic acid (CAS No.: 149818-23-5)
W4F: (S)-2-Amino-3-(4-fluoro-1H-indol-3 (4-fluoro-1H-indol-3-yl) propanoic acid (CAS No.: 106034-22-4)
W4C: (S)-2-Amino-3-(4-chloro-1H-indol-3-yl) propanoic acid (CAS No.: 52448-14-3)
W2N:(S)-2-Amino-3-(1H-indol-3-yl) propanoic acid (CAS No.: 53538-54-8)
W1iPr: 1-lsopropyl-L-tryptophan (CAS No.: 1219485-46-7)
W1Et7CI: (S)-2-Amino-3-(7-chloro-1-ethyl-1H-indol-3-yl) propanoic acid
W1Et: 1-Ethyl-L-tryptophan (CAS No.: 168471-23-6)
Tbg: (S)-2-Amino-3,3-dimethylbutanoic acid (CAS No.: 158059-28-0)
pHPeG: N-(4-Hydroxyphenethyl) glycine (CAS No.: 169836-45-7)
PeG: N-(2-Phenylethyl)-glycine (CAS No.: 7738-38-7)
Nva: L-Norvaline (CAS No.: 6600-40-4)
Nle: L-Norleucine (CAS No.: 327-57-1)
Nal2: β-(2-Naphthyl) L-alanine (CAS No. 58438-03-2)
Nal1: β-(1-Naphthyl) L-alanine (CAS No. 55516-54-6)
MeoBph: N-α-Methyl-2-phenyl-L-phenylalanine
MeNal2: N-α-Methyl-β-(2-naphthyl)-L-alanine (CAS No.: 179385-30-9)
MeNal1: N-α-Methyl-β-(1-naphthyl)-L-alanine (CAS No.: 2137057-01-1)
MemBph: N-α-Methyl-3-phenyl-L-phenylalanine
Hph: L-Homophenylalanine (CAS No.: 943-73-7)
Hly: (S)-2,7-Diaminoheptanoic acid (CAS No.: 498-56-6)
F4OMe: (S)-2-Amino-3-(4-methoxyphenyl) propanoic acid (CAS No.: 7635-29-2)
F4G: (4-Guadinyl)-L-phenylalanine (CAS No.: 59574-11-7)
F4F: 4-Fluoro-L-phenylalanine (CAS No.: 1132-68-9)
F4C: 4-Chloro-L-phenylalanine (CAS No.: 14173-39-8)
F3OMe: (S)-2-Amino-3-(3-methoxyphenyl) propanoic acid (CAS No.: 98813-19-5) F3F: 3-Fluoro-L-phenylalanine (CAS No.: 19883-77-3)
F3C: 3-Chloro-L-phenylalanine (CAS No.: 80126-51-8)
F2OMe: (S)-2-Amino-3-(2-methoxyphenyl) propanoic acid (CAS No.: 193546-31-5)
F2C: (S)-2-Amino-3-(2-chlorophenyl) propanoic acid (CAS No.: 103616-89-3)
MeF4OMe: (S)-3-(4-Methoxyphenyl)-2-(methylamino) propanoic acid (CAS No.: 52939-33-0)
MeF4F: N-α-Methyl-4-fluoro-L-phenylalanine (CAS No.: 347851-71-2)
MeF: N-Methylphenylalanine
MeF3F: N-α-Methyl-3-fluoro-L-phenylalanine (CAS No.: 347851-71-2)
MeF3C: N-α-Methyl-3-chloro-L-phenylalanine (CAS No.: 2255324-91-3)
MeBph: N-α-Methyl-4-phenyl-L-phenylalanine
Me4Py: N-α-Methyl-4-pyridyl-L-alanine
Me3Py: N-α-Methyl-3-pyridyl-L-alanine
dr: D-arginine
dp: D-proline
dc: D-Cysteine
dk: D-lysine
Dap: L-α,β-Diaminopropionic acid (CAS No.: 515-94-6)
Dab: (S)-2,4-Diaminobutanoic acid (CAS No.: 1758-80-1)
Cit: 2-Amino-5-ureidopentanoic acid (CAS No.: 627-77-0)
Cha: β-Cyclohexyl-L-alanine (CAS No.: 4441-50-3)
CeG: N-(2-Carboxyethyl)-glycine (CAS No.: 505-72-6)
Cbg: (S)-2-Amino-2-cyclobutylacetic acid (CAS No.: 49607-08-1)
Cba: Cyclobutylalanine (CAS No.: 1201593-65-8)
aMeY: α-Methyl-L-tyrosine (CAS No.: 658-48-0)
aMeW: α-Methyl-tryptophan (CAS No.: 153-91-3)
aMeK: α-Methyl-lysine (CAS No.: 111717-28-3)
aMeC: α-Methyl-L-cysteine (CAS No.: 441317-73-3)
Aib: α-Methylalanine (CAS No.: 62-57-7)
Ahp/Alahp: (S)-2-Aminoheptanoic acid (CAS No.: 1115-90-8)
Abu: L-α-Aminobutanoic acid (CAS No.: 1492-24-6)
A4paa: (S)-2-Amino-3-(1-(carboxymethyl) piperazin-4-yl) propanoic acid (Kishida Chemical Inc.)
5lnd: (S)-2-Amino-3-(1H-indol-5-yl) propanoic acid (CAS No.: 460096-38-2)
4Py2NH2: (S)-2-Amino-3-(2-aminopyridin-4-yl) propanoic acid (Kishida Chemical Inc.)
4Py: 4-Pyridyl-L-alanine (CAS No.: 1956-21-4)
3Py6NH2: (S)-2-Amino-3-(6-aminopyridin-3-yl) propanoic acid
3Py: 3-Pyridyl-L-alanine (CAS No.: 17470-24-5)
W1aa: 1-(Carboxymethyl)-L-tryptophan (CAS No.: 773823-50-0)
KCOpipzMe: N6-(4-Methylpiperazine-1-carbonyl)-L-lysine (Kishida Chemical Inc.)
W1mCON: 1-(2-Amino-2-oxoethyl)-L-tryptophan
W1EtOH: 1-(2-Hydroxyethyl)-L-tryptophan
3Py6OMe: (S)-2-Amino-3-(6-methoxypyridin-3-yl) propanoic acid (CAS No.: 1270317-99-1)
Epyrl2RCOO: 2-Amino-(5-((R)-2-((allyloxy) carbonyl) pyrrolidin-1-yl)-5-oxopentanoic acid (Glu(d-Pro-O-allyl)-OH)
Dpyrl2RCOO: 2-Amino-(4-((R)-2-((allyloxy) carbonyl) pyrrolidin-1-yl)-4-oxobutanoic acid (Asp(d-Pro-O-allyl)-OH)
MeF3COO: 3-Carboxy-N-methyl-phenylalanine (CAS No.: 1499826-56-0)
3lmp: 2-Amino-3-(imidazo[1,2-a]pyridin-3-yl) propanoic acid (CAS No.: 2276942-95-9) KaAc: N6-Glycyl-L-lysine (Lys(Gly-O-allyl)-OH)
A1Me4pip: 4-Amino-1-methylpiperazine-4-carboxylic acid (CAS No.: 15580-66-2)
Har: N6-Carbamimidoyl-L-lysine (CAS No.: 156-86-5)
Acpr: (S)-2-Amino-3-cyclopropylpropanoic acid (CAS No.: 1492156-90-7)
Atb: (S)-2-Amino-4,4-dimethylpentanoic acid (CAS No.: 1934633-35-8)
MeF35dC: (S)-3-(3,5-Dichlorophenyl)-2-(methylamino) propanoic acid (CAS No.: 1542508-65-5)
Adod: 12-Aminododecanoic acid (CAS No.: 693-57-2)
Hly: L-Homolysine (CAS No.: 37689-89-7)
W5C: 5-Chloro-L-tryptophan (CAS No.: 52448-15-4)
F3COO: L-3-Carboxyphenylalanine (CAS No.: 13861-02-4)
F3CON: L-3-Carbamoylphenylalanine (CAS No.: 1217651-22-3)
Hgl: L-2-Amino adipic acid (CAS No.: 1118-90-7)
Ndm: N,N-Dimethyl-L-asparagine (CAS No.: 62937-43-3)
KN3 or LysN3: 6-Azido-L-norleucine (CAS No.: 159610-92-1)
KAc: N6-Acetyl-L-lysine (CAS No.: 692-04-6)
dorn: D-ornithine (CAS No.: 348-66-3)
F3H: 3-Hydroxy-L-phenylalanine (CAS No.: 587-33-7)
Yae: O-(2-Aminomethyl)-L-tyrosine (CAS No.: 1909283-20-0)
F4aao: O-(2-Carboxymethyl)-L-tyrosine (CAS No.: 24558-63-2)
F4OEt: O-Ethyl-L-tyrosine (CAS No.: 32795-52-1)
F34dOMe: 3,4-Dimethoxy-L-phenylalanine (CAS No.: 142995-28-6)
alT: L-Arothreonine (CAS No.: 28954-12-3)
all: L-Alloisoleucine (CAS No.: 1509-34-8)
MeK: N-Methyl-L-lysine (CAS No.: 7431-89-2)
Tbg: (S)-2-Amino-3,3-dimethylbutyric acid (CAS No.: 20859-02-3)
Nva: L-Norvaline (CAS No.: 6600-40-4)
Abu: (S)-(+)-2-Aminobutyric acid (CAS No.: 1492-24-6)
da: D-Alanine
Bph: 4-Phenyl-L-phenylalanine (CAS No.: 155760-02-4)
ds: D-Serine
de: D-Glutamic acid
MeA: N-Methyl-L-alanine (CAS No.: 3913-67-5)
MeR: N-Methyl-L-arginine (CAS No.: 2480-28-6)
MeW: N-Methyl-L-tryptophan (CAS No.: 526-31-8)
MeY: N-Methyl-L-tyrosine
MeG: N-Methyl-glycine
K (Maleimide): N6-(4-((2,5-Dioxo-2,5-dihydro-1H-pyrrole-1-yl) methyl) cyclohexane-1-carbonyl)-L-lysine
dk (Maleimide): N6-(4-((2,5-Dioxo-2,5-dihydro-1H-pyrrole-1-yl) methyl) cyclohexane-1-carbonyl)-D-lysine
KTrzMal: (S)-2-Amino-6-(4-((2,5-dioxo-2,5-dihydro-1H-pyrrol-1-yl) methyl)-1H-1,2,3-triazol-1-yl) hexanoic acid
gAbu: 4-Aminobutanoic acid

The newly synthesized amino acids are useful in the production of various peptide derivatives because they may add new functions to various peptides.

The peptide of the present invention is:
(i) a peptide comprising 1st to 15th amino acid sequence (Ala-Val-Phe-Val-Trp-Asn-Tyr-Tyr-Ile-Ile-Arg-Arg-Tyr-MeY-Cys) of an amino acid sequence described in SEQ ID NO: 1;
(ii) a peptide comprising an amino acid sequence having substitution, deletion, addition and/or insertion of 1 to 11 amino acid residues in the 1st to 15th amino acid sequence of the amino acid sequence described in SEQ ID NO: 1;
(iii) a peptide comprising 1st to 12th amino acid sequence (Ala-Val-Phe-Val-Trp-Asn-Tyr-Tyr-Ile-Ile-Ser-Cys) of an amino acid sequence described in SEQ ID NO: 14; or
(iv) a peptide comprising an amino acid sequence having substitution, deletion, addition and/or insertion of 1 to 8 amino acid residues in the 1st to 10th amino acid sequence of the amino acid sequence described in SEQ ID NO: 14. Said peptide is a peptide that binds to the transferrin receptor.

Each of the above options (i) to (iv) may be selected in any combination.

A preferred example of this peptide is a peptide that can pass through the blood-brain barrier or has cell permeability.

### About Peptide Sequence

The number of substituted, deleted, added and/or inserted amino acids may be between 1 to 10 in the 1st to 15th amino acid sequence of the amino acid sequence described in SEQ ID NO: 1, and its lower limit is 1. The upper limit is 15, 14, 13, 12, 11, 10, 9, 8, 7, 6, 5, 4, 3, 2, and the minimum is 1.

In the amino acid sequence listed in SEQ ID NO: 14, from the 1st to the 12th amino acid sequence, it may be between 1 to 8, and its lower limit is 1. The upper limit is 8, 7, 6, 5, 4, 3, 2, and the minimum is 1. Such amino acid substitution is suitably conservative amino acid substitution.

### Conservative Amino Acid Substitution

The term "conservative amino acid substitution" means a substitution of functionally equivalent or similar amino acids. In general, a substitution within a certain group may be considered conservative regarding structure and function. However, as is clear to a person having ordinary skill in the art, the role played by a defined amino acid residue may be determined by its implication in the three-dimensional structure of the molecule containing the amino acid. For example, a cysteine residue may be an oxidized-type (disulfide) foam having a lower polarity than that of a reduced-type (thiol) foam. The long aliphatic part of the arginine side chain may constitute structurally and functionally important features. Furthermore, the side chain including an aromatic ring (tryptophan, tyrosine, phenylalanine) may contribute to ion-aromatic interaction or cation-pi interaction. In such a case, even if the amino acids having these side chains are substituted for amino acids belonging to the acidic or non-polar groups, they may be structurally and functionally conservative. There is a possibility that residues such as proline, glycine, cysteine (disulfide foam) have a direct effect on the three-dimensional structure of the main chain and often may not be substituted without structural distortion.

Conservative amino acid substitution, as shown below, includes specific substitution based on the similarity of side chains (for example, substitutions are described in Lehninger, Biochemistry, Revised 2nd Edition, published in 1975, pp. 73 to 75: L. Lehninger, Biochemistry, 2nd edition, pp. 73 to 75, Worth Publisher, New York (1975)), and typical substitution.

A preferred example of the peptide comprises an amino acid sequence containing one or more substitutions selected from the following Group 1 or Group2, wherein the Group 1 contains:
(I) substitution of the 1st alanine residue of SEQ ID NO: 1 for an aliphatic amino acid or a methylated aliphatic amino acid;
(II) substitution of the 2nd amino acid residue of SEQ ID NO: 1 for any amino acid residue or any N-methylamino acid;
(III) substitution of the 3rd amino acid residue of SEQ ID NO:1 for an aromatic amino acid residue, a methylated aromatic amino acid residue, or an amino acid residue having an aromatic ring in a side chain;
(IV) substitution of the 5th amino acid residue of SEQ ID NO:1 for an aromatic amino acid residue, a methylated aromatic amino acid residue, or an amino acid residue having an aromatic ring in a side chain;
(V) substitution of the 6th asparagine residue of SEQ ID NO:1 for a hydrophilic amino acid or alanine;
(VI) substitution of the 8th tyrosine residue of SEQ ID NO:1 for an aromatic amino acid residue, a methylated aromatic amino acid residue, or an amino acid residue having an aromatic ring in a side chain;
(VII) substitution of the 10th isoleucine residue of SEQ ID NO:1 for any amino acid;
(VIII) substitution of the 11th arginine residue of SEQ ID NO:1 for any amino acid;
(IX) substitution of the 12th arginine residue of SEQ ID NO:1 for any amino acid; and
(X) substitution of the 13th tyrosine residue of SEQ ID NO:1 for any amino acid;
(XI) substitution of the 14th N-methyltyrosine residue of SEQ ID NO:1 for any amino acid; and
the Group 2 contains:
(I) substitution of the 1st alanine residue of SEQ ID NO: 14 for an aliphatic amino acid or a methylated aliphatic amino acid;
(II) substitution of the 2nd amino acid residue of SEQ ID NO: 14 for any amino acid residue or any N-methylamino acid;
(III) substitution of the 3rd amino acid residue of SEQ ID NO: 14 for an aromatic amino acid residue, a methylated aromatic amino acid residue, or an amino acid residue having an aromatic ring in a side chain;
(IV) substitution of the 5th amino acid residue of SEQ ID NO: 14 for an aromatic amino acid residue, a methylated aromatic amino acid residue, or an amino acid residue having an aromatic ring in a side chain;
(V) substitution of the 6th asparagine residue of SEQ ID NO: 14 for a hydrophilic amino acid or alanine;
(VI) substitution of the 8th tyrosine residue of SEQ ID NO: 14 for an aromatic amino acid residue, a methylated aromatic amino acid residue, or an amino acid residue having an aromatic ring in a side chain;
(VII) substitution of the 10th isoleucine residue of SEQ ID NO: 14 for any amino acid; and
(X) substitution of the 11th serine residue of SEQ ID NO: 14 for a hydrophilic amino acid residue.

The term "methylation" means N-methylation, i.e., the addition of a methyl group to the amino group of the amino acid, and for example, when it is described as methylated alanine, it means N-methylalanine (MeA).

The term "having an aromatic ring in the side chain" means an amino acid having an aromatic ring in the side chain, which may be a fused ring or a heterocyclic ring. The aromatic ring may have substituents. For example, F4C is a kind of amino acid having an aromatic ring in its side chain because it has a benzyl group in which a carbon at position 4 is substituted for a carbon binding to chlorine, in its side chain.

Naturally occurring amino acids can be divided into the following groups based on the properties of their common side chains.
(1) Hydrophobic (also called non-polar) amino acids: Amino acids that exhibit hydrophobic (also called non-polar) and include alanine ("Ala" or simply "A"), glycine ("Gly" or simply "G"), valine ("Va!" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W'), tyrosine ("Tyr" or simply "Y"), methionine ("Met" or simply "M").

The hydrophobic amino acids can be further divided into the following groups.

Aliphatic amino acids: Amino acids with fatty acids or hydrogen in the side chain, including Ala, Gly, Val, Ile and Leu.

Aliphatic and branched-chain amino acids: Amino acids with branched fatty acids in the side chain, including Val, Ile and Leu.

Aromatic amino acids: Amino acids having aromatic rings in the side chain, including Trp, Tyr and Phe.

(2) Hydrophilic (also called polar) amino acids: Amino acids that exhibit hydrophilicity (polarity) and include serine ("Ser" or simply "S"), threonine ("Thr" or simply "T"), cysteine ("Cys" or simply "C"), asparagine ("Asn" or simply "N"), glutamine ("Gln" or simply "Q"), aspartic acid ("Asp" or simply "D"), glutamic acid ("Glu" or simply "E"), lysine ("Lys" or simply "K"), arginine ("Arg" or simply "R"), and histidine ("His" or simply "H").

The hydrophilic amino acids can be further divided into the following groups.

Acidic amino acids: Amino acids whose side chains are acidic, including Asp and Glu.

Basic amino acids: Amino acids whose side chains are basic, including Lys, Arg and His.

Neutral amino acids: Amino acids whose side chains indicate neutrality, including Ser, Thr, Asn, Gln and Cys.

Gly and Pro can be divided into "amino acids affecting the direction of the main chain", and amino acids containing a sulfur molecule in the side chain, Cys and Met, can be divided into "sulfur-containing amino acids".

The group with aromatics in the side chain includes Trp, Tyr and Phe.

An example of a preferred peptide in this specification is a peptide that can bind to the human transferrin receptor (hTfR) as well as the peptides described above. Also a preferred example is a peptide that can pass through the blood-brain barrier or has cellular permeability.

An example of preferred peptide in this specification may be a peptide having an amino acid sequence in which one of the 1st, 2nd, 3rd, 5th, 6th, 8th, 10th, or 14th amino acid residues of the amino acid sequence from the 1st to the 15th amino acid residue (Ala-Val-Phe-Val-Trp-Asn-Tyr-Tyr-Ile-Ile-Arg-Arg-Tyr-MeY-Cys) of the amino acid sequence described in SEQ ID NO: 1 is substituted.

The term "amino acid residue is substituted" means that a specific amino acid residue has been replaced by another amino acid residue that may be modified.

In the peptide comprising the 1st to 15th amino acid residue (Ala-Val-Phe-Val-Trp-Asn-Tyr-Tyr-Ile-Ile-Arg-Arg-Tyr-MeY-Cys) of the amino acid sequence described in SEQ ID NO: 1, the peptide may be a peptide in which:
the 1st alanine residue of SEQ ID NO: 1 is alanine (Ala), which may be modified, or glutamic acid (Glu), which may be modified;
the 2nd valine residue of SEQ ID NO: 1 is valine (Val), which may be modified, or glutamic acid (Glu), which may be modified;
the 3rd phenylalanine residue in SEQ ID NO: 1 is phenylalanine (Phe), which may be modified;
the 5th tryptophan residue in SEQ ID NO: 1 is tryptophan (Trp), which may be modified;
the 6th asparagine residue in SEQ ID NO: 1 is alanine;
the 8th tyrosine residue of SEQ ID NO: 1 is phenylalanine, which may be modified;
the 10th isoleucine residue of SEQ ID NO: 1 is isoleucine (Ile), which may be modified, alanine (Ala), which may be modified, or valine (Val), which may be modified;
the 11th arginine residue of SEQ ID NO: 1 is any amino acid residue; and
the 12th isoleucine residue of SEQ ID NO: 1 is isoleucine (Ile), which may be modified, or valine (Val), which may be modified.

The term "may be modified" means that known amino acid modifications or alterations may be made. Examples of modifications are N-methylation (also called methylation), amino acid modifications with abbreviations as described below, modification (conversion) to D-type, and conversion to known derivatives of the amino acid.

In addition, in the peptide comprising the 1st to 15th amino acid residue (Ala-Val-Phe-Val-Trp-Asn-Tyr-Tyr-Ile-Ile-Arg-Arg-Tyr-MeY-Cys) of the amino acid sequence described in SEQ ID NO: 1, the peptide may be a peptide in which:
the 1st amino acid residue of SEQ ID NO:1 is Ala, Aib, Abu, Glu, Gly, Ser, Phe, Pro or MeA, especially preferably Ala or Abu;
the 2nd amino acid residue of SEQ ID NO:1 is Val, Glu, Ala, Arg, Lys, Asp, Phe, Dap, Har, Abu, Nva, AcPr, Atb, Ahp or Hgl, especially preferably Val, Glu or Hgl;
the 3rd amino acid residue of SEQ ID NO:1 is Phe, F3C, F2C, F2OMe, F4C, Cha, MeF, MeF35dC, MeF4F, MeF4Ome, MeNal1, Me3Py, Me4Py, Me3OMe, MeF3COO, MeF3F, Glu, Epyrl2RCOO, Dpyrl2RCOO or MeF3C, especially preferably Phe, MeF or MeF3C;
the 5th amino acid residue of SEQ ID NO:1 is Trp, MeW, aMeW, dp, F3C, F3F, F3OMe, F4C, F4F, Hph, MemBph, MeNal1, MeNal2, MeoBph, W4OMe, W1Et, W1Et7Cl, W1iPr, Yph, W1Pr, W5C, W5F, W1aa, W1EtOH, W4OMe, W1mCON or W6F, especially preferably Trp or MeTrp;
the 6th amino acid residue of SEQ ID NO:1 is Asn, Ala or Asp, especially preferably Ala or Asn;
the 8th amino acid residue of SEQ ID NO:1 is Phe, Tyr, Typ, Ahp, MeY, F4OMe, 3lmp, 4Py, 3Py, 3Py6OMe, F3C, F3CON, F4C, F4aao, F4F, F4OEt, MeF34dOMe, Yae, Lys, Orn, or Nal1, especially preferably Tyr, Ahp or F4OMe;
the 10th amino acid residue of SEQ ID NO:1 is Ala, Abu, Acpr, Ahp, Aib, all, alT, Atb, Dab, Dap, dorn, Gln, Hly, Ile, Lys, KCOpipzMe, Leu, Nle, Nva, Pro, Arg, Ser, Thr, Tbg, Val or Tyr, especially preferably Ile or Ala;
the 11th amino acid residue of SEQ ID NO:1 is Arg, Ala, Asp, Gly, Glu, Lys, MeK, MeR, Dap, Dab, Abu, Aib, Hly, dorn, aMeK, A1 Me4pip, KCOpipzMe, F4G, Nle, Nva or Orn, especially preferably Ala Glu, Lys, Arg or Hly;
the 12th amino acid residue of SEQ ID NO:1 is Lys, Glu, Arg, dr, Tyr, F4G, Orn, Hly, da, Cit, Dap or Dab, especially preferably Glu, Arg or dr;
the 13th amino acid residue of SEQ ID NO:1 is Ala, Phe, Asn, Tyr or pHPeG, especially preferably Phe or Tyr; and
the 14th amino acid residue of SEQ ID NO:1 is MeY, Tyr, Phe, Ala, aMeY, Glu, Gly, Arg, Val, MeoBphMeBph, MeF, MemBph, MeNal1, MeNal2, MeoBph, MeW or pHPeG, especially preferably Tyr or MeTyr.

Another preferred example of this peptide is a peptide comprising an amino acid sequence containing 1 to 8 substitutions, deletions, and/or insertions of amino acid residues in the 1st to 12th amino acid residue (Ala-Val-Phe-Val-Trp-Asn-Tyr-Tyr-Ile-Ile-Ser-Cys) of the amino acid sequence described in SEQ ID NO: 14.

In the peptide containing the 1st to 12th amino acid sequence (Ala-Val-Phe-Val-Trp-Asn-Tyr-Tyr-Ile-Ile-Ser-Cys) of the amino acid sequence described in SEQ ID NO: 14, the peptide may contain a substitution in any one of 1st, 2nd, 3rd, 5th, 6th, 8th, 10th, or 11th amino acid residue of SEQ ID NO: 14.

In the peptide containing the 1st to 12th amino acid sequence (Ala-Val-Phe-Val-Trp-Asn-Tyr-Tyr-Ile-Ile-Ser-Cys) of the amino acid sequence described in SEQ ID NO: 14, the peptide may be a peptide in which:
the 1st alanine residue of SEQ ID NO:14 is alanine (Ala), which may be modified, or glutamic acid (Glu), which may be modified;
the 2nd valine residue of SEQ ID NO:14 is valine (Val), which may be modified, or glutamic acid (Glu), which may be modified;
the 3rd phenylalanine residue of SEQ ID NO:14 is phenylalanine (Phe), which may be modified;
the 5th tryptophan residue of SEQ ID NO:14 is tryptophan (Trp), which may be modified;
the 6th asparagine residue of SEQ ID NO:14 is alanine;
the 8th tyrosine residue of SEQ ID NO:14 is phenylalanine, which may be modified;
the 10th isoleucine residue of SEQ ID NO:14 is isoleucine (Ile), which may be modified, alanine (Ala), which may be modified, or valine (Val), which may be modified; and
the 11th serine residue of SEQ ID NO:14 is histidine (His) or asparagine (Asn).

These peptides may have a peptide length of between 12 and 15, preferably between 12 and 14, more preferably between 12 and 13, and most preferably 12. In this case, peptide length refers to the number of amino acid sequences in the cyclic structure and does not include the number of amino acid linkers.

In addition, in the peptide containing the 1st to 12th amino acid sequence (Ala-Val-Phe-Val-Trp-Asn-Tyr-Tyr-Ile-Ile-Ser-Cys) of the amino acid sequence described in SEQ ID NO: 14, the peptide may be a peptide in which:
the 1st amino acid residue of SEQ ID NO:14 is Ala, Aib, Abu, Glu, Gly, Ser, Phe, Pro or MeA, especially preferably Ala;
the 2nd amino acid residue of SEQ ID NO:14 is Val, Glu, Ala, Arg, Lys, Asp, Phe, Dap, Har, Abu, Nva, AcPr, Atb, Ahp or Hgl, especially preferably Val;
the 3rd amino acid residue of SEQ ID NO:14 is Phe, F3C, F2C, F2OMe, F4C, Cha, MeF, MeF35dC, MeF4F, MeF4Ome, MeNal1, Me3Py, Me4Py, Me3OMe, MeF3COO, MeF3F, Glu, Epyrl2RCOO, Dpyrl2RCOO or MeF3C, especially preferably Phe;
the 5th amino acid residue of SEQ ID NO:14 is Trp, MeW, aMeW, dp, F3C, F3F, F3OMe, F4C, F4F, Hph, MemBph, MeNal1, MeNal2, MeoBph, W4OMe, W1Et, W1Et7Cl, W1iPr, Yph, W1Pr, W5C, W5F, W1aa, W1EtOH, W4OMe, W1mCON or W6F, especially preferably Trp;
the 6th amino acid residue of SEQ ID NO: 14 is Asn, Ala or Asp, especially preferably Asn;
the 8th amino acid residue of SEQ ID NO:14 is Phe, Tyr, Typ, Ahp, MeY, F4OMe, 3lmp, 4Py, 3Py, 3Py6OMe, F3C, F3CON, F4C, F4aao, F4F, F4OEt, MeF34dOMe, Yae, Lys, Orn, or Nal1, especially preferably Tyr;
the 10th amino acid residue of SEQ ID NO:14 is Ala, Abu, Acpr, Ahp, Aib, all, alT, Atb, Dab, Dap, dorn, Gln, Hly, Ile, Lys, KCOpipzMe, Leu, Nle, Nva, Pro, Arg, Ser, Thr, Tbg, Val or Tyr, especially preferably Ile; and
the 11th amino acid residue of SEQ ID NO:14 is Ser, His orAsn, especially preferably Ser.

A preferred example of this peptide is a peptide consisting of the 1st to 15th amino acid sequence of SEQ ID NOs: 1 to 13, 15, 18 to 86, 90 to 110, or the 1st to 12th amino acid sequence of SEQ ID NOs: 14, 16, 17, 87 to 89.

A preferred example of this peptide is any one of the above-mentioned peptides, which are cyclic peptides. Preferably, the 1st amino acid in the 1st to 15th amino acid sequence of SEQ ID NOs: 1 to 13, 15, 18 to 86, 90 to 110, or the 1st to 12th amino acid sequence of SEQ ID NOs: 14, 16, 17, 87 to 89, is chloroacetylated, and its acetyl group is cyclized with cysteine at the end of the amino acid sequence.

A preferred example of this peptide is an amino acid sequence described in SEQ ID NOs: 1 to 110 or a complex of the amino acid sequence and a linker (a linker-attached peptide described below), wherein the peptide is (1) a peptide comprising the 1st to 15th amino acid sequence part of SEQ ID NOs: 1 to 13, 15, 18 to 86, 90 to 110, and the part containing a cyclic structure, or (2) a peptide comprising the 1st to 12th amino acid sequence part of SEQ ID NOs: 14, 16, 17, 87 to 89, and the part containing a cyclic structure.

### About Cyclic Peptide

It refers to a peptide in which two amino acids are bound and the entirety or a part thereof are cyclic. In the present invention, this peptide also includes amino acids in the peptide forming a cross-linked structure; forming a cyclic structure by lactam ring formation or a macrocyclization reaction; having a lasso peptide-like structure; and the like. That is, a part of the cyclic peptide may form a cyclic structure or it may have a straight-chain part. It may also take a complex cyclic structure, such as a bicyclic structure in which two amino acids contained in a cyclic peptide are further bound.

In general, peptides exhibit poor metabolic stability *in vivo,* and peptides are large in size, making it difficult for them to penetrate cell membranes. A method for cyclizing a peptide has been adopted in light of such problems. It has been suggested that when a peptide is cyclized, protease resistance is improved, metabolic stability is improved, and restrictions are also added to conformational change, so that rigidity is increased and membrane permeability and affinity for the target protein is improved.

### Cyclization Method

Cyclization of the peptide may be carried out according to a known method.

In a non-limiting manner, by designing the peptide to comprise two or more cysteine residues, for example, a cyclic structure may be formed by a disulfide bond after translation. Furthermore, according to the method of Goto et al. (Y. Goto, et al. ACS Chem. Biol. 3 120-129 (2008)), a peptide having a chloroacetyl group at its N-terminal may be synthesized by genetic code reprogramming technology and may also be circularized by disposing a cysteine residue containing a sulfur molecule in the peptide. Thus, a mercapto group spontaneously performs a nucleophilic attack on the chloroacetyl group after translation, and the peptide is circularized by thioether binding. Other amino acid combinations that bind to form a ring may be disposed within the peptide and circularized by genetic code reprogramming technology. The peptide can also be cyclized by synthesizing a peptide with a cycloamide at the N-terminal and placing Hgl residue in the peptide. In this manner, a known circularization method may be used without any particular limitation.

Said peptide has a cyclic structure in which the N-terminal amino acid (the 1st amino acid residue) and a cysteine residue in said peptide are bonded. In one aspect, said peptide has a cyclic structure in which the N-terminal amino acid (the 1st amino acid residue) is bonded to the 15th or 12th cysteine residue in said peptide. In one aspect, said peptide has a cyclic structure in which the chloroacetylated N-terminal amino acid (the 1st amino acid residue) is bonded to the 15th or 12th cysteine residue in said peptide. "Chloroacetylation" can also be "halogen acetylation" with other halogens. "Acetylation" can also be acylation with an acyl group other than an acetyl group.

In this specification, some amino acids may be modified for cyclization of peptides. Amino acids with such partial modifications are also included in the amino acid of the present application. For example, as mentioned above, a chloroacetyl group may be added to the amino acid located at the N-terminal, which is combined with a cysteine residue in the peptide for cyclization, and various (natural/unnatural) amino acids to which such a chloroacetyl group is added are also included in the amino acids in this application.

A preferred example of the peptide of the present invention is any one of the above peptides, consisting of 15 amino acid residues or 12 amino acid residues.

### Peptide Length

The number of amide bonds (the number and length of amino acids) in the peptide or peptide moiety is not particularly limited, but the total amino acid residues (if the substance bound to the peptide or the linker that binds said substance and peptide contains amino acids, those amino acids are not included) should not exceed 20 residues. Preferably, the number of amino acids is 6 or more, 7 or more, 8 or more, 9 or more, 10 or more, 11 or more, and preferably the number of amino acids is 19 or less, 18 or less, 17 or less, 16 or less, 15 or less.

### Linker

The conjugate (complex) of the present invention contains a peptide and an antibody or an antigen-binding fragment thereof, and the peptide and the antibody or the antigen-binding fragment thereof are preferably bound via a linker. The linker between said peptide and the antibody or the antigen-binding fragment thereof may be formed by chemical bonding of the linker of the peptide containing the linker (hereinafter referred to as linker-attached peptide) and the antibody or the antigen-binding fragment thereof by any suitable reactive group, including reactive functional groups as described below.

Linker-attached peptides are described below.

An example of the linker in the linker-attached peptide is that its amino acid length of the linker is between 1 and 15, and the linker contains either or both of glycine (Gly) and serine (Ser).

Preferred example of this linker is cysteine (Cys), which may be modified at the N-terminal, or lysine (Lys), which may be modified.

Another example of a linker in the linker-attached peptide is one that has an amino acid length between 1 and 5 and contains either or both D-body glutamic acid (de) and methylated glycine (MeG).

Preferred example of this linker is cysteine (Cys), which may be modified at the N-terminal, or lysine (Lys), which may be modified.

Another example of a linker is a PEG linker containing polyethylene glycol (PEG) or a derivative of polyethylene glycol. Derivatives of polyethylene glycol include all those known as PEG linkers. Some may be partially substituted for a functional group or functionalized by a functional group. Examples include, but are not limited to, methyl groups, amino groups, azide groups, etc.

Preferably, the PEG linkers are PEG4c, PEG12c, PEG8c, PEG36, PEG4c-PEG4c-PGE4c, PEG4c-PEG4c.

It is also preferable that the PEG linker further contains one or more of glycine (Gly), serine (Ser), glutamic acid (Glu), arginine (Arg), gAbu, KTrzMal and lysine (Lys).

In addition, the linker may also contain reactive functional groups to bind the desired substance. Examples of reactive functional groups are maleimides, hydrazides, NHS, and functional groups used for click reaction.

Another example of a linker in a linker-attached peptide is a linker having the sequence indicated as Linker SEQ in TABLE 1 or one having a sequence described in any one of the SEQ ID NOs: 111 to 161 shown in Table 5.

A preferred example of the linker in the linker-attached peptide is:
polyethylene glycol (PEG);
a G linker which is a peptide linker consisting of polyethylene Gly or MeG; a GS linker which is a peptide linker consisting of Gly or MeG and Ser; a linker described as Linker SEQ in TABLE 1; or a linker having the amino acid sequence shown in any one of SEQ ID NOs: 111 to 161.

A linker (also called crosslinker) herein refers to an intermolecular linkage between a peptide that binds to the transferrin receptor and an antibody or an antigen-binding fragment thereof, and also it may be any linker known or described herein. In certain embodiments, said linker is, for example, a chemical linker, a fatty acid linker, a peptide linker (polypeptide linker). It may also be a complex of, for example, a chemical linker and a peptide linker. For example, it may be a linker structure having both PEG and amino acid residues or peptide parts, as shown in the sequence shown as Linker SEQ in TABLE 1 or in any one of SEQ ID NOs: 111 to 161 in TABLE 5.

Linkers may, for example, be such that they diverge or separate depending on the environment and condition, or they may maintain a stable structure.

Chemical linker: In some embodiments, the linker may be a chemical linker. Chemical linkers include, but are not limited to, substituted or unsubstituted alkylene, substituted or unsubstituted heteroalkylene, substituted or unsubstituted cycloalkylene, substituted or unsubstituted heterocycloalkylene, substituted or unsubstituted arylene and/or substituted or unsubstituted heteroarylene. The peptide and linker can also be conjugated via sulfhydryl group, amino group (amine), and/or carbohydrate or any suitable reaction group. Homobifunctional and heterobifunctional crosslinkers (conjugating agents) are available from many commercial sources. Crosslinkers may contain flexible arms, e.g., 2, 3, 4, 5, 6, 7, 8, 9, 10, 11, 12, 13, 14 or 15 carbon atoms. Examples of crosslinkers include BSS3 (bis(sulfosuccinimidyl)suberate), NHSS/EDC (N-hydroxysuccinimide and N-ethyl-(dimethylaminopropyl)carbodiimide), sulfo-EMCSS ([N-e-maleimidocaproic acid] hydrazide), hydrazide, and SSATA (N-succinimidyl-SS-acetylthioacetic acid), and others.

Preferred examples of the chemical linker include a PEG (polyethylene glycol) linker. For example, the PEG linker may consist of 1 to 24 ethylene glycol units.

Fatty acid linker: The linker may be a fatty acid linker containing a divalent chemical moiety derived from a fatty acid. For example, the fatty acid linker may be a linker with 12-aminododecanoic acid.

Peptide linker: A peptide linker contains at least one amino acid (e.g., a peptide of at least 2, 3, 4, 5, 6, 7, 10, 15, 20, 25, 40 or 50 amino acids). In a certain embodiment, the linker is one amino acid (e.g., any natural amino acid such as Cys). In another embodiment, a glycine-rich peptide such as a peptide comprising a sequence [Gly-Gly-Gly-Gly-Ser]n (in the formula, n is 1, 2, 3, 4, 5, or 6) such as that according to US Patent No. 7,271,149 may be used. In another embodiment, a serine-rich peptide linker according to US Patent No. 5,525,491 may be used. As the serin-rich peptide linker, the linker which has the formula [X-X-X-X-Gly]y (wherein up to two of the Xs are Thr, the remaining Xs are Ser, and y is from 1 to 5) (e.g. Ser-Ser-Ser-Ser-Gly (where y is 2 or more)) can be mentioned. In some cases, the linker is a single amino acid (e.g., any amino acid such as Gly or Ala).

Alternatively, other known linkers, such as those described in WO2021/054370, WO2020/209285, WO2020/028832, WO2017/221883, WO2015/194520, WO2012/150960, WO2012/029986 and the like can be mentioned.

### Production Method

The peptide of the present invention may be produced by, for example, any known method for producing a peptide, such as a chemical synthesis method such as a liquid phase method, a solid phase method, a hybrid method combining a liquid phase method and a solid phase method or the like, and a genetic recombination method; or the like.

In the solid phase method, for example, a hydroxy group of a resin having a hydroxy group and a carboxy group of a 1st amino acid (normally a C-terminal amino acid of a target peptide) in which an α-amino group is protected by a protecting group are subjected to an esterification reaction. For the esterification catalyst, a known dehydrating and condensing agent such as 1-mesitylenesulfonyl-3-nitro-1, 2, 4-triazole (MSNT), dicyclohexylcarbodiimide (DCC), and diisopropylcarbodiimide (DIPCDI) may be used.

Next, the protecting group of the α-amino group of the 1st amino acid is removed, a second amino acid in which all functional groups except the carboxy group of the main chain are protected is added, and the carboxy group is activated, binding the 1st and 2nd amino acids. Furthermore, the α-amino group of the 2nd amino acid is deprotected, a 3rd amino acid in which all functional groups except the carboxy group of the main chain are protected is added, the carboxy group is activated, binding the 2nd and 3rd amino acids. This is repeated, and after a peptide having a target length is synthesized, all of the functional groups are deprotected.

Examples of the resin for solid-phase synthesis include Merrifield resin, MBHA resin, C!-Trt resin, SASRIN resin, Wang resin, Rink amide resin, HMFS resin, Amino-PEGA resin (Merck KGaA), HMPA-PEGA resin (Merck KGaA), and the like. These resins may be used after being washed using a solvent (dimethylformamide (DMF), 2-propanol, methylene chloride, and the like).

Examples of the protecting group of the α-amino group include the benzyloxycarbonyl (Cbz or Z) group, tert-butoxycarbonyl (Boc) group, fluorenylmethoxycarbonyl (Fmoc) group, benzyl group, allyl group, allyloxycarbonyl (Alloc) group, and the like. The Cbz group may be deprotected by a treatment using hydrofluoric acid, hydrogenation, or the like, the Boc group may be deprotected by a treatment using trifluoroacetic acid (TFA), and the Fmoc group may be deprotected by a treatment using piperidine.

Examples such as methyl ester, ethyl ester, benzyl ester, tert-butyl ester, cyclohexyl ester, and the like may be used to protect the α-carboxy group.

As other functional groups of amino acids, the hydroxy groups of serine and threonine can be protected by benzyl or tert-butyl groups, and the hydroxy group of tyrosine can be protected by 2-bromobenzyloxycarbonyl group or tert-butyl group. The amino group of the lysine side chain and the carboxy group of glutamic acid and aspartic acid can be protected as well as the α-amino and α-carboxy groups.

Activation of the carboxy group may be performed using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazole-1-yloxy)tris(dimethylamino)phosphonium hexafluorophosphate (BOP), 1-[bis(dimethylamino)methyl]-1H-benzotriazolium-3-oxide hexafluorophosphate (HBTU), and the like.

Cleavage of the peptide chain from the resin may be performed by treating the peptide chain using an acid such as TFA, hydrogen fluoride (HF), or the like.

Production of a peptide by a gene recombination method (translation/synthesis system) may be performed by using a nucleic acid encoding the peptide according to the present invention. The nucleic acid encoding the peptide according to the present invention may be DNA or RNA.

The nucleic acid encoding the peptide according to the present invention may be prepared by a known method or a method equivalent thereto. For example, the nucleic acid may be synthesized by an automated synthesizer. A restriction enzyme recognition site may be added to insert the obtained DNA into a vector, or a base sequence encoding an amino acid sequence for splicing a formed peptide chain using an enzyme or the like may be incorporated.

As described above, when the peptide according to the present invention is fused to a cell-penetrating peptide or the like, the nucleic acid also includes a nucleic acid encoding the cell-penetrating peptide.

A chimeric protein expression method for expressing the target peptide as a chimeric peptide of another peptide may also be used to suppress degradation by a host-derived protease. In this case, a nucleic acid encoding the target peptide and the peptide bound thereto may be used as the nucleic acid.

Subsequently, an expression vector is prepared using the nucleic acid encoding the peptide according to the present invention. The nucleic acid may be inserted downstream of a promoter of the expression vector, either as is; after digestion with restriction enzymes; or with the addition of a linker. Examples of the vector include an *Escherichia* coli-derived plasmids (pBR322, pBR325, pUC12, pUC13, pUC18, pUC19, pUC118, pBluescript II, and the like), a *Bacillus subtilis*-derived plasmids (pUB110, pTP5, pC1912, pTP4, pE194, pC194, and the like), a yeast-derived plasmids (pSH19, pSH15, YEp, YRp, Ylp, YAC, and the like), bacteriophages (e phage, M13 phage, and the like), viruses (retrovirus, vaccinia virus, adenovirus, adeno-associated virus (AAV), cauliflower mosaic virus, tobacco mosaic virus, baculovirus, and the like), cosmids, and the like.

The promoter may be selected appropriately according to the type of host. When the host is an animal cell, for example, a promoter derived from SV40 (simian virus 40) or a promoter derived from CMV (cytomegalovirus) may be used. When the host is *Escherichia coli,* a trp promoter, a T7 promoter, a lac promoter, or the like may be used.

The expression vector may incorporate, for example, a DNA replication starting point (ori), a selective marker (antibiotic resistance, auxotrophy, or the like), an enhancer, a splicing signal, a poly-A addition signal, a nucleic acid encoding a tag (FLAG, HA, GST, GFP, or the like), or the like.

Next, an appropriate host cell is transformed by the expression vector. The host may be appropriately selected in relation to the vector. Examples such as *Escherichia coli, Bacillus subtilis* (*Bacillus*), yeast, insects or insect cells, animal cells, or the like may be used as the host. As the animal cells, for example, HEK293T cells, CHO cells, COS cells, myeloma cells, HeLa cells, and Vero cells may be used. Transformation may be carried out according to a known method, such as a lipofection method, a calcium phosphate method, an electroporation method, a microinjection method, a gene gun method, or the like depending on the type of host. The target peptide is expressed by culturing a transformant according to a conventional method.

As for purification of the peptide from the transformant culture, cultured cells are recovered and then suspended in an appropriate buffer solution, followed by disruption of cells by a method such as sonication, freeze-thawing, or the like, and then a crude extract is obtained by centrifugation or filtration. When the peptide is secreted into the culture solution, a supernatant is recovered.

Purification of the crude extract or the culture supernatant may also be performed by a known method or a method equivalent thereto (for example, salting-out, dialysis, an ultrafiltration method, gel filtration method, SDS-PAGE method, ion exchange chromatography, affinity chromatography, reversed-phase high-performance liquid chromatography, and the like).

The obtained peptide may be converted from a free body to a salt or from a salt to a free body by a known method or a method equivalent thereto.

The translation/synthesis system may be a cell-free translation system. The cell-free translation system includes, for example, a ribosome protein, an aminoacyl-tRNA synthase (ARS), a ribosome RNA, an amino acid, rRNA, GTP, ATP, a translation initiation factor (IF), an elongation factor (EF), a release factor (RF), and a ribosome regeneration factor (RRF), or another factor required for translation. An *Escherichia coli* extract or a wheat embryo extract may be added to increase expression efficiency. In addition, a rabbit red blood cell extract or an insect cell extract may be added.

By continuously supplying energy to a system including these using dialysis, a protein of several hundred µg to several mg/mL may be produced. The system may include an RNA polymerase to concurrently perform transcription of genomic DNA. Examples of commercially available cell-free translation systems that may be used include RTS-100 (registered trademark) by Roche Diagnostics K.K., PURE System by GeneFrontier Corporation, PURExpress In Vitro Protein Synthesis Kit by New England Biolabs Inc., and the like for a system derived from *Escherichia coli,* and a system by ZOIGENE, CellFree Sciences Co., Ltd., or the like for a system using wheat embryo extract.

According to the cell-free translation systems, the expression product can be obtained in a highly pure form without purification.

In the cell-free translation system, artificial aminoacyl-tRNA in which a desired amino acid or hydroxy acid may be linked (acylated) to a tRNA may be used in place of an aminoacyl-tRNA synthesized by a natural aminoacyl-tRNA synthetase. The aminoacyl-tRNA may be synthesized using an artificial ribozyme.

An example of the ribozyme includes a flexizyme (H. Murakami, H. Saito, and H. Suga, (2003), Chemistry & Biology, Vol. 10, 655-662; H. Murakami, D. Kourouklis, and H. Suga, (2003), Chemistry & Biology, Vol. 10, 1077-1084; H. Murakami, A. Ohta, H. Ashigai, and H. Suga (2006) Nature Methods 3, 357-359 "The flexizyme system: a highly flexible tRNA aminoacylation tool for the synthesis of nonnatural peptides"; N. Niwa, Y. Yamagishi, H. Murakami, H. Suga (2009) Bioorganic & Medicinal Chemistry Letters 19, 3892-3894 "A flexizyme that selectively charges amino acids activated by a water-friendly leaving group"; and WO 2007/066627 and the like). Flexizymes are also known under the names of prototype flexizyme (Fx), and dinitrobenzyl flexizyme (dFx), enhanced flexizyme (eFx), aminoflexizyme (aFx) and the like which are modified from it.

A desired codon may be translated in association with the desired amino acid or hydroxy acid by using the tRNA produced by flexizyme and to which the desired amino acid or hydroxy acid is linked. A specialty amino acid may be used as the desired amino acid. For example, an unnatural amino acid required for the above circularization may also be introduced into the binding peptide by this method.

Various methods commonly used in the technical field may be used for chemical synthesis of the cyclic peptides regarding the present invention and their analogs, including, for example, stepwise solid-phase synthesis, semisynthesis of peptide fragments undergoing conformationally supported religation, and chemical ligation. Synthesis of the peptides and their analogs described herein is chemical synthesis using various solid phase technologies described in, for example, K. J. Jensen, P. T. Shelton, S. L. Pedersen, Peptide Synthesis and Applications, 2nd Edition, Springer, 2013, and the like. A preferable strategy is based on a combination of an Fmoc group capable of temporarily protecting the α-amino group and being selectively removed using a base, and a protecting group that temporarily protects a side chain functional group and is stable under Fmoc deprotection conditions. Selection of this kind of general peptide side chain is known according to the aforementioned Peptide Synthesis and Applications, 2nd Edition; G. B. Fields, R. L. Noble, Solid Phase Peptide Synthesis Utilizing 9-Fluorenylmethoxycarbonyl Amino Acids, Int. J. Peptide Protein Res. 35, 1990, 161-214, and the like; however, preferable peptide side chain protecting groups include, for example, a Boc group and Mtt group for the amino group of the lysine side chain; a *tert-*butyl group for carboxyl groups, such as glutamic acid and aspartic acid; or a Trt group and a Mmt group for the thiol group of cysteine.

The peptides and their analogs described herein may be synthesized by a stepwise method on the solid-phase resin described above. The C-terminal amino acid used and all amino acids and peptides used in the synthesis must have the α-amino protecting group selectively removed in the synthetic process. Preferably, the solid-phase resin described above is used, and once a C-terminal carboxyl group of a peptide having its N-terminal properly protected by Fmoc or the like or a C-terminal carboxyl group of an amino acid having its N-terminal protected by Fmoc is made into an activated ester by an appropriate reagent, this is then added to the amino group on the solid-phase resin to start. Subsequent elongation of the peptide chain may be achieved by removing the N-terminal protecting group (Fmoc group) then successively repeating condensation of the protected amino acid derivative according to the amino acid sequence of the target peptide. Note that these may release the target peptide in a final stage. Examples of releasing conditions are given in Teixeira, W. E. Benckhuijsen, P. E. de Koning, A. R. P. M. Valentijn, J. W. Drijfhout, Protein Pept. Lett., 2002, 9, 379-385, and the like, and the peptide may be released in a TFA solution containing water/silyl hydride/thiol as a scavenger in TFA. Typical examples include TFA/Water/TIS/DODT (volume ratio 92.5:2.5:2.5:2.5).

Synthesis of the peptide analogs described in the present specification may be carried out using a single or multi-channel peptide synthesizer, for example, a Liberty Blue synthesizer from CEM Corporation, a Syro I synthesizer or a successor machine thereof from Biotage Japan, Ltd., or the like.

Activation of the carboxy group may be performed using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazole-1-yloxy)tris(dimethylamino)phosphonium hexafluorophosphate (BOP), 1-[bis(dimethylamino)methyl]-1H-benzotriazolium-3-oxide hexafluorophosphate (HBTU), and the like.

### Conjugate (Complex)

The conjugate of the present invention contains the aforementioned peptide and the antibody or the antigen-binding fragment thereof, and the peptide and the antibody or antigen-binding fragment thereof are preferably linked via a linker. The linker between said peptide-antibody or antigen-binding fragment thereof may be formed by chemical bonding of the linker of the peptide containing the linker (hereinafter also referred to as linker-attached peptide) and the antibody or antigen-binding fragment thereof, occurred in any suitable reactive group, including reactive functional groups.

Since the conjugate of the present invention contains the peptide that has binding ability to hTfR and can pass through the blood-brain barrier, the conjugate of the present invention has binding ability to hTfR and can transport compounds containing the antibody or antigen-binding fragment thereof inside the blood-brain barrier, as shown in the examples.

In addition, since the conjugate of the present invention contains the peptide that has cell-permeability, the conjugate is cell-permeable and can transport compounds containing the antibody or antigen-binding fragments thereof into the cell, as shown in the examples.

Thus, the conjugate may be particularly useful in the prevention or treatment of brain-related diseases.

### Pharmaceutical Composition

Another embodiment disclosed herein relates to pharmaceutical composition. This pharmaceutical composition contains the above-mentioned TfR binding peptide-antibody conjugate, pharmaceutically acceptable salts or solvates thereof (for simplicity, they are hereinafter also referred to simply as a compound containing an antigen or antigen-binding fragment). The pharmaceutical composition preferably contains an effective amount of the above conjugate as an active ingredient.

In the present specification, the form of administration of the pharmaceutical composition is not particularly limited and may be oral or parenteral. Examples of parenteral administration include injection, such as intramuscular injection, intravenous injection, or subcutaneous injection; transdermal administration; transmucosal administration (transnasal, transoral, transocular, transpulmonary, transvaginal, or transrectal); or the like.

The pharmaceutical composition may be modified in various ways, considering a property where a polypeptide is easily metabolized and excreted. For example, polyethylene glycol (PEG) or a sugar chain may be added to the polypeptide to extend its retention time in the blood to reduce antigenicity. Furthermore, bio-degradable polymeric compounds such as polylactic acid and glycol (PLGA), porous hydroxyapatite, liposomes, surface-modified liposomes, emulsions prepared with unsaturated fatty acids, nanoparticles, or nanospheres may be used as sustained release base, and the polypeptide can be encapsulated in them. In the case of transdermal administration, a weak current is allowed to pass through the skin surface and penetrate the stratum corneum (iontophoresis).

The above pharmaceutical compositions may be prepared by using the active ingredients as they are or formulated by adding pharmaceutically acceptable carriers, excipients, additives, or the like. Examples of the dosage form include a liquid agent (for example, an injection), a dispersant, a suspension, a tablet, a pill, a powder, a suppository, a powdered drug, a fine granule, a granule, a capsule, a syrup, a lozenge, an inhalant, an ointment, an eye drop, a nasal drop, an ear drop, a patch, or the like.

The formulation may be carried out by a common method using, for example, an excipient, a binder, a disintegrant, a lubricant, a dissolving agent, a solubilizing agent, a colorant, a flavoring agent, a stabilizer, an emulsifier, an absorption promoter, a surfactant, a pH regulator, a preservative, an antioxidant, or the like as appropriate.

Examples of ingredients used for formulation include but are not limited to purified water, saline, phosphate buffer solution, dextrose, glycerol, a pharmaceutically acceptable organic solvent such as ethanol, animal and vegetable oil, lactose, mannitol, glucose, sorbitol, crystalline cellulose, hydroxypropyl cellulose, starch, corn starch, anhydrous silicic acid, magnesium aluminum silicate, collagen, polyvinyl alcohol, polyvinyl pyrrolidone, carboxyvinyl polymer, sodium carboxymethylcellulose, sodium polyacrylate, sodium alginate, watersoluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohol, stearyl alcohol, stearic acid, human serum albumin, or the like.

The pharmaceutical composition may comprise an absorption promoter for improving absorption of a poorly absorbable drug, in consideration of the fact that it is generally difficult for peptides to be absorbed through mucous membranes. The following may be used as the absorption promoter: a surfactant such as polyoxyethylene lauryl ether, sodium lauryl sulphate, and saponin; a bile salt such as glycocholic acid, deoxycholic acid, and taurocholic acid; a chelating agent such as EDTA and salicylic acid; a fatty acid such as caproic acid, capric acid, lauric acid, oleic acid, linoleic acid, a mixed micelle; an enamine derivative, an N-acyl collagen peptide, an N-acyl amino acid, a cyclodextrin, chitosan, a nitric oxide donor, or the like.

When the pharmaceutical composition is a pill or tablet, it may be coated using a sugar coating, or a gastric-soluble or enteric-coated substance.

When the pharmaceutical composition is an injection, it may comprise distilled water for injection, physiological saline, propylene glycol, polyethylene glycol, vegetable oil, alcohol, or the like. Additionally, a humectant, an emulsifier, a dispersant, a stabilizer, a dissolving agent, a solubilizing agent, a preservative, or the like may be added.

The dosage in the case of administering to a mammal (e.g., human, mouse, rat, guinea pig, rabbit, dog, horse, monkey, pig and the like), especially human, changes depending on symptoms, age of the patient, sex, weight, sensitivity difference, administration method, administration interval, type of active ingredient, and type of formulation, and it may be administered in a non-limiting manner: for example, by administering between 30 µg to 100 g, between 100 µg to 500 mg, or between 100 µg to 100 mg once or divided into several doses. In the case of injection, between 1 µg/kg and 3,000 µg/kg, or between 3 µg/kg and 1,000 µg/kg may be administered once or divided into several doses, according to the bodyweight of the patient.

This specification provides for the use of the conjugate to produce a pharmaceutical for the prevention or treatment of brain-related diseases. The conjugate in this case can be any of those described above.

Another embodiment disclosed in this specification is a method for conjugating the peptide of the present invention with a compound containing an antibody or an antigen-binding fragment thereof, wherein the method contains steps of:
(i) reducing disulfide bond possessed by the compound containing the antibody or the antigen-binding fragment thereof;
(ii) preparing the peptide to which a linker having a maleimide at the terminal is added; and
(iii) contacting the compound containing the antibody or the antigen-binding fragment thereof, in which the disulfide bond is reduced in (i), with the peptide prepared in (ii).

For step (i), since each chain constituting the antibody is bonded by a disulfide bond, the thiol group can be exposed by reducing the disulfide bond. Known reducing agents can be used for the reduction of the disulfide bond, for example, but not limited to, DTT (dithiothreitol), 2-MEA (ethylamine), BME (β-mercaptoethanol), TCEP (tris(2-carboxyethyl) phosphine) can be preferably used.

For step (ii), said linker may be the linker described above. Maleimide can be bound to the terminal of the linker by known method.

For step (iii), the maleimide group reacts with a thiol group to form a stable thioether group in a solution at pH 6.5 to 7.5, which allows binding of the compound containing the aforementioned antibody or the antigen-binding fragment thereof with the aforementioned peptide. Since an antibody usually has multiple disulfide bonds, the above method can be used to bind multiple peptides to a compound containing the target antibody or the antigen-binding fragment thereof.

In addition, another embodiment disclosed in this specification is a method for conjugating the peptide of the present invention with a compound containing an antibody or an antigen-binding fragment thereof, wherein the method contains steps of:
(i) oxidizing a sugar chain possessed by the compound containing the antibody or the antigen-binding fragment thereof;
(ii) preparing the peptide to which a linker having a hydrazide at its terminal is added; and
(iii) contacting the compound containing the antibody or the antigen-binding fragment thereof, in which the sugar chain is oxidized in (i), with the peptide prepared in (ii).

For step (i), it is known that an antibody has a sugar chain, which can be oxidized to generate a carbonyl group. For oxidation, known oxidants can be used, not limiting, but sodium periodate can be preferably used.

For step (ii), the linker may be the linker described above. Hydrazide can be bound to the terminal of the linker by known method.

For step (iii), the hydrazide group reacts with a carbonyl group to form a stable hydrazine bond in a solution at pH 5 to 7, which allows binding of the compound containing the aforementioned antibody or the antigen-binding fragment thereof with the aforementioned peptide.

Moreover, another embodiment disclosed in this specification is a method for conjugating the peptide of the present invention with a compound containing an antibody or an antigen-binding fragment thereof, wherein the method contains steps of:
(i) preparing the peptide to which a linker having N-hydroxysuccinimide (NHS) at its terminal is added; and
(ii) contacting the compound containing the antibody or the antigen-binding fragment thereof with the peptide prepared in (i).

For step (i), said linker may be the linker described above. NHS can be bound to the terminal of the linker by known method.

For step (ii), since NHS and amine react efficiently at pH above neutral to form an amide bond, the peptide can be bound to the compound containing the target antibody or antigen-binding fragment thereof by binding the NHS of the peptide-linker to the amine possessed by the antibody.

This specification also provides a method for producing a peptide-antibody conjugate using the binding method described above.

### Abbreviation

Fmoc as 9-fluorenylmethyloxycarbonyl;
HOAt as 1-hydroxybenzotriazole;
HATU as O-(7-azabenzotriazol-1-yl)-N,N,N',N'-tetramethyluronium hexafluorophosphate; MeCN as acetonitrile;
Ac as acetyl;
BSA as bovine serum albumin;
CIAc as chloroacetyl;
CO₂ as carbon dioxide;
DBU as 1, 8-diazabicyclo "5.4.0"-7-undecene;
DIPEA or DIEA as N,N-diisopropylethylamine;
DODT as 3, 6-dioxa-1,8-octane-dithiol;
DMSO as dimethylsulfoxide;
DMF as N,N-dimethylformamide;
EDC as ethylene dichloride;
EDTA as ethylenediaminetetraacetic acid;
FBS as fetal bovine serum;
HEPES as hydroxyethylpiperazine ethanesulfonic acid;
IC50 as 50% inhibitory concentration;
Mtt as monomethyltrityl;
Mmt as monomethoxytrityl;
o-Ns as 2-nitrobenzenesulfonyl;
TFA as trifluoroacetic acid;
TIS as triisopropylsilane;
Trt as trityl
TCEP as tris(2-carboxyethyl)phosphine;
mL as milliliter (unit);
M as moler (unit);
V/V as volume/volume;
DIPCI as N,N'-diisopropylcarbodiimide
Oxyma pure as ethyl cyano(hydroxyimino)acetate;
PBS as phosphate buffered saline;
PBST as phosphate buffered saline-Tween 20;
AA as amino acid;
HOSu as N-hydroxysuccinimide;
DCM as dichloromethane;
CIAcOSu as N-(chloroacetoxy)succinimide;
SMCC as succinimidyl 4-(N-maleimidomethyl)cyclohexane-1-carboxylate;
mM as millimoler (unit);
µm as micrometer (unit);
mm as millimeter (unit);
nm as nanometer (unit);
Å as angstrom (unit);
min as minute (unit);
MS as a mass spectrometry;
conc as a concentration;
mmol as millimol (unit);
mg as milligram (unit);
rpm as revolution per minute (unit);
h as hour (unit);
G as gravitational acceleration (unit);
HPLC as high-performance liquid chromatography;
LC-MS or LC/MS as liquid chromatography-mass spectrometer;
DMAP as 4-dimethylaminopyridine;
1-(3-dimethylaminopropyl)-3-ethylcarbodiimide hydrochloride as EDCI • HCl
hydrazide Hydrazide
NHS ester or NHS as N-hydroxysuccinimide
PEG4c or PEG3 or PEG4 as 1-amino-3,6,9,12-tetraoxapentadecan-15-oic acid
PEG8c as 1-amino-3,6,9,12,15,18,21,24-octaoxaheptacosane-27-oic acid
PEG12c or PEG11 or PEG12 as 1-amino-3,6,9,12,15,18,21,24,27,30,33,36-dodecaoxanonatriacontane-39-oic acid
PEG36 as 1-amino-3,6,9,12,15,18,21,24,27,30,33,36,39,42,45,48,51,54,57,60,63,66, 69,72,75,78,81,84,87,90,93,96,99,102,105,108-hexatriacontaoxaundecahectanoic acid

### Examples

The present invention is further described in detail by the following reference examples and examples, which do not limit the invention and may be modified without departing from the scope of the invention.

In this specification, the international application number PCT/JP2021/006709 (International Publication No. WO2021-167107 pamphlet; unpublished at the time of filing the base application), in which the applicant is identical to this application, is cited.

### Chemical Synthesis

Synthesis may be completed using commercially available products as is for all raw materials, building blocks, reagents, acids, bases, solid-phase resins, and solvents used in chemical synthesis in the following examples, or by a person having ordinary skill in the art using organic chemistry techniques. Note that commercial products were used for amino acids containing protecting groups unless otherwise specified.

The general methods for synthesis, cyclization, purification, and analysis of peptides are shown below, but the conditions can be changed as appropriate depending on the sequence and other factors. If the method is described in the examples, the method was taken.

Elongation of the peptide chain in a solid-phase resin is performed by using the resin described in each example as a starting material and using a standard peptide coupling reaction condition and Fmoc removal reaction condition. Reactions were carried out using Liberty Blue, an automated peptide synthesizer manufactured by CEM, in accordance with the manufacturer's manual. As an example, some of the common amino acids used are listed below, and side chain protecting groups are shown in parentheses.

Fmoc-Trp(Boc)-OH; Fmoc-Thr(tBu)-OH; Fmoc-N-Me-Gly-OH; Fmoc-Asp(OtBu)-OH; Fmoc-N-Me-Phe-OH; Fmoc-Ala-OH; Fmoc-N-Me-Ala-OH; Fmoc-His(Trt)-OH; Fmoc-Tyr(tBu)-OH; Fmoc-Val-OH; Fmoc-HydPro(tBu)-OH; Fmoc-Cys(Trt)-OH; Fmoc-Lys(Mtt)-OH; Fmoc-Ser(tBu)-OH; and Fmoc-N-Me-Ser(tBu)-OH.

Introduction of the acetyl group was performed by removing the Fmoc group of the α-amino group from the solid-phase resin holding the Fmoc-protected peptide obtained in the previous step by the method described above, and then adding chloroacetic acid (3 equivalents) to a DMF solution of 3 equivalents of N,N'-diropropylcarbodiimide (0.5 M) and a DMF solution of 3 equivalents of HOAt (0.5 M) and shaking at room temperature for 40 min.

For deprotection of side chains and cut-out from the solid-phase resin, the resin obtained after the chloroacetyl group introduction step was first washed five times each with DMF and methylene chloride, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin and shaken at room temperature for 150 min. The reaction solution was collected by filtration through a frit. The solid-phase resin remaining in the reaction vessel was shaken again with the cut-out cocktail, and the solution component was collected from the frit and mixed with the aforementioned filtrate. When this filtrate was added to excess diethyl ether cooled to 0°C, a cloudy precipitate was formed. The mixture was centrifuged (9000 rpm, 3 min) and the solution was decanted. The resulting solid was washed again with a small amount of diethyl ether cooled to 0°C, and the resulting solid was used for the next cyclization reaction.

The peptide cyclization reaction was performed by dissolving the peptide in DMSO so that the final concentration of peptide was 5 mM based on the number of moles of solid-phase resin, adding 6 or 7 equivalents of triethylamine, and stirring at room temperature for about 16 hours. The resulting reaction solution was acidified with acetic acid and concentrated under reduced pressure using Biotage (registered trademark) V-10 (manufactured by Biotage Japan).

Reversed-phase separation HPLC was carried out as the method for purifying the obtained crude purified peptide using an AutoPurification System-SQD2 single quadruple mass spectrometer, manufactured by Waters, and elution was performed while monitoring m/z ions derived from the target product. It was confirmed that the mass spectrum obtained in ESI-positive scanning mode and the mass spectrum containing polyvalent ions calculated by the molecular formula of the target product matched within the error range of the mass spectrometer used. Note that the purification conditions including the columns used are shown in the respective examples.

As for the structure determination of chemically synthesized peptides, the molecular weight calculated in consideration of the amino acid used according to the target sequence and the building block used as necessary was confirmed by ESI-MS(+) in the mass spectrum analysis method. Note that "ESI-MS(+)" indicates an electrospray ionization mass spectrometry method performed in positive ion mode. The detected mass is reported in "m/z" units. Note that compounds having a molecular weight greater than approximately 1,000 are frequently detected as bivalent ions or trivalent ions. The analysis method are shown in the respective examples.

### [Example 1]

In this example, peptides that bind to hTfR were synthesized. The synthesized peptides are listed in TABLE 1. In addition, only the linker sequence was taken out from TABLE 1 and described in TABLE 5. Synthesis was performed in the same manner as described above and in Example 2. The 894_Bicycle_002_GGRGRS_K (Mal) in SEQ ID NO: 23 has a bicyclic structure, in which the 1st Ala residue and the 15th Cys are cyclized, and the 2nd Hgl residue and the 11th Hly residue are joined. The synthesized peptides were analyzed under the analyzing conditions described in each example, and the structure was confirmed by ESI-MS(+) in mass spectrometry. The obtained ESI-MS(+) observation and the value of X when expressed as the number of proton additions (M+XH)X+ in such cases are shown in TABLE 1.

Naked peptides (peptides without linker part) consisting of 1st to 15th amino acid sequences of the peptides listed in TABLE 1 (i.e., peptides having amino acid sequences of SEQ ID NOs: 1 to 110) were synthesized as described in Example 7 of International Application No. PCT/JP2021/006709 (International Publication No. WO2021-167107 pamphlet), and their binding ability to hTfR were confirmed by SPR. The KD values thus obtained in these SPR measurements are shown in TABLE 1, denoted A for KD values less than 1 nM, B for KD values between 1 nM and 100 nM, C for KD values between 100 nM and 1 mM, and D for KD values greater than 1 mM. The results are listed in columns of TABLE 1 for peptides with the corresponding naked peptide. Note that ND indicates No Data (here, it means that no data was obtained). For SEQ ID NOs: 104-107, SPR measurements were performed for those the linker was bound, and all of them were confirmed to have the binding ability to hTfR. As a result, it was confirmed that all of the naked peptides listed in TABLE 1 have hTfR binding ability. Furthermore, the peptide 894_3m_PEG12_dk (Maleimide) listed in TABLE 1 (peptide of SEQ ID NO: 36 with an additional linker sequence of SEQ ID NO: 114) was confirmed to have binding ability to hTfR by SPR in the same way as above, and it fell into category B of the above classification. This indicates that the binding ability to hTfR can be maintained even when a linker is attached to the hTfR-binding peptide (Naked).

**[TABLE 1]**

| [TABLE 1-1-1] | | | | | | | | | | |
|---|---|---|---|---|---|---|---|---|---|---|
| peptide name | SEQ ID No. | | | | | | | | | |
| | | 1 | 2 | 3 | 4 | 5 | 6 | 7 | 8 | 9 |
| hTfR_000894_PEG11_K(Maleimide) | 1 | A | V | F | V | W | N | Y | Y | I |
| hTfR_000894_PEG36_K(Maleimide) | 2 | A | V | F | V | W | N | Y | Y | I |
| hTfR_894_A11PEG11K(Maleimide) | 3 | A | V | F | V | W | N | Y | Y | I |
| JCR_hTfR_000894_PEG36_(NHS) | 4 | A | V | F | V | W | N | Y | Y | I |
| hTfR_894_A11PEG11(NHS) | 5 | A | V | F | V | W | N | Y | Y | I |
| hTfR_000894_PEG11_(Hydradine) | 6 | A | V | F | V | W | N | Y | Y | I |
| hTfR_000894_PEG36_(Hydrazine) | 7 | A | V | F | V | W | N | Y | Y | I |
| hTfR_894_A11PEG11(Hydrazine) | 8 | A | V | F | V | W | N | Y | Y | I |
| hTfR_894_E12_PEG12_(Hydrazide) | 9 | A | V | F | V | W | N | Y | Y | I |
| hTfR_894_E11E12_PEG12_Hydrazide | 10 | A | V | F | V | W | N | Y | Y | I |
| hTfR_894_(GE)PEG12_Hydrazide | 11 | A | V | F | V | W | N | Y | Y | I |
| hTfR_894_(GEGE)PEG12_Hydrazide | 12 | A | V | F | V | W | N | Y | Y | I |
| hTfR_000894_PEG4_(Hydrazide) | 13 | A | V | F | V | W | N | Y | Y | I |
| 36_G4S2_gAbu(NHS) | 14 | A | V | F | V | W | N | Y | Y | I |
| 894_3m_G4S2_gAbu(NHS) | 15 | A | V | MeF | V | W | N | Y | Y | I |
| 36_GG_gAbu(NHS) | 16 | A | V | F | V | W | N | Y | Y | I |
| 36_G4S2_gAbu(NHS) | 17 | A | V | F | V | W | N | Y | Y | I |
| 894_3m_GG_gAbu(NHS) | 18 | A | V | MeF | V | W | N | Y | Y | I |
| 894_PEG12_(NHS) | 19 | A | V | F | V | W | N | Y | Y | I |
| 894_3m_10A_GGRGRS_K(Mal) | 20 | A | V | MeF | V | W | N | Y | Y | I |
| 894_3m_6A_GGRGRS_K(Mal) | 21 | A | V | MeF | V | W | A | Y | Y | I |
| 894 variant 03 GGRGRS_K(Mal) | 22 | A | V | MeF3C | V | MeW | N | Y | F4OMe | I |
| 894_Bicycle_002_GGRGRS_K(Mal) | 23 | A | Hgl | MeF3C | V | MeW | N | Y | Y | I |
| 894_3m_G_PEG12_gAbu(NHS) | 24 | A | V | MeF | V | W | N | Y | Y | I |
| 894_3m_G_PEG4_gAbu(NHS) | 25 | A | V | MeF | V | W | N | Y | Y | I |
| 894_3m_G_PEG4x3_gAbu(NHS) | 26 | A | V | MeF | V | W | N | Y | Y | I |
| 894_3m_GGRGRS_gAbu(NHS) | 27 | A | V | MeF | V | W | N | Y | Y | I |
| 894_3m_G_PEG4_R_PEG4_R_PEG4_gAbu(NHS) | 28 | A | V | MeF | V | W | N | Y | Y | I |
| 894_3m_GGQGQS_gAbu(NHS) | 29 | A | V | MeF | V | W | N | Y | Y | I |
| 894_3m_G4S2x2_gAbu(NHS) | 30 | A | V | MeF | V | W | N | Y | Y | I |

**[TABLE 1-1-2]**

| | | | | | | | | | | | Linker SEQ |
|---|---|---|---|---|---|---|---|---|---|---|---|
| 10 | 11 | 12 | 13 | 14 | 15 | | | | | | |
| I | R | R | Y | MeY | C | PEG12 | K(Maleimide) | | | | |
| I | R | R | Y | MeY | C | PEG36 | K(Maleimide) | | | | |
| I | A | R | Y | MeY | C | PEG12 | K(Maleimide) | | | | |
| I | R | R | Y | MeY | C | PEG36 | NHS ester | | | | |
| I | A | R | Y | MeY | C | PEG12 | NHS ester | | | | |
| I | R | R | Y | MeY | C | PEG12 | Hydrazide | | | | |
| I | R | R | Y | MeY | C | PEG36 | Hydrazide | | | | |
| I | A | R | Y | MeY | C | PEG12 | Hydrazide | | | | |
| I | A | E | Y | MeY | C | PEG12 | Hydrazide | | | | |
| I | E | E | Y | MeY | C | PEG12 | Hydrazide | | | | |
| I | R | R | Y | MeY | C | G | E | PEG12 | Hydrazide | | |
| I | R | R | Y | MeY | C | G | E | G | E | PEG12 | Hydrazide |
| I | R | R | Y | MeY | C | PEG4 | Hydrazide | | | | |
| I | S | C | | | | G | G | G | G | S | S gAbu |
| I | R | R | Y | MeY | C | G | G | G | G | S | S gAbu |
| I | S | C | | | | G | G | gAbu | NHS | | |
| I | S | C | | | | G | G | G | G | S | S gAbu |
| I | R | R | Y | MeY | C | G | G | gAbu | NHS | | |
| I | R | R | Y | MeY | C | PEG12 | NHS | | | | |
| A | R | R | Y | MeY | C | G | G | R | G | R | S K(Maleimide) |
| I | R | R | Y | MeY | C | G | G | R | G | R | S K(Maleimide) |
| I | R | dr | F | MeY | C | G | G | R | G | R | S K(Maleimide) |
| I | Hly | R | Y | MeY | C | G | G | R | G | R | S K(Maleimide) |
| I | R | R | Y | MeY | C | G | PEG12c | gAbu | NHS | | |
| I | R | R | Y | MeY | C | G | PEG4c | gAbu | NHS | | |
| I | R | R | Y | MeY | C | G | PEG4c | PEG4c | PEG4c | gAbu | NHS |
| I | R | R | Y | MeY | C | G | G | R | G | R | S gAbu |
| I | R | R | Y | MeY | C | G | PEG4c | R | PEG4c | R | PEG4c gAbu |
| I | R | R | Y | MeY | C | G | G | Q | G | Q | S gAbu |
| I | R | R | Y | MeY | C | G | G | G | G | S | S G |

**[TABLE 1-1-3]**

| | | | | | | | m/z | [M+XH]X* | Reactive functional group of linker | SPR of naked peptide |
|---|---|---|---|---|---|---|---|---|---|---|
| | | | | | | | 1010.68 | 3 | Maleimide | A |
| | | | | | | | 1363.01 | 3 | Maleimide | A |
| | | | | | | | 982.33 | 3 | Maleimide | B |
| | | | | | | | 1279.87 | 3 | NHS | A |
| | | | | | | | 1348.37 | 2 | NHS | B |
| | | | | | | | 899.92 | 3 | Hydrazide | A |
| | | | | | | | 1252.15 | 3 | Hydrazide | A |
| | | | | | | | 1306.86 | 2 | Hydrazide | B |
| | | | | | | | 1293.37 | 2 | Hydrazide | B |
| | | | | | | | 1322.37 | 2 | Hydrazide | ND |
| | | | | | | | 962.01 | 3 | Hydrazide | A |
| | | | | | | | 1024 | 3 | Hydrazide | A |
| | | | | | | | 782.47 | 3 | Hydrazide | A |
| NHS | | | | | | | 1051.98 | 2 | NHS | B |
| NHS | | | | | | | 1341.69 | 2 | NHS | B |
| | | | | | | | 907.83 | 2 | NHS | B |
| NHS | | | | | | | 1051.98 | 2 | NHS | B |
| | | | | | | | 1197.63 | 2 | NHS | B |
| | | | | | | | 1390.89 | 2 | NHS | A |
| -NH2 | | | | | | | 991.78 | 3 | Maleimide | B |
| -NH2 | | | | | | | 991.5 | 3 | Maleimide | C |
| -NH2 | | | | | | | 1021.17 | 3 | Maleimide | B |
| -NH2 | | | | | | | 1025.81 | 3 | Maleimide | B |
| | | | | | | | 979.76 | 3 | NHS | B |
| | | | | | | | 1292.86 | 2 | NHS | B |
| | | | | | | | 1027.18 | 3 | NHS | B |
| NHS | | | | | | | 951.01 | 3 | NHS | B |
| NHS | | | | | | | 1131.22 | 3 | NHS | B |
| NHS | | | | | | | 1397.8 | 2 | NHS | B |
| G | G | G | S | S | gAbu | NHS | 1028.95 | 3 | NHS | B |

**[TABLE 1-2-1]**

| | | | | | | | | | |
|---|---|---|---|---|---|---|---|---|---|
| 894_3m_GGRGRSx2_gAbu(NHS) | 31 | A | V | MeF | V | W | N | Y | Y |
| 894_3m_GGQGQSx2_gAbu(NHS) | 32 | A | V | MeF | V | W | N | Y | Y |
| 894_G4S2_K(Mal) | 33 | A | V | F | V | W | N | Y | Y |
| 894_3m_PEG04_K(Mal) | 34 | A | V | MeF | V | W | N | Y | Y |
| 894_3m_PEG08_K(Mal) | 35 | A | V | MeF | V | W | N | Y | Y |
| 894_3m_PEG12_K(Mal) | 36 | A | V | MeF | V | W | N | Y | Y |
| 894_3m_G4S2_K(Mal) | 37 | A | V | MeF | V | W | N | Y | Y |
| 894_5m_PEG04_K(Mal) | 38 | A | V | F | V | MeW | N | Y | Y |
| 894_5m_PEG08_K(Mal) | 39 | A | V | F | V | MeW | N | Y | Y |
| 894_5m_PEG12_K(Mal) | 40 | A | V | F | V | MeW | N | Y | Y |
| 894_5m_G4S2_K(Mal) | 41 | A | V | F | V | MeW | N | Y | Y |
| 894_3_5m_PEG04_K(Mal) | 42 | A | V | MeF | V | MeW | N | Y | Y |
| 894_3_5m_PEG08_K(Mal) | 43 | A | V | MeF | V | MeW | N | Y | Y |
| 894_3_5m_PEG12_K(Mal) | 44 | A | V | MeF | V | MeW | N | Y | Y |
| 894_3_5m_G4S2_K(Mal) | 45 | A | V | MeF | V | MeW | N | Y | Y |
| 894_K11_PEG4c_KTrzMal | 46 | A | V | F | V | W | N | Y | Y |
| 894_K11_PEG8c_KTrzMal | 47 | A | V | F | V | W | N | Y | Y |
| 894_K11_PEG12c_KTrzMal | 48 | A | V | F | V | W | N | Y | Y |
| 894_K11_G4S2_KTrzMal | 49 | A | V | F | V | W | N | Y | Y |
| 894_11K_3Me_PEG4c_KTrzMal | 50 | A | V | MeF | V | W | N | Y | Y |
| 894_11K_3MeF_PEG8c_KTrzMal | 51 | A | V | MeF | V | W | N | Y | Y |
| 894_11K_3MeF_PEG12c_KTrzMal | 52 | A | V | MeF | V | W | N | Y | Y |
| 894_11K_3MeF_G4S2_KTrzMal | 53 | A | V | MeF | V | W | N | Y | Y |
| 894_11K_3Me_5Me_PEG4c_KTrzMal | 54 | A | V | MeF | V | MeW | N | Y | Y |
| 894_11K_3Me_5Me_PEG8c_KTrzMal | 55 | A | V | MeF | V | MeW | N | Y | Y |
| 894_11K_3Me_5Me_PEG12c_KTrzMal | 56 | A | V | MeF | V | MeW | N | Y | Y |
| 894_11K_3Me_5Me_G4S2_KTrzMal | 57 | A | V | MeF | V | MeW | N | Y | Y |
| 894_3m_G2SGSG2SGS_K(Mal) | 58 | A | V | MeF | V | W | N | Y | Y |
| 894_3m_G2SGSGSS_K(Mal) | 59 | A | V | MeF | V | W | N | Y | Y |
| 894_3m_GGSGSS_K(Mal) | 60 | A | V | MeF | V | W | N | Y | Y |

**[TABLE 1-2-2]**

| | | | | | | | | | | | |
|---|---|---|---|---|---|---|---|---|---|---|---|
| R | R | Y | MeY | C | G | G | R | G | R | S | G |
| R | R | Y | MeY | C | G | G | Q | G | Q | S | G |
| R | R | Y | MeY | C | G | G | G | G | S | S | **K(Maleimide)** |
| R | R | Y | MeY | C | PEG4c | K(Maleimide) | -NH2 | | | | |
| R | R | Y | MeY | C | PEG8c | K(Maleimide) | -NH2 | | | | |
| R | R | Y | MeY | C | PEG12c | K(Maleimide) | -NH2 | | | | |
| R | R | Y | MeY | C | G | G | G | G | S | S | **K(Maleimide)** |
| R | R | Y | MeY | C | PEG4c | K(Maleimide) | -NH2 | | | | |
| R | R | Y | MeY | C | PEG8c | K(Maleimide) | -NH2 | | | | |
| R | R | Y | MeY | C | PEG12c | K(Maleimide) | -NH2 | | | | |
| R | R | Y | MeY | C | G | G | G | G | S | S | **K(Maleimide)** |
| R | R | Y | MeY | C | PEG4c | K(Maleimide) | -NH2 | | | | |
| R | R | Y | MeY | C | PEG8c | K(Maleimide) | -NH2 | | | | |
| R | R | Y | MeY | C | PEG12c | K(Maleimide) | -NH2 | | | | |
| R | R | Y | MeY | C | G | G | G | G | S | S | **K(Maleimide)** |
| K | R | Y | MeY | C | PEG4c | KTrzMal | -NH2 | | | | |
| K | R | Y | MeY | C | PEG8c | KTrzMal | -NH2 | | | | |
| K | R | Y | MeY | C | PEG12c | KTrzMal | -NH2 | | | | |
| K | R | Y | MeY | C | G | G | G | G | S | S | KTrzMal |
| K | R | Y | MeY | C | PEG4c | KTrzMal | -NH2 | | | | |
| K | R | Y | MeY | C | PEG8c | KTrzMal | -NH2 | | | | |
| K | R | Y | MeY | C | PEG12c | KTrzMal | -NH2 | | | | |
| K | R | Y | MeY | C | G | G | G | G | S | S | KTrzMal |
| K | R | Y | MeY | C | PEG4c | KTrzMal | -NH2 | | | | |
| K | R | Y | MeY | C | PEG8c | KTrzMal | -NH2 | | | | |
| K | R | Y | MeY | C | PEG12c | KTrzMal | -NH2 | | | | |
| K | R | Y | MeY | C | G | G | G | G | S | S | KTrzMal |
| R | R | Y | MeY | C | G | G | S | G | S | S | G |
| R | R | Y | MeY | C | G | G | S | G | S | S | G |
| R | R | Y | MeY | C | G | G | S | G | S | S | **K(Maleimide)** |

**[TABLE 1-2-3]**

| | | | | | | | | | | |
|---|---|---|---|---|---|---|---|---|---|---|
| G | R | G | R | S | gAbu | NHS | 856.11 | 4 | NHS | B |
| G | Q | G | Q | S | gAbu | NHS | 1103.65 | 3 | NHS | B |
| -NH2 | | | | | | | 1416.95 | 2 | Maleimide | A |
| | | | | | | | 898.07 | 3 | Maleimide | B |
| | | | | | | | 956.79 | 3 | Maleimide | B |
| | | | | | | | 1015.47 | 3 | Maleimide | B |
| -NH2 | | | | | | | 1423.95 | 2 | Maleimide | B |
| | | | | | | | 1346.4 | 2 | Maleimide | A |
| | | | | | | | 956.76 | 3 | Maleimide | A |
| | | | | | | | 1015.71 | 3 | Maleimide | A |
| -NH2 | | | | | | | 949.78 | 3 | Maleimide | A |
| | | | | | | | 1353.42 | 2 | Maleimide | B |
| | | | | | | | 961.42 | 3 | Maleimide | B |
| | | | | | | | 1020.36 | 3 | Maleimide | B |
| -NH2 | | | | | | | 1430.8 | 2 | Maleimide | B |
| | | | | | | | 1296.29 | 2 | Maleimide | A |
| | | | | | | | 1384.4 | 2 | Maleimide | A |
| | | | | | | | 982.05 | 3 | Maleimide | A |
| -NH2 | | | | | | | 1373.74 | 2 | Maleimide | A |
| | | | | | | | 1303.33 | 2 | Maleimide | B |
| | | | | | | | 928 | 3 | Maleimide | B |
| | | | | | | | 986.71 | 3 | Maleimide | B |
| -NH2 | | | | | | | 1380.72 | 2 | Maleimide | B |
| | | | | | | | 873.96 | 3 | Maleimide | B |
| | | | | | | | 932.71 | 3 | Maleimide | B |
| | | | | | | | 991.38 | 3 | Maleimide | B |
| -NH2 | | | | | | | 1387.75 | 2 | Maleimide | B |
| G | S | G | S | S | K(Maleimide) | -NH2 | 1103.76 | 3 | Maleimide | B |
| S | S | K(Maleimide) | -NH2 | | | | 1036.67 | 3 | Maleimide | B |
| -NH2 | | | | | | | 1438.78 | 2 | Maleimide | B |

**[TABLE 1-3-1]**

| | | | | | | | | | |
|---|---|---|---|---|---|---|---|---|---|
| 894_3m_GmGSmGSS_K(Mal) | 61 | A | V | MeF | V | W | N | Y | Y |
| 894_3m_GGSGES_K(Mal) | 62 | A | V | MeF | V | W | N | Y | Y |
| 894_3m_GGEGES_K(Mal) | 63 | A | V | MeF | V | W | N | Y | Y |
| 894_3m_GGGSS_K(Mal) | 64 | A | V | MeF | V | W | N | Y | Y |
| 894_3m_GGSS_K(Mal) | 65 | A | V | MeF | V | W | N | Y | Y |
| 894_3m_GGS_K(Mal) | 66 | A | V | MeF | V | W | N | Y | Y |
| 894_3m_GG_K(Mal) | 67 | A | V | MeF | V | W | N | Y | Y |
| 894_3m_G_K(Mal) | 68 | A | V | MeF | V | W | N | Y | Y |
| 894_3m_K(Mal) | 69 | A | V | MeF | V | W | N | Y | Y |
| 894_3m_PEG4_PEG4_K(Mal) | 70 | A | V | MeF | V | W | N | Y | Y |
| 894_3m_PEG4_PEG4_PEG4_K(Mal) | 71 | A | V | MeF | V | W | N | Y | Y |
| 894_5m_GSSGSS_K(Mal) | 72 | A | V | F | V | MeW | N | Y | Y |
| 894_5m_GGRGRS_K(Mal) | 73 | A | V | F | V | MeW | N | Y | Y |
| 894_5m_GGSGES_K(Mal) | 74 | A | V | F | V | MeW | N | Y | Y |
| 894_5m_GGRSES_K(Mal) | 75 | A | V | F | V | MeW | N | Y | Y |
| 894_5m_GGESES_K(Mal) | 76 | A | V | F | V | MeW | N | Y | Y |
| 894_3m_5m_GGSGES_K(Mal) | 77 | A | V | MeF | V | MeW | N | Y | Y |
| 894_3m_5m_GGESES_K(Mal) | 78 | A | V | MeF | V | MeW | N | Y | Y |
| 894_3m_5m_GGSGRS_K(Mal) | 79 | A | V | MeF | V | MeW | N | Y | Y |
| 894_3m_5m_GGRGRS_K(Mal) | 80 | A | V | MeF | V | MeW | N | Y | Y |
| 894_3m_5m_GGRSES_K(Mal) | 81 | A | V | MeF | V | MeW | N | Y | Y |
| 894_3m_GGRGRS_K(Mal) | 82 | A | V | MeF | V | W | N | Y | Y |
| 894_5m_GG_K(Mal) | 83 | A | V | F | V | MeW | N | Y | Y |
| 894_5m_GGRGRS_K(Mal) | 84 | A | V | F | V | MeW | N | Y | Y |
| 894_3m_5m_GG_K(Mal) | 85 | A | V | MeF | V | MeW | N | Y | Y |
| 894_3m_5m_GGRGRS_K(Mal) | 86 | A | V | MeF | V | MeW | N | Y | Y |
| 36_GG_K(Mal) | 87 | A | V | F | V | W | N | Y | Y |
| 36_GGRGRS_K(Mal) | 88 | A | V | F | V | W | N | Y | Y |
| 36_G4S2_K(Ma)) | 89 | A | V | F | V | W | N | Y | Y |
| 894_3m_1Abu_GG_K(Mal) | 90 | Abu | V | MeF | V | W | N | Y | Y |

**[TABLE 1-3-2]**

| | | | | | | | | | | | |
|---|---|---|---|---|---|---|---|---|---|---|---|
| R | R | Y | MeY | C | G | MeG | S | MeG | S | S | K(Maleimide) |
| R | R | Y | MeY | C | G | G | S | G | E | S | K(Maleimide) |
| R | R | Y | MeY | C | G | G | E | S | E | S | K(Maleimide) |
| R | R | Y | MeY | C | G | G | G | S | S | K(Maleimide) | -NH2 |
| R | R | Y | MeY | C | G | G | S | S | K(Maleimide) | -NH2 | |
| R | R | Y | MeY | C | G | G | S | K(Maleimide) | -NH2 | | |
| R | R | Y | MeY | C | G | G | K(Maleimide) | -NH2 | | | |
| R | R | Y | MeY | C | G | K(Maleimide) | -NH2 | | | | |
| R | R | Y | MeY | C | K(Maleimide) | -NH2 | | | | | |
| R | R | Y | MeY | C | PEG4c | PEG4c | K(Maleimide) | -NH2 | | | |
| R | R | Y | MeY | C | PEG4c | PEG4c | PEG4c | K(Maleimide) | -NH2 | | |
| R | R | Y | MeY | C | G | S | S | G | S | S | K(Malelmide) |
| R | R | Y | MeY | C | G | G | R | G | R | S | K(Maleimide) |
| R | R | Y | MeY | C | G | G | S | G | E | S | K(Malaimide) |
| R | R | Y | MeY | C | G | G | R | S | E | S | K(Maleimide) |
| R | R | Y | MeY | C | G | G | E | S | E | S | K(Maleimide) |
| R | R | Y | MeY | C | G | G | S | G | E | S | K(Maleimide) |
| R | R | Y | MeY | C | G | G | E | S | E | S | K(Malelmlde) |
| R | R | Y | MeY | C | G | G | S | G | R | S | K(Maleimide) |
| R | R | Y | MeY | C | G | G | R | G | R | S | K(Maleimide) |
| R | R | Y | MeY | C | G | G | R | S | E | S | K(Maleimide) |
| R | R | Y | MeY | C | G | G | R | G | R | S | K(Maleimide) |
| R | R | Y | MeY | C | G | G | K(Maleimide) | -NH2 | | | |
| R | R | Y | MeY | C | G | G | R | G | R | S | K(Malelmide) |
| R | R | Y | MeY | C | G | G | K(Maleimide) | -NH2 | | | |
| R | R | Y | MeY | C | G | G | R | G | R | S | K(Maleimide) |
| S | C | | | | G | G | K(Maleimide) | -NH2 | | | |
| S | C | | | | G | G | R | G | R | S | K(Malelmlde) |
| S | C | | | | G | G | G | G | S | S | K(Maleimide) |
| R | R | Y | MeY | C | G | G | K(Maleimide) | -NH2 | | | |

**[TABLE 1-3-3]**

| | | | | |
|---|---|---|---|---|
| -NH2 | 968.91 | 3 | Maleimide | B |
| -NH2 | 1460 | 2 | Maleimide | B |
| -NH2 | 1496.04 | 2 | Maleimide | B |
| | 1395.41 | 2 | Maleimide | B |
| | 911.85 | 3 | Maleimide | B |
| | 882.79 | 3 | Maleimide | B |
| | 853.81 | 3 | Maleimide | B |
| | 1251.26 | 2 | Maleimide | B |
| | 1222.77 | 2 | Maleimide | B |
| | 980.38 | 3 | Maleimide | B |
| | 1062.81 | 3 | Maleimide | B |
| -NH2 | 1453.99 | 2 | Maleimide | A |
| -NH2 | 1005.65 | 3 | Maleimide | A |
| -NH2 | 1459.81 | 2 | Maleimide | A |
| -NH2 | 1006.63 | 3 | Maleimide | A |
| -NH2 | 997.65 | 3 | Maleimide | A |
| -NH2 | 1466.79 | 2 | Maleimide | B |
| -NH2 | 1002.31 | 3 | Maleimide | B |
| -NH2 | 987.26 | 3 | Maleimide | B |
| -NH2 | 1010.35 | 3 | Maleimide | B |
| -NH2 | 1011.29 | 3 | Maleimide | B |
| -NH2 | 1005.73 | 3 | Maleimide | B |
| | 1279.6 | 2 | Maleimide | A |
| -NH2 | 1005.65 | 3 | Maleimide | A |
| | 1286.97 | 2 | Maleimide | B |
| -NH2 | 1010.35 | 3 | Maleimide | B |
| | 989.98 | 2 | Maleimide | B |
| -NH2 | 1218.21 | 2 | Maleimide | B |
| -NH2 | 1133.96 | 2 | Maleimide | B |
| | 1286.76 | 2 | Maleimide | B |

**[TABLE 1-4-1]**

| | | | | | | | | | |
|---|---|---|---|---|---|---|---|---|---|
| 894_3m_1Abu_GGRGRS_K(Mal) | 91 | Abu | V | MeF | V | W | N | Y | Y |
| 894_3m_1Abu_G4S2_K(Mal) | 92 | Abu | V | MeF | V | W | N | Y | Y |
| 894_3m_8Ahp_GG_K(Mal) | 93 | A | V | MeF | V | W | N | Y | Ahp |
| 894^{_}3m_8Ah_GGRGRS_K(Mal) | 94 | A | V | MeF | V | W | N | Y | Ahp |
| 894_3m_8Ah_G4S2_K(Mal) | 95 | A | V | MeF | V | W | N | Y | Ahp |
| 894_3m_Linke01_dk(Mal) | 96 | A | V | MeF | V | W | N | Y | Y |
| 894_3m_Linke02_dk(Mal) | 97 | A | V | MeF | V | W | N | Y | Y |
| 894_3m_Linke03_dk(Mal) | 98 | A | V | MeF | V | W | N | Y | Y |
| 894_3m_Linke04_dk(Mal) | 99 | A | V | MeF | V | W | N | Y | Y |
| 894_v03_Linke01_dk(Mal) | 100 | A | V | MeF3C | V | MeW | N | Y | F4OMe |
| 894_v03_Linke02 dk(Mal) | 101 | A | V | MeF3C | V | MeW | N | Y | F4OMe |
| 894_v03_Linke03_dk(Mal) | 102 | A | V | MeF3C | V | MeW | N | Y | F4OMe |
| 894_v03_Linke04 dk(Mal) | 103 | A | V | MeF3C | V | MeW | N | Y | F4OMe |
| 894_v157_K(Mal) | 104 | A | V | F | V | W | N | Y | Y |
| 894_v158_K(Mal) | 105 | A | V | MeF3C | V | W | N | Y | F4OMe |
| 894_v159_K(Mal) | 106 | A | V | MeF3C | V | W | N | Y | F4OMe |
| 894_v164_K(Mal) | 107 | A | E | MeF3C | V | W | N | Y | F4OMe |
| 894_G4S2_K(Mal) | 108 | A | V | F | V | W | N | Y | Y |
| 894_PEG8_K(Mal) | 109 | A | V | F | V | W | N | Y | Y |
| 894_PEG4_K(Mal) | 110 | A | V | F | V | W | N | Y | Y |

**[TABLE 1-4-2]**

| | | | | | | | | | | | |
|---|---|---|---|---|---|---|---|---|---|---|---|
| R | R | Y | MeY | C | G | G | R | G | R | S | K(Maleimide) |
| R | R | Y | MeY | C | G | G | G | G | S | S | K(Maleimide) |
| R | R | Y | MeY | C | G | G | K(Maleimide) | -NH2 | | | |
| R | R | Y | MeY | C | G | G | R | G | R | S | K(Maleimide) |
| R | R | Y | MeY | C | G | G | G | G | S | S | K(Maleimide) |
| R | R | Y | MeY | C | G | G | S | G | S | G | dK(Maleimide) |
| R | R | Y | MeY | C | G | G | S | G | S | G | G |
| R | R | Y | MeY | C | G | G | de | G | de | G | dK(Maleimide) |
| R | R | Y | MeY | C | G | G | de | G | G | de | G |
| R | dr | F | MeY | C | G | G | S | G | S | G | dK(Maleimide) |
| R | dr | F | MeY | C | G | G | S | G | S | G | G |
| R | dr | F | MeY | C | G | G | de | G | de | G | dK(Maleimide) |
| R | dr | F | MeY | C | G | G | de | G | G | de | G |
| R | R | F | Y | C | G | G | S | G | S | G | K(Maleimide) |
| R | dr | F | MeY | C | G | G | S | G | S | G | K(Maleimide) |
| R | R | F | MeY | C | G | G | S | G | S | G | K(Maleimide) |
| R | dr | F | MeY | C | G | G | S | G | S | G | K(Maleimide) |
| R | R | Y | MeY | C | G | G | G | G | S | S | K(Maleimide) |
| R | R | Y | MeY | C | PEG8c | K(Maleimide) | -NH2 | | | | |
| R | R | Y | MeY | C | PEG4c | K(Maleimide) | -NH2 | | | | |

**[TABLE 1-4-3]**

| | | | | | | | | | |
|---|---|---|---|---|---|---|---|---|---|
| -NH2 | | | | | | 1010.33 | 3 | Maleimide | B |
| -NH2 | | | | | | 1430.85 | 2 | Maleimide | B |
| | | | | | | 1261.67 | 2 | Maleimide | B |
| -NH2 | | | | | | 993.69 | 3 | Maleimide | B |
| -NH2 | | | | | | 1405.88 | 2 | Maleimide | B |
| -NH2 | | | | | | 949.61 | 3 | Maleimide | B |
| S | S | G | G | dK(Maleimide) | -NH2 | 1064.85 | 3 | Maleimide | B |
| -NH2 | | | | | | 977.67 | 3 | Maleimide | B |
| G | de | G | G | dK(Maleimide) | -NH2 | 1096.74 | 3 | Maleimide | B |
| -NH2 | | | | | | 965.12 | 3 | Maleimide | B |
| S | S | G | G | dK(Maleimide) | -NH2 | 1080.24 | 3 | Maleimide | B |
| -NH2 | | | | | | 993.16 | 3 | Maleimide | B |
| G | de | G | G | dK(Maleimide) | -NH2 | 1112.35 | 3 | Maleimide | B |
| -NH2 | | | | | | 934.92 | 3 | Maleimide | ND |
| -NH2 | | | | | | 1440.31 | 2 | Maleimide | ND |
| -NH2 | | | | | | 1440.22 | 2 | Maleimide | ND |
| -NH2 | | | | | | 1455.27 | 2 | Maleimide | ND |
| NHS | | | | | | 1416.95 | 2 | Maleimide | A |
| | | | | | | 952.12 | 3 | Maleimide | A |
| | | | | | | 893.39 | 3 | Maleimide | A |

**[TABLE 5]**

| [TABLE 5-1] | | | | | | | | | | | | | | |
|---|---|---|---|---|---|---|---|---|---|---|---|---|---|---|
| 111 | PEG12 | | | | | | | | | | | | | |
| 112 | PEG4c | K(Maleimide) | | | | | | | | | | | | |
| 113 | PEG8c | K(Maleimide) | | | | | | | | | | | | |
| 114 | PEG12 | K(Maleimide) | | | | | | | | | | | | |
| 115 | PEG36 | K(Maleimide) | | | | | | | | | | | | |
| 116 | PEG36 | NHSester | | | | | | | | | | | | |
| 117 | PEG12 | NHS ester | | | | | | | | | | | | |
| 118 | PEG12 | Hydrazide | | | | | | | | | | | | |
| 119 | PEG36 | Hydrazide | | | | | | | | | | | | |
| 120 | PEG4 | Hydrazide | | | | | | | | | | | | |
| 121 | PEG4c | KTrzMal | | | | | | | | | | | | |
| 122 | PEG8c | KTrzMal | | | | | | | | | | | | |
| 123 | PEG12c | KTrzMal | | | | | | | | | | | | |
| 124 | PEG4c | PEG4c | K(Maleimide) | | | | | | | | | | | |
| 125 | PEG4c | PEG4c | PEG4c | K(Maleimide) | | | | | | | | | | |
| 126 | G | PEG12c | gAbu | NHS | | | | | | | | | | |
| 127 | G | PEG4c | gAbu | NHS | | | | | | | | | | |
| 128 | G | PEG4c | PEG4c | PEG4c | gAbu | NHS | | | | | | | | |
| 129 | G | PEG4c | R | PEG4c | R | PEG4c | gAbu | NHS | | | | | | |
| 130 | G | E | PEG12 | Hydrazide | | | | | | | | | | |
| 131 | G | E | G | E | PEG12 | Hydrazide | | | | | | | | |
| 132 | G | G | gAbu | | | | | | | | | | | |
| 133 | G | G | G | G | S | S | G | G | G | G | S | S | gAbu | NHS |
| 134 | G | G | R | G | R | S | G | G | R | G | R | S | gAbu | NHS |
| 135 | G | G | Q | G | Q | S | G | G | Q | G | Q | S | gAbu | NHS |

**[TABLE 5-2]**

| | | | | | | | | | | | | |
|---|---|---|---|---|---|---|---|---|---|---|---|---|
| 136 G | G | S | G | S | S | G | G | S | G | S | S | K(Maleimide) |
| 137 G | G | S | G | S | S | G | S | S | K(Maleimide) | | | |
| 138 G | G | Q | G | Q | S | gAbu | NHS | | | | | |
| 139 G | G | G | G | S | S | gAbu | NHS | | | | | |
| 140 G | G | R | G | R | S | gAbu | NHS | | | | | |
| 141 G | G | G | G | S | S | KTrzMal | | | | | | |
| 142 G | S | S | G | S | S | K(Maleimide) | | | | | | |
| 143 G | MeG | S | MeG | S | S | K(Maleimide) | | | | | | |
| 144 G | G | R | G | R | S | K(Maleimide) | | | | | | |
| 145 G | G | R | S | E | S | K(Maleimide) | | | | | | |
| 146 G | G | G | G | S | S | K(Maleimide) | | | | | | |
| 147 G | G | S | G | S | S | K(Maleimide) | | | | | | |
| 148 G | G | S | G | E | S | K(Maleimide) | | | | | | |
| 149 G | G | S | G | R | S | K(Maleimide) | | | | | | |
| 150 G | G | S | G | S | G | K(Maleimide) | | | | | | |
| 151 G | G | S | G | S | G | dK(Maleimide) | | | | | | |
| 152 G | G | E | S | E | S | K(Maleimide) | | | | | | |
| 153 G | G | de | G | de | G | dK(Maleimide) | | | | | | |
| 154 G | G | S | G | S | G | G | S | S | G | G | dK(Maleimide) | |
| 155 G | G | de | G | G | de | G | G | de | G | G | dK(Maleimide) | |
| 156 G | G | G | S | S | K(Maleimide) | | | | | | | |
| 157 G | G | S | S | K(Maleimide) | | | | | | | | |
| 158 G | G | S | K(Maleimide) | | | | | | | | | |
| 159 G | G | K(Maleimide) | | | | | | | | | | |
| 160 G | K(Maleimide) | | | | | | | | | | | |
| 161 K(Maleimide) | | | | | | | | | | | | |

### [Example 2-1]

### Synthesis of 894_3m_G4S2_K(Mal) (SEQ ID NO: 37)

This example describes the exemplary synthesis of 894_3m_G4S2_K(Mal) (the corresponding linker listed in TABLE 1 is bound to the peptide of SEQ ID NO: 37)

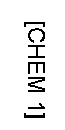

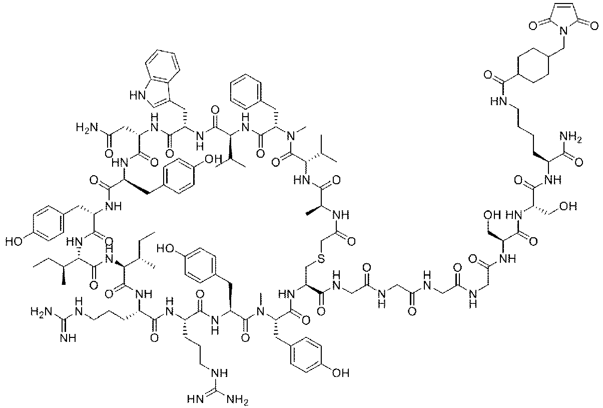

The target peptides were synthesized using Sieber Amide Resin (Watanabe Chemical Industries, Ltd., 0.52 mmol/g, 2.4 g × 3), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (5.3 equivalents/10 equivalents/5 equivalents) was used per 1 equivalent of resin, and the reaction was carried out once at 75°C for 10 min in DMF. However, the 15th residue was reacted at 50°C for 20 min. The 3rd, 4th, 8th, 10th, 13th, and 14th residues were reacted twice at 75°C for 10 min. The 11th and 12th residues were reacted twice at 50°C for 20 min. The 2nd residue was reacted three times at 75°C for 60 min. The 1st residue was reacted twice at 75°C for 20 min. Fmoc removal was performed by reacting with 20% piperidine in DMF at 75°C for 3 min, this is set as a basic condition. However, Fmoc removal of the 15th, 16th and 17th residues was carried out by reaction at room temperature for 5 min followed by reaction at 75°C for 3 min. Fmoc removal of the 2nd and 13th residues was carried out by reaction at 25°C for 5 min followed by reaction for 10 min. For the introduction of the chloroacetyl group, the Fmoc group of the α-amino group was removed from the solid-phase resin holding the Fmoc-protected peptide obtained in the previous step by the method described above. Chloroacetic acid (5 equivalents), DIPCI (5 equivalents), and HOSu (5 equivalents) were then added and the reaction was carried out by shaking in DMF, and adding the same volume of DMF as DCM to prepare a DCM/DMF solution of CIAcOSu (0.015 M), then adding the prepared solution to the solid phase resin, and shaking at room temperature for 180 min. For deprotection of the side chains and removal from the solid-phase resin, the resin obtained after the chloroacetyl group introduction step was first washed five times with DMF and three times with methylene chloride, and drying under reduced pressure. Then, to the reaction vessel containing the solid-phase resin, reactant cocktail-A (200 mL, mixture of TFA/H₂O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether cooled to 0°C, and a cloudy precipitate was formed. The mixture was filtered, washed with diethyl ether cooled to 0°C, and the resulting solid was used for the next cyclization reaction. For the cyclization reaction of the peptide, the peptide was dissolved in DMSO (containing 5% water) to a final concentration of 4.9 mM based on the mol number of the solid-phase resin, and then 7 equivalents of triethylamine was added and stirred at room temperature for 1 hour. To the resulting reaction solution, 1.05 equivalents of SMCC was added and shaken at room temperature for 1.5 hours. The resulting reaction solution was concentrated under reduced pressure using GenevaC EZ-2 Elite.

The resulting crude product was purified using the following conditions (Column: Waters Xbridge (registered trademark) C18 5µm 50x250 mm; Mobile Phase: A = 0.1% TFA in H₂O, B = 0.1% TFA in MeCN; Temperature: 60°C; Gradient (%B conc): 0-0% over 5.1 min, then 0-5% over 1.9 min, then 5-29% over 5 min, then 29-34% over 13.5min, then 34-60% over 1.5 min; Flow Rate: 1-1mL/min over 5.1 min, then 1-119mL/min over 1.9 min, then 119 mL/min).

The purity of the target product was 94.1%, calculated from the area ratio of the LC/MS (UV wavelength 225 nm) chromatogram under analyzing conditions.

Analyzing Conditions: Retention Time = 11.33 min; Column: Kinetex EVO C18 2.6 µm 2.1x150 mm, 100Å; Mobile Phase: A = 0.025% TFA in H₂O, B = 0.025% TFA in MeCN; Temperature: 40°C; Gradient (% B conc): 20-60% over 20 min, then 60-95% over 1 min, then 95% over 5 min; Flow Rate: 0.25 mL/min.
ESI-MS (+) observed m/z = 1423.95 (M+2H)²⁺

### [Example 2-2]

### Synthesis of 894_3m_GGRGRS_K(Mal) (SEQ ID NO: 82)

This example describes the exemplary synthesis of 894_3m_GGRGRS_K(Mal) (the corresponding linker listed in TABLE 1 is bound to the peptide of SEQ ID NO: 82).

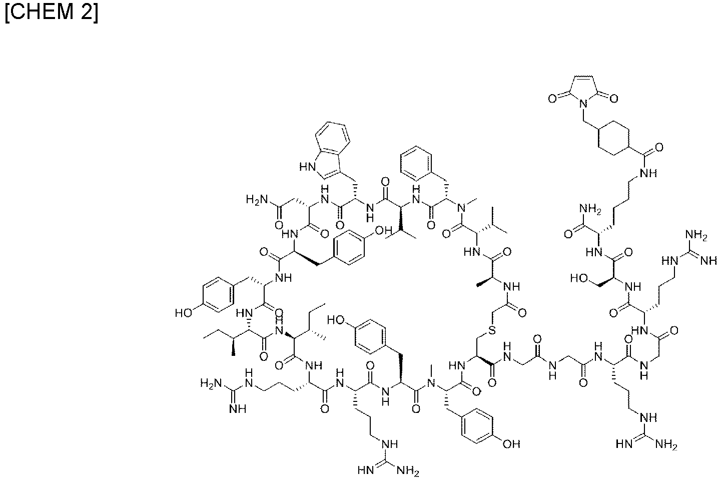

The target peptides were synthesized using Sieber Amide Resin (Watanabe Chemical Industries, Ltd., 0.47 mmol/g, 0.53 g), starting with the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/HATU/DIEA (4.2 equivalents/4 equivalents/8 equivalents) was used per 1 equivalent of resin, and the reaction was carried out twice at 75°C for 10 min in DMF, this is set as a basic condition. However, the 2nd residue was reacted twice at 75°C for 30 min. The 5th, 6th, 7th, 16th, 17th, 19th, 21th and 22th residues were reacted once at 75°C for 10 min. The 11th residue was reacted twice at 50°C for 15 min. The 12th, 15th, 18th and 20th residues were reacted once at 50°C for 15 min. Fmoc removal was performed by reacting with 20% piperidine in DMF at 75°C for 3 min, this is set as a basic condition. However, Fmoc removal of the 2nd and 13th residues was carried out by reacting twice at room temperature for 5 min. For the introduction of the chloroacetyl group, chloroacetic acid (5 equivalents), DIPCI (5 equivalents), and HOSu (5 equivalents) were added and stirred in DMF, and the same volume of DMF as DCM was added to prepare a DCM/DMF solution of CIAcOSu (0.25 M), then the prepared solution was added to the solid phase resin obtained in the previous step, and shaked at room temperature for 60 min. For deprotection of the side chains and removal from the solid-phase resin, the resin obtained after the chloroacetyl group introduction step was first washed five times with DMF and three times with methylene chloride, and drying under reduced pressure. Then, to the reaction vessel containing the solid-phase resin, reactant cocktail-A (10 mL, mixture of TFA/H₂O/TIS/DODT in a volume ratio of 92.5/2.5/2.5/2.5) was added, and the vessel was shaken at room temperature for 150 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of the mixed solvent diethyl ether and hexane cooled to 0°C, and a cloudy precipitate was formed. The mixture was centrifuged (9500 rpm, 1 min), and the supernatant was decanted and washed with diethyl ether cooled to 0°C, then the resulting solid was used for the next cyclization reaction. For the cyclization reaction of the peptide, the peptide was dissolved in DMSO (containing 5% water) to a final concentration of 5 mM based on the mol number of the solid-phase resin, and then 7 equivalents of triethylamine was added and stirred at room temperature for 3 hour. To the resulting reaction solution, 1.1 equivalents of SMCC was added and shaken at room temperature for 3 hours. The resulting reaction solution was concentrated under reduced pressure using GenevaC EZ-2 Elite.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B conc): 5-29% over 3 min, then 29-34% over 8 min, then 34-60% over 1 min; Flow Rate: 120 mL/min).

The purity of the target product was 95.4%, calculated from the area ratio of the LC/MS (UV wavelength 225 nm) chromatogram under analyzing conditions.

Analyzing Conditions: Retention Time = 9.53 min; Column: Kinetex EVO C18 2.6 µm 2.1x150 mm, 100Å; Mobile Phase: A = 0.025% TFA in H₂O, B = 0.025% TFA in MeCN; Temperature: 40°C; Gradient (% B conc): 20-60% over 20 min, then 60-95% over 1 min, then 95% over 5 min; Flow Rate: 0.25 mL/min.
ESI-MS (+) observed m/z = 1005.73 (M+3H)³⁺

### [Example 2-3]

### Synthesis of hTfR_000894_PEG11_(Hydrazine) (SEQ ID NO: 6)

This example describes the exemplary synthesis of hTfR_000894_PEG11_(Hydrazine) (the corresponding linker listed in TABLE 1 is bound to the peptide of SEQ ID NO: 6).

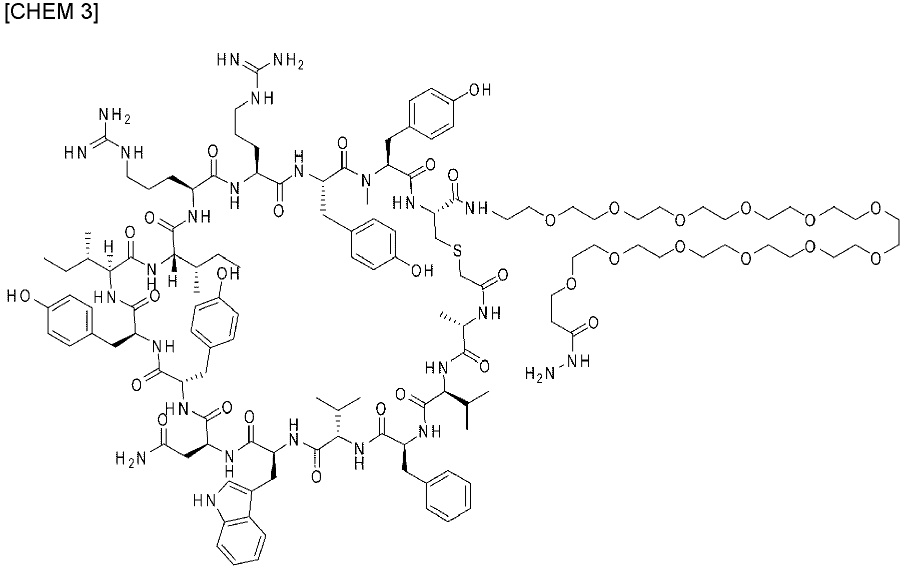

The target peptides were synthesized using NH2-NH-Trt(2-Cl)-resin (0.77 mmol/g, 0.13 g), prepared according to the general method from commercially available CI-Trt(2-CI)-resin, starting with the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/HATU/DIEA (5.3 equivalents/5 equivalents/10 equivalents) was used per 1 equivalent of resin, and the condensation reaction was carried out once at 25°C for 30 min, this is set as a basic condition. The 11th, 12th, 13th and 14th residues were reacted twice at 25°C for 30 min. The 16th residue was reacted once at 25°C for 60 min. Fmoc removal was performed by reacting with 20% piperidine in DMF at 25°C for 5 min, followed by reacting for 10 min. For the introduction of the chloroacetyl group, the Fmoc group of the α-amino group was removed from the solid-phase resin holding the Fmoc-protected peptide obtained in the previous step by the method described above, and then DMF solution of 0.2 M chloroacetic acid (5 equivalents), DMF solution of 0.5 M HATU (5 equivalents), and DMF solution of 1 M DIEA (10 equivalents) were added to the solid-phase resin and the reaction was carried out by shaking at room temperature for 30 min. For deprotection of the side chains and removal from the solid-phase resin, the resin obtained after the chloroacetyl group introduction step was first washed five times with DMF and three times with methylene chloride, and drying under reduced pressure. Then, to the reaction vessel containing the solid-phase resin, reactant cocktail (4 mL, mixture of TFA/H₂O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane (1/1) cooled to 0°C, and a cloudy precipitate was formed. The mixture was centrifuged (10000 rpm, 1 min), and the supernatant was decanted. The obtained solid was washed again with a small amount of diethyl ether cooled to 0°C, and the resulting solid was dried and used for the next cyclization reaction. For the cyclization reaction of the peptide, the peptide was dissolved in DMSO to a final concentration of 5 mM based on the mol number of the solid-phase resin, and then 6 equivalents of triethylamine was added and stirred at room temperature for about 15 hour. The resulting reaction solution was concentrated under reduced pressure using Savant Explorer SpeedVac.

The resulting crude product was purified using the following conditions (Column: Waters Xbridge (registered trademark) C18 5µm 19x150 mm; Mobile Phase: A = 0.1% TFA in H₂O, B = 0.1% TFA in MeCN; Temperature: 40°C; Gradient (%B conc): 5-30% over 3 min, then 30-35% over 8 min, then 35-60% over 1 min; Flow Rate: 17 mL/min).

The purity of the target product was 76.5%, calculated from the area ratio of the LC/MS (UV wavelength 225 nm) chromatogram under analyzing conditions.

Analyzing Conditions: Retention Time = 10.28 min; Column: Kinetex EVO C18 2.6 µm 2.1x150 mm, 100Å; Mobile Phase: A = 0.025% TFA in H₂O, B = 0.025% TFA in MeCN; Temperature: 40°C; Gradient (% B conc): 20-60% over 20 min, then 60-95% over 1 min, then 95-95% over 5 min; Flow Rate: 0.25 mL/min.
ESI-MS (+) observed m/z = 899.92 (M+3H)³⁺

### [Example 2-4]

### Synthesis of hTfR_000894_PEG11_K (Maleimide) (SEQ ID NO: 1)

This example describes the exemplary synthesis of hTfR_000894_PEG11_K (Maleimide) (the corresponding linker listed in TABLE 1 is bound to the peptide of SEQ ID NO: 1).

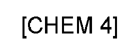

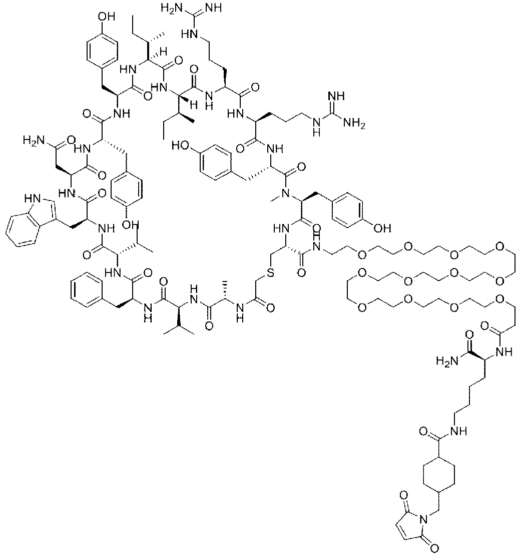

The target peptides were synthesized using Fmoc-NH-SAL-PEG resin (Watanabe Chemical Industries, Ltd., 0.38 mmol/g, 0.66 g), starting from the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/HATU/DIEA (4.2 equivalents/4 equivalents/8 equivalents) was used per 1 equivalent of resin, and the reaction was carried out once at 75°C for 10 min, this is set as a basic condition. However, the 11th and 12th residues were reacted twice at 25°C for 20 min. The 13th and 14th residue were reacted twice at 75°C for 10 min. The 15th residue was reacted once at 25°C for 30 min. The 16th residue was reacted once at 25°C for 60 min. Fmoc removal was performed by reacting with 20% piperidine in DMF once at 75°C for 3 min, this is set as a basic condition. However, for the 13th and 15th residues, Fmoc removal was performed by reacting at 5°C for 5 min, followed by reacting for 10 min. For the introduction of the chloroacetyl group, the Fmoc group of the α-amino group was removed from the solid-phase resin holding the Fmoc-protected peptide obtained in the previous step by the method described above. Then DMF solution of 0.2 M chloroacetic acid (5 equivalents), DMF solution of 0.5 M HATU (5 equivalents), and DMF solution of 1 M DIEA (10 equivalents) were added to the solid-phase resin and the reaction was carried out by shaking at room temperature for 30 min. For deprotection of the side chains and removal from the solid-phase resin, the resin obtained after the chloroacetyl group introduction step was first washed five times with DMF and three times with methylene chloride, and drying under reduced pressure. Then, to the reaction vessel containing the solid-phase resin, reactant cocktail-A (10 mL, mixture of TFA/H₂O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane cooled to 0°C, and a cloudy precipitate was formed. The mixture was centrifuged (10000 rpm, 1 min), and the supernatant was decanted. The obtained solid was washed with diethyl ether cooled to 0°C, and the resulting solid was used for the next cyclization reaction. For the cyclization reaction of the peptide, the peptide was dissolved in DMSO to a final concentration of 5 mM based on the mol number of the solid-phase resin, and then 10 equivalents of triethylamine was added and stirred at room temperature for about 15 hours. To the resulting reaction solution, 1.2 equivalents of SMCC was added based on the mol number of solid-phase resin, and the solution was stirred at room temperature for 3.5 hours. Then acetic acid was added and the resulting reaction solution was concentrated under reduced pressure using Genevac EZ-2 Elite.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B conc): 8-33% over 3 min, then 33-38% over 8 min, then 38-60% over 1 min; Flow Rate: 120 mL/min).

The purity of the target product was 92.2%, calculated from the area ratio of the LC/MS (UV wavelength 225 nm) chromatogram under analyzing conditions.

Analyzing Conditions: Retention Time = 12.03 min; Column: Kinetex EVO C18 2.6 µm 2.1x150 mm, 100Å; Mobile Phase: A = 0.025% TFA in H₂O, B = 0.025% TFA in MeCN; Temperature: 40°C; Gradient (% B conc): 20-60% over 20 min, then 60-95% over 1 min, then 95-95% over 5 min; Flow Rate: 0.25 mL/min.
ESI-MS (+) observed m/z = 1010.68 (M+3H)³⁺

### [Example 2-5]

### Synthesis of 894_PEG12_(NHS) (SEQ ID NO: 19)

This example describes the exemplary synthesis of 894_PEG12_(NHS) (the corresponding linker listed in TABLE 1 is bound to the peptide of SEQ ID NO: 19).

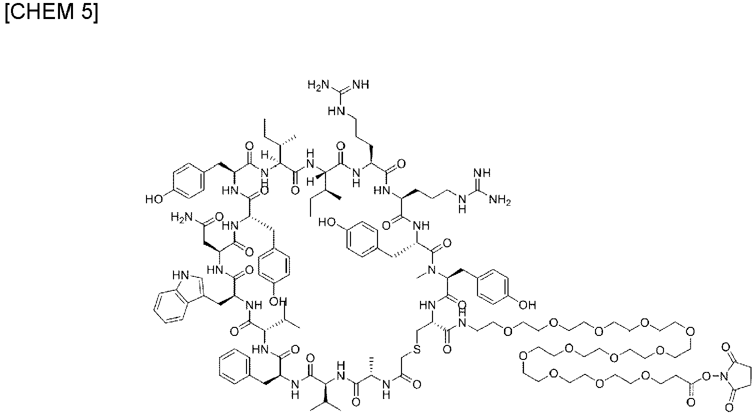

The target peptides were synthesized using Fmoc-Wang Resin (1.2 mmol/g, 0.21 g), starting from the removal of the Fmoc group by the general method described above. Liberty Blue (CEM Inc.) was used as the solid-phase synthesizer, and the synthesis was performed according to the manufacturer's manual. For the introduction of each residue, Fmoc-AA/HATU/DIPEA (4.2 equivalents/4 equivalents/8 equivalents) was used per 1 equivalent of resin, and the reaction was carried out once at 75°C for 10 min, this is set as a basic condition. However, the 11th and 12th residues were reacted twice at 25°C for 20 min. The 13th and 14th residue were reacted twice at 75°C for 10 min. The 15th residue was reacted once at 25°C for 30 min. The 16th residue was reacted by using Fmoc-AA/DIPCI/DMAP (3 equivalents/3 equivalents/0.75 equivalents) and reacting once at 25°C for 60 min. Fmoc removal was performed by reacting with 20% piperidine in DMF once at 75°C for 3 min. However, for the 13th and 15th residues, Fmoc removal was performed by reacting at 25°C for 5 min, followed by reacting for 10 min. For the introduction of the chloroacetyl group, the Fmoc group of the α-amino group was removed from the solid-phase resin holding the Fmoc-protected peptide obtained in the previous step by the method described above. Then DMF solution of 0.2 M chloroacetic acid (5 equivalents), DMF solution of 0.5 M HATU (5 equivalents), and DMF solution of 1 M DIEA (10 equivalents) were added to the solid-phase resin and the reaction was carried out by stirring at 25°C for 30 min. For deprotection of the side chains and removal from the solid-phase resin, the resin obtained after the chloroacetyl group introduction step was first washed five times with DMF and three times with methylene chloride, and drying under reduced pressure. Then, to the reaction vessel containing the solid-phase resin, reactant cocktail-A (10 mL, mixture of TFA/H₂O/TIS/DODT in a volume ratio of 92.5/2.5/2.5/2.5) was added, and the vessel was shaken at 25°C for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane = 1/1 cooled to 0°C, and a cloudy precipitate was formed. The mixture was centrifuged (10000 rpm, 1 min), and the supernatant was decanted. The obtained solid was washed with diethyl ether cooled to 0°C, and the resulting solid was used for the next cyclization reaction. For the cyclization reaction of the peptide, the peptide was dissolved in DMSO to a final concentration of 5 mM based on the mol number of the solid-phase resin, and then 6 equivalents of triethylamine was added and stirred at 25°C for about 16 hours. The resulting reaction solution was concentrated under reduced pressure using Savant Explorer SpeedVac.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B conc): 7-32% over 3 min, then 32-37% over 8 min, then 37-60% over 1 min; Flow Rate: 120 mL/min).

After lyophilization, the resulting cyclic peptide (51.9 mg, 19.3 µmol) was dissolved in DMSO/water (0.7 mL, 9/1), then N-hydroxysuccinimide (11 mg, 96.5 µmol) and EDCI (18.5 mg, 96.5 µmol) were added and stirred at 25°C for 3 hours, then the mixture was quenched with acetic acid.

The resulting reaction mixture was purified using the following conditions (Column: Waters Xbridge (registered trademark) C18 5µm 30x150 mm; Mobile Phase: A = 0.1% TFA in H₂O, B = 0.1% TFA in MeCN; Temperature: 40°C; Gradient (%B conc): 9-35% over 3 min, then 35-40% over 8 min, then 40-60% over 1 min; Flow Rate: 45 mL/min).

The purity of the target product was 88.0%, calculated from the area ratio of the LC/MS (UV wavelength 225 nm) chromatogram under analyzing conditions.

Analyzing Conditions: Retention Time = 12.74 min; Column: Kinetex EVO C18 2.6 µm 2.1x150 mm, 100Å; Mobile Phase: A = 0.025% TFA in H₂O, B = 0.025% TFA in MeCN; Temperature: 40°C; Gradient (% B conc): 20-60% over 20 min, then 60-95% over 1 min, then 95% over 5 min; Flow Rate: 0.25 mL/min.
ESI-MS (+) observed m/z = 1390.89 (M+2H)²⁺

### [Example 3]

Conjugates of the peptides obtained in Example 1 with antibodies were synthesized, purified, and analyzed by the following method.

### General Method

### [RP-UPLC-MS Chromatography]

### Reversed-phase chromatography (RP-UPLC)

### [Analyzing Condition A]

Antibodies and antibody-peptide conjugates were separated on an ACQUITY UPLC (registered trademark) Protein BEH C4 (Cat. No. 186004495, Waters) column with a size of 2.1 mm×50 mm and a mean particle size of 1.7 µm with 300Å pores. Analysis is performed in the analyzing condition of: Mobile phase: A = water/0.1% trifluoroacetic acid, B = acetonitrile/0.1% trifluoroacetic acid, Column Temperature 80°C, Flow Rate 0.4 mL/min, Gradient (%B conc.) as listed in TABLE 2-1, Absorption Wavelength 220 nm.

**[TABLE 2-1]**

| Retention Time (min) | %B Conc. |
|---|---|
| Initial | 32% |
| 1.00 | 32% |
| 1.01 | 32% |
| 9.00 | 50% |
| 9.30 | 95% |
| 9.31 | 95% |
| 9.40 | 5.0% |
| 9.41 | 5.0% |
| 9.49 | 32% |
| 10.50 | 32% |

### [Analyzing Condition B]

Antibodies and antibody-peptide conjugates were separated on an ACQUITY UPLC (registered trademark) Protein BEH C4 (Cat. No. 186004495, Waters) column with a size of 2.1 mm×50 mm and a mean particle size of 1.7 µm with 300Å pores. Analysis is performed in the analyzing condition of: Mobile phase: A = water/0.1% trifluoroacetic acid, B = acetonitrile/0.1% trifluoroacetic acid, Column Temperature 80°C, Flow Rate 0.4 mL/min, Gradient (%B conc.) as listed in TABLE 2-2, Absorption Wavelength 280 nm.

**[TABLE 2-2]**

| **Retention Time (min)** | %B Conc. |
|---|---|
| Initial | 20% |
| 1.00 | 20% |
| 1.01 | 20% |
| 9.00 | 65% |
| 9.50 | 95% |
| 9.80 | 95% |
| 9.90 | 25% |
| 10.50 | 25% |

### [Analyzing Condition C]

Antibodies and antibody-peptide conjugates were separated on an ACQUITY UPLC (registered trademark) Protein BEH C4 (Cat. No. 186004495, Waters) column with a size of 2.1 mm×50 mm and a mean particle size of 1.7 µm with 300Å pores. Analysis is performed in the analyzing condition of: Mobile phase: A = water/0.1% trifluoroacetic acid, B = acetonitrile/0.1% trifluoroacetic acid, Column Temperature 80°C, Flow Rate 0.4 mL/min, Gradient (%B conc.) as listed in TABLE 2-3, Absorption Wavelength 280 nm.

**[TABLE 2-3]**

| **Retention Time (min)** | %B Conc. |
|---|---|
| Initial | 25% |
| 1.00 | 25% |
| 1.01 | 25% |
| 9.00 | 55% |
| 9.30 | 95% |
| 9.31 | 95% |
| 9.40 | 5.0% |
| 9.41 | 5.0% |
| 9.49 | 25% |
| 10.50 | 25% |

### [Analyzing Condition D]

Antibodies and antibody-peptide conjugates were separated on an ACQUITY UPLC (registered trademark) Protein BEH C4 (Cat. No. 186004495, Waters) column with a size of 2.1 mm×50 mm and a mean particle size of 1.7 µm with 300Å pores. Analysis is performed in the analyzing condition of: Mobile phase: A = water/0.1% trifluoroacetic acid, B = acetonitrile/0.1% trifluoroacetic acid, Column Temperature 80°C, Flow Rate 0.4 mL/min, Gradient (%B conc.) as listed in TABLE 2-4, Absorption Wavelength 220 nm.

**[TABLE 2-4]**

| Retention Time (min) | %B Conc. |
|---|---|
| Initial | 32% |
| 1.00 | 32% |
| 1.01 | 32% |
| 9.00 | 42% |
| 9.30 | 95% |
| 9.31 | 95% |
| 9.40 | 5.0% |
| 9.41 | 5.0% |
| 9.49 | 32% |
| 10.50 | 32% |

### [Analyzing Condition E]

Antibodies and antibody-peptide conjugates were separated on an ACQUITY UPLC (registered trademark) Protein BEH C4 (Cat. No. 186004495, Waters) column with a size of 2.1 mm×50 mm and a mean particle size of 1.7 µm with 300Å pores. Analysis is performed in the analyzing condition of: Mobile phase: A = water/0.1% trifluoroacetic acid, B = acetonitrile/0.1% trifluoroacetic acid, Column Temperature 80°C, Flow Rate 0.4 mL/min, Gradient (%B conc.) as listed in TABLE 2-5, Absorption Wavelength 220 nm.

**[TABLE 2-5]**

| **Retention Time (min)** | %B Conc. |
|---|---|
| Initial | 30% |
| 1 | 30% |
| 1.01 | 30% |
| 7 | 45% |
| 7.3 | 95% |
| 7.31 | 95% |
| 7.4 | 5.0% |
| 7.41 | 5.0% |
| 7.49 | 30% |
| 8.5 | 30% |

### Mass Spectrometry (MS)

Mass spectrometry data for antibodies and antibody-peptide conjugates were obtained using Xevo (registered trademark) G2-XSQTof (Waters) in the 500-4000 m/z range in positive ESI mode. The source temperature and the desolventizing gas temperatures were 150°C and 500°C, respectively, and the flow rates of the desolventizing gas and nebulizer gas were 800 L/hour and 50 L/hour, respectively. The capillary voltage was set to 3.00 kV. The conversion for mass spectrum was performed by using MaxEnt (trademark) 1 deconvolution algorithm in MassLynx (trademark) software, according to the manufacturer's specified method.

### Measurement of Antibody and Antibody-peptide Conjugate Concentration

The UV analyzer (DeNovix DS-11) was used to measure antibody concentrations according to the manufacturer's specified method. In doing so, the 280 nM molar absorption coefficient, which varies with the type of antibody, was used.

### Purification of Antibody-peptide Conjugate

### [Purification Method A]

70 uL of antibody-peptide conjugate and 4 mL of 100 mM HEPES buffer pH 7.8 were added per column in the vessel of Amicon Ultra-4 (30, 000 MWCO, Merck Millipore Ltd.), and centrifugation (5000 G×15 min) was performed using a centrifuge (TOMY SEIKO Co., Ltd.). The antibody-peptide conjugate was purified by repeating the addition of the buffer solution to the antibody-peptide conjugate and centrifugation a total of three times.

### [Purification Method B]

125 uL of antibody-peptide conjugate and 4mL of 20% acetic acid solution pH 2.0 were added per column in the vessel of Amicon Ultra-4 (30, 000 MWCO, Merck Millipore Ltd.), and centrifugation (5000 G×15 min) was performed using a centrifuge (TOMY SEIKO Co., Ltd.). Addition of the solution to the antibody-peptide conjugate and centrifugation were repeated a total of three times. Then, 4 mL of 100 mM HEPES buffer pH 8.5 was added to the column, and centrifugation (5000 G × 15 min) was performed once using a centrifuge (TOMY SEIKO Co., Ltd.) to purify the antibody-peptide conjugate.

### [Purification Method C]

60 uL of antibody-peptide conjugate and 4mL of 10% acetic acid solution pH 2.0 were added per column in the vessel of Amicon Ultra-4 (50, 000 MWCO, Merck Millipore Ltd.), and centrifugation (5000 G×15 min) was performed using a centrifuge (TOMY SEIKO Co., Ltd.). Addition of the solution to the antibody-peptide conjugate and centrifugation were repeated a total of three times. Then, 4 mL of 100 mM HEPES buffer pH 8.5 was added to the column, and centrifugation (5000 G × 15 min) was performed once using a centrifuge (TOMY SEIKO Co., Ltd.) to purify the antibody-peptide conjugate.

### [Purification Method D]

150 uL of antibody-peptide conjugate and 4mL of 50mM HEPES buffer pH 7.8 were added per column in the vessel of Amicon Ultra-4 (30, 000 MWCO, Merck Millipore Ltd.), and centrifugation (5000 G×15 min) was performed using a centrifuge (TOMY SEIKO Co., Ltd.). Addition of the solution to the antibody-peptide conjugate and centrifugation were repeated a total of three times, then the purification of the antibody-peptide conjugate was performed.

### [Example 3-1]

### Buffer Exchange and Concentration of Nivolumab

For nivolumab, an antibody, the following operations were performed prior to conjugation with the peptide.

### [Buffer Exchange Method A]

Buffer exchange of nivolumab (Selleckchem, Cat. No. A2002) was performed by using Superdex 200 Increase 10/300GL (Cat. No. 28-9909-44, GE Healthcare) with an average particle size of 8.6 µm and a column size of 10 mm × 300 mm. The aforementioned column and AKTA pure25 M2 (GE Healthcare) were used with HEPES (Cat. No. 02443-05, Nacalai Tesque, Inc.) prepared at 50 mM, pH 7.8 as the mobile phase, then the eluted fractions were collected from 11.5 to 13.5 min at the detection wavelength of 220 nm and the flow rate of 0.5 mL/min. The collected fractions were concentrated by using Amicon Ultra (30, 000 MWCO, Merck Millipore Ltd.) to obtain a final nivolumab solution of 13.0 g/L.

### [Buffer Exchange Method B]

Buffer exchange of nivolumab (Selleckchem, Cat. No. A2002) was performed by using Bio-Gel P-30 (Bio-Rad, Cat. No. 1504150). Nivolumab dissolved in 5 g/L with 100 uL of 1xPBS per column is added to a column packed with 1 mL of Bio-Gel P-30 swollen with 100 mM HEPES pH 7.8 (Cat. No. 02443-05, Nacalai Tesque, Inc.), then 4.78 g/L of buffer-substituted nivolumab was collected in a plate centrifuge (Plate Spin, Kubota) at the condition of 1000 G, 4 min.

### [Buffer Exchange Method C]

Buffer exchange of nivolumab (Selleckchem, Cat. No. A2002) was performed by using Centricon Plus-70, Ultracel-PL membrane, 30 kDa (Merck, Cat. No. UFC703008). 1 mL of 5 mg/mL nivolumab was added to 60 mL of buffer prepared with HEPES (Cat. No. 02443-05, Nacalai Tesque, Inc.) at 50 mM and pH 7.8, and then centrifugation (2,300 g × 15 min) was performed using a centrifuge (TOMY SEIKO Co., Ltd.).

After centrifugation, 60 mL of the same solution was added and centrifuged again. This operation was repeated three times to obtain a final nivolumab solution of 11.1 g/L.

### [Example 3-2]

### Buffer Exchange and Concentration of Trastuzumab

For trastuzumab, an antibody, the following operations were performed prior to conjugation with the peptide.

### [Buffer Exchange Method A]

Buffer exchange of trastuzumab (Chugai Pharmaceutical Co., Ltd., Cat. No. 002224228) was performed by using Superdex 200 Increase 10/300GL (Cat. No. 28-9909-44, GE Healthcare) with an average particle size of 8.6 µm and a column size of 10 mm × 300 mm. The aforementioned column and AKTA pure25 M2 (GE Helathcare) were used with HEPES (Cat. No. 02443-05, Nacalai Tesque, Inc.) prepared at 50 mM, pH 7.8 as the mobile phase, then the eluted fractions ware collected from 20 min to 35 min at the flow rate of 0.5 mL/min. The collected fractions were concentrated by using Amicon Ultra (30, 000 MWCO, Merck Millipore Ltd.). Finally, 17.0 g/L of trastuzumab solution and 19.9 g/L trastuzumab solution were obtained.

### [Buffer Exchange Method B]

Buffer exchange of trastuzumab (Chugai Pharmaceutical Co., Ltd., Cat. No. 002224231) was performed by using Centricon Plus-70, Ultracel-PL membrane, 30 kDa (Merck, Cat. No. UFC703008). 1 mL of 5 mg/mL trastuzumab was added to 60 mL of buffer prepared with HEPES (Cat. No. 02443-05, Nacalai Tesque, Inc.) at 50 mM and pH 7.8, and centrifugation (2,300 g × 15 min) was performed using a centrifuge (TOMY SEIKO Co., Ltd.). After centrifugation, 60 mL of the same solution was added and centrifuged again. This operation was repeated three times to obtain a final trastuzumab solution of 10.0 g/L.

### [Example 3-3]

### Buffer Exchange and Concentration of Pertuzumab

For pertuzumab, an antibody, the following operations were performed prior to conjugation with the peptide.

Buffer exchange of pertuzumab (Selleck Biotech Co., Ltd., Cat. No. A2008) was performed by using Centricon Plus-70, Ultracel-PL membrane, 30 kDa (Merck, Cat. No. UFC703008). 50mM of HEPES (Nacalai Tesque, Inc., Cat. No. 02443-05) and 1 mL of 5 mg/mL pertuzumab per 60 mL of prepared buffer at pH 7.8 are added, and then centrifugation (2,300 g × 15 min) was performed using a centrifuge (TOMY SEIKO Co., Ltd.). 1 mL of 5 mg/mL pertuzumab was added to 60 mL of buffer prepared with HEPES (Cat. No. 02443-05, Nacalai Tesque, Inc.) at 50 mM and pH 7.8, and centrifugation (2,300 g × 15 min) was performed using a centrifuge (TOMY SEIKO Co., Ltd.). After centrifugation, 60 mL of the same solution was added and centrifuged again. This operation was repeated three times to obtain a final pertuzumab solution of 8.2 g/L.

### [Example 3-4]

### Buffer Exchange and Concentration of Cetuximab

For cetuximab, an antibody, the following operations were performed prior to conjugation with the peptide.

Buffer exchange of cetuximab (Selleck Biotech Co., Ltd., Cat. No. A2000) was performed by using Centricon Plus-70, Ultracel-PL membrane, 30 kDa (Merck, Cat. No. UFC703008). 1 mL of 5 mg/mL cetuximab was added to 60 mL of buffer prepared with HEPES (Cat. No. 02443-05, Nacalai Tesque, Inc.) at 50 mM and pH 7.8, and centrifugation (2,300 g × 15 min) was performed using a centrifuge (TOMY SEIKO Co., Ltd.). After centrifugation, 60 mL of the same solution was added and centrifuged again. This operation was repeated three times to obtain a final cetuximab solution of 6.7 g/L.

### [Example 3-5]

### Buffer Exchange and Concentration of Ipilimumab

For ipilimumab, an antibody, the following operations were performed prior to conjugation with the peptide.

Buffer exchange of ipilimumab (Selleck Biotech Co., Ltd., Cat. No. A2001) was performed by using Centricon Plus-70, Ultracel-PL membrane, 30 kDa (Merck, Cat. No. UFC703008). 50mM of HEPES (Nacalai Tesque, Inc., Cat. No. 02443-05) and 1 mL of 5 mg/mL ipilimumab per 60 mL of prepared buffer at pH 7.8 are added, and then centrifugation (2,300 g × 15 min) was performed using a centrifuge (TOMY SEIKO Co., Ltd.). 1 mL of 5 mg/mL ipilimumab was added to 60 mL of buffer prepared with HEPES (Cat. No. 02443-05, Nacalai Tesque, Inc.) at 50 mM and pH 7.8, and centrifugation (2,300 g × 15 min) was performed using a centrifuge (TOMY SEIKO Co., Ltd.). After centrifugation, 60 mL of the same solution was added and centrifuged again. This operation was repeated three times to obtain a final ipilimumab solution of 6.4 g/L.

### [Example 3-6]

### Buffer Exchange and Concentration of Atezolizumab

For atezolizumab, an antibody, the following operations were performed prior to conjugation with the peptide.

Buffer exchange of atezolizumab (Selleck Biotech Co., Ltd., Cat. No. A2004) was performed by using Centricon Plus-70, Ultracel-PL membrane, 30 kDa (Merck, Cat. No. UFC703008). 50mM of HEPES (Nacalai Tesque, Inc., Cat. No. 02443-05) and 1 mL of 5 mg/mL cetuximab per 60 mL of prepared buffer at pH 7.8 are added, and then centrifugation (2,300 g × 15 min) was performed using a centrifuge (TOMY SEIKO Co., Ltd.). 1 mL of 5 mg/mL atezolizumab was added to 60 mL of buffer prepared with HEPES (Cat. No. 02443-05, Nacalai Tesque, Inc.) at 50 mM and pH 7.8, and centrifugation (2,300 g × 15 min) was performed using a centrifuge (TOMY SEIKO Co., Ltd.). After centrifugation, 60 mL of the same solution was added and centrifuged again. This operation was repeated three times to obtain a final atezolizumab solution of 6.4 g/L.

### [Example 3-7]

### Buffer Exchange and Concentration of Pembrolizumab

For pembrolizumab, an antibody, the following operations were performed prior to conjugation with the peptide.

Buffer exchange of pembrolizumab (Selleck Biotech Co., Ltd., Cat. No. A2005) was performed by using Centricon Plus-70, Ultracel-PL membrane, 30 kDa (Merck, Cat. No. UFC703008). 1 mL of 5 mg/mL pembrolizumab was added to 60 mL of buffer prepared with HEPES (Cat. No. 02443-05, Nacalai Tesque, Inc.) at 50 mM and pH 7.8, and centrifugation (2,300 g × 15 min) was performed using a centrifuge (TOMY SEIKO Co., Ltd.). After centrifugation, 60 mL of the same solution was added and centrifuged again. This operation was repeated three times to obtain a final pembrolizumab solution of 9.5 g/L.

### [Example 3-8]

### Creation Method of Nivolumab-894_3m_G4S2_K(Mal) Conjugate (Conjugate No. 11) Using Maleimide

13 g/L of nivolumab whose buffer is exchanged by using [Buffer Exchange Method A] described in Example 3-1 was diluted to 5.2 g/L with 100 mM HEPES pH 7.8 (Cat. No. 02443-05, Nacalai Tesque, Inc.). The disulfide bonds in the antibody were reduced by adding 12.5 mM tris(2-carboxyethyl) phosphine (TCEP, 24 equivalents per one molecule of antibody) to the diluted nivolumab and incubating at 30°C for 60 hours.

Desalting was performed by adding 70 uL of the above solution per one column to a column packed with 1 mL of swollen Bio-Gel P-30 (Bio-Rad, Cat. No. 1504150). After repeating this operation one more time, a solution of the compound obtained in Example 2-1 dissolved to 10 mM with dimethyl sulfoxide (8.7 equivalents per one antibody molecule) was added, and incubated at 30°C for 30 min to react the disulfide bond in the antibody with the linker of the compound. The reaction of nivolumab with the peptide was confirmed by using [Analyzing Conditions A] described in [RP-UPLC-MS Chromatography], and the results are shown in TABLE 3-1 below.

The nivolumab-peptide conjugate obtained in the above reaction was purified using [Purification Method A] described in the section [Purification of Antibody-Peptide Conjugate] and nivolumab-peptide conjugate was obtained at a concentration of 4.37 g/L and antibody yield of 1.75 mg.

The peptides with maleimide as the reactive functional group of the linker part listed in TABLE 1 were reacted with nivolumab in the same manner using the method described above and the nivolumab-peptide conjugates listed in TABLE 3-1 were created.

### [Example 3-9]

### Creation Method of Nivolumab-894_3m_GGRGRS_K(Mal) Conjugate (Conjugate No. 43) Using Maleimide

13 g/L of nivolumab whose buffer is exchanged by using [Buffer Exchange Method A] described in Example 3-1 was diluted to 5.2 g/L with 100 mM HEPES pH 7.8 (Cat. No. 02443-05, Nacalai Tesque, Inc.). The disulfide bonds in the antibody were reduced by adding 12.5 mM tris(2-carboxyethyl) phosphine (TCEP, 24 equivalents per one molecule of antibody) to the diluted nivolumab and incubating at 30°C for 60 hours.

Desalting was performed by adding 70 uL of the above solution per one column to a column packed with 1 mL of swollen Bio-Gel P-30 (Bio-Rad, Cat. No. 1504150). After repeating this operation one more time, a solution of the compound obtained in Example 2-2 dissolved to 10 mM with dimethyl sulfoxide (8.7 equivalents per one antibody molecule) was added, and incubated at 30°C for 30 min to react the disulfide bond in the antibody with the linker of the compound. The reaction of nivolumab with the peptide was confirmed by using [Analyzing Conditions A] described in [RP-UPLC-MS Chromatography], and the results are shown in TABLE 3-1 below.

The nivolumab-peptide conjugate obtained in the above reaction was purified using [Purification Method A] described in the section [Purification of Antibody-Peptide Conjugate] and nivolumab-peptide conjugate was obtained at a concentration of 4.85 g/L and antibody yield of 2.42 mg.

### [Example 3-10]

### Creation Method of Trastuzumab-hTfR_000894_PEG11_K (Maleimide) Conjugate (Conjugate No. 1) Using Maleimide

19.9 g/L of trastuzumab whose buffer is exchanged by using [Buffer Exchange Method A] described in Example 3-2 was diluted to 5.2 g/L with 100 mM HEPES pH 7.8 (Cat. No. 02443-05, Nacalai Tesque, Inc.). The disulfide bonds in the antibody were reduced by adding 12.5 mM tris(2-carboxyethyl) phosphine (TCEP, 3.6 equivalents per one molecule of antibody) to the diluted trastuzumab and incubating at 25°C for 30 min.

Desalting was performed by adding 70 uL of the above solution per one column to a column packed with 1 mL of swollen Bio-Gel P-30 (Cat. 1504150). A solution of the compound obtained in Example 2-4 dissolved to 10 mM with dimethyl sulfoxide (8.7 equivalents per one antibody molecule) was added, and incubated at 30°C for 30 min to react the disulfide bond in the antibody with the linker of the compound. The reaction of trastuzumab with the peptide was confirmed by using [Analyzing Conditions D] described in [RP-UPLC-MS Chromatography], and the results are shown in TABLE 3-1 below.

The trastuzumab-peptide conjugate obtained in the above reaction was purified using the method described in the [Purification of Antibody-Peptide Conjugate] and trastuzumab-peptide conjugate was obtained at a concentration of 5.87 g/L and antibody yield of 0.88 mg.

Peptides with maleimide as the reactive functional group of the linker part listed in TABLE 1 were reacted with trastuzumab in the same manner using the method described above (analyzing conditions are listed in TABLE 3-1), and trastuzumab-peptide conjugates listed in TABLE 3-1 were created.

### [Example 3-11]

### Creation Method of Trastuzumab-hTfR_000894_PEG11_(Hydradine) Conjugate (Conjugate No. 60) Using Hydrazide

19.9 g/L of trastuzumab whose buffer is exchanged by using [Buffer Exchange Method A] described in Example 3-2 was diluted to 10.4 g/L with 100 mM citric acid buffer (pH 3.5). 200uL of diluted trastuzumab was mixed with the same volume of sodium periodate (Thermo Fisher Scientific) diluted to 20mM in 100mM citrate buffer as the antibody diluent and allowed to react for 30 minutes at room temperature to oxidize the sugar chains on the antibody.

After oxidation reaction, desalting was performed by adding 100 uL of the above solution per one column to a column packed with 1 mL of swollen Bio-Gel P-30 (Cat. 1504150). 7.2 uL of a solution of the compound obtained in Example 2-3 (the peptide of SEQ ID NO: 6 bounds with the linker listed in TABLE 1) dissolved to 10 mM with dimethyl sulfoxide (10 equivalents per one antibody molecule) and aniline (Wako Pure Chemical Industries, Ltd.) diluted to 50% with DMSO were added per 100uL of desalted sample solution, and incubated at 25°C for 3 to 4 hours to react with the carbonyl group on the sugar chain of the purified antibody. The reaction of trastuzumab with the peptide was confirmed by using [Analyzing Conditions C] described in [RP-UPLC-MS Chromatography], and the results are shown in TABLE 3-2 below.

The trastuzumab-peptide conjugate obtained in the above reaction was purified using the method described in the [Purification of Antibody-Peptide Conjugate] and trastuzumab-peptide conjugate was obtained at a concentration of 4.6 g/L and antibody yield of 0.23 mg.

The peptides with hydrazide as the reactive functional group in the linker part (peptides of SEQ ID NOs: 7 and 11 bound with the linkers listed in TABLE 1) were reacted with trastuzumab in the same manner using the method described above and the trastuzumab-peptide conjugates were created.

### [Example 3-12]

### Creation Method of Nivolumab-894_PEG12(NHS) Conjugate (Conjugate No. 64) Using N-Hydroxysuccinimide (NHS)

4.9 g/L of nivolumab whose buffer is exchanged by using [Buffer Exchange Method B] described in Example 3-1 was mixed with 10 mM solution of the compound obtained in Example 2-5 (peptide of SEQ ID NO: 19 binding with the linker listed in TABLE 1) dissolved in dimethyl sulfoxide (15.0 equivalents to one molecule of antibody), and incubated at 25°C for 60 min to react the amine group of the antibody with the linker of the compound. The reaction of nivolumab with the peptide was confirmed by using [Analyzing Conditions D] described in [RP-UPLC-MS Chromatography], and the results are shown in TABLE 3-3 below.

The nivolumab-peptide conjugate obtained in the above reaction was purified using the method described in the [Purification Method B] of [Purification of Antibody-Peptide Conjugate], and nivolumab-peptide conjugate was obtained at a concentration of 2.2 g/L and antibody yield of 0.11 mg.

The peptides with N-Hydroxysuccinimide as the reactive functional group in the linker part (peptides of SEQ ID NO: 4 binding with the linkers listed in TABLE 1) were reacted with nivolumab using the method described above (analyzing conditions are listed in TABLE 3-3) and the nivolumab-peptide conjugates described in TABLE 3-3 were created.

### [Example 3-13]

### Creation Method of Trastuzumab-894_PEG12(NHS) Conjugate (Conjugate No. 63) Using N-Hydroxysuccinimide (NHS)

17 g/L of trastuzumab whose buffer is exchanged by using [Buffer Exchange Method A] described in Example 3-2 was mixed with 10 mM solution of the compound obtained in Example 2-5 (peptide of SEQ ID NO: 19 binding with the linker listed in TABLE 1) dissolved in dimethyl sulfoxide (4.0 equivalents to one molecule of antibody), and incubated at 25°C for 60 min to react the amine group of the antibody with the linker of the compound. The reaction of nivolumab with the peptide was confirmed by [RP-UPLC-MS Chromatography], and the results are shown in TABLE 3-3 below.

The trastuzumab-peptide conjugate obtained in the above reaction was purified using the method described in the [Purification Method C] of [Purification of Antibody-Peptide Conjugate], and trastuzumab-peptide conjugate was obtained at a concentration of 1.2 g/L and antibody yield of 0.12 mg.

### [Example 3-14]

### Creation Method of Pertuzumab-894_3m_PEG12_dk (Maleimide) Conjugate (Conjugate No. 66) Using Maleimide

8.2 g/L of pertuzumab whose buffer is exchanged by using [Buffer Exchange and Concentration of Pertuzumab] described in Example 3-3 was diluted to 5.2 g/L with 50 mM HEPES pH 7.8 (Nacalai Tesque, Inc., Cat. No. 02443-05). The disulfide bonds in the antibody were cleaved by adding 12.5 mM tris(2-carboxyethyl) phosphine (TCEP, 12 equivalents per one molecule of antibody) to the diluted pertuzumab and incubating at room temperature for 1 hour.

Desalting was performed by adding 70 uL of the above solution per one column to a column packed with 1 mL of swollen Bio-Gel P-30 (Bio-Rad, Cat. No. 1504150). A solution of the compound obtained in Example 1 dissolved to 10 mM with dimethyl sulfoxide (10 equivalents per one antibody molecule) was added, and incubated at 30°C for 30 min to react the disulfide bond in the antibody with the linker of the compound. The reaction of pertuzumab with the peptide was confirmed by using [Analyzing Conditions E] described in [RP-UPLC-MS Chromatography], and the results are shown in TABLE 3-1 below.

### [Example 3-15]

### Creation Method of Cetuximab-894_3m_PEG12_dk (Maleimide) Conjugate (Conjugate No. 67) Using Maleimide

8.2 g/L of cetuximab whose buffer is exchanged by using [Buffer Exchange and Concentration of Cetuximab] described in Example 3-4 was diluted to 5.2 g/L with 50 mM HEPES pH 7.8 (Nacalai Tesque, Inc., Cat. No. 02443-05). The disulfide bonds in the antibody were cleaved by adding 12.5 mM tris(2-carboxyethyl) phosphine (TCEP, 12 equivalents per one molecule of antibody) to the diluted cetuximab and incubating at room temperature for 1 hour.

Desalting was performed by adding 70 uL of the above solution per one column to a column packed with 1 mL of swollen Bio-Gel P-30 (Bio-Rad, Cat. No. 1504150). A solution of the compound obtained in Example 1 dissolved to 10 mM with dimethyl sulfoxide (10 equivalents per one antibody molecule) was added, and incubated at 30°C for 30 min to react the disulfide bond in the antibody with the linker of the compound. The reaction of cetuximab with the peptide was confirmed by using [Analyzing Conditions E] described in [RP-UPLC-MS Chromatography], and the results are shown in TABLE 3-1 below.

### [Example 3-16]

### Creation Method of lpilimumab-894_3m_PEG12_dk (Maleimide) Conjugate (Conjugate No. 68) Using Maleimide

6.4 g/L of ipilimumab whose buffer is exchanged by using [Buffer Exchange and Concentration of Ipilimumab] described in Example 3-5 was diluted to 5.2 g/L with 50 mM HEPES pH 7.8 (Nacalai Tesque, Inc., Cat. No. 02443-05). The disulfide bonds in the antibody were cleaved by adding 12.5 mM tris(2-carboxyethyl) phosphine (TCEP, 12 equivalents per one molecule of antibody) to the diluted ipilimumab and incubating at room temperature for 1 hour.

Desalting was performed by adding 70 uL of the above solution per one column to a column packed with 1 mL of swollen Bio-Gel P-30 (Bio-Rad, Cat. No. 1504150). A solution of the compound obtained in Example 1 dissolved to 10 mM with dimethyl sulfoxide (10 equivalents per one antibody molecule) was added, and incubated at 30°C for 30 min to react the disulfide bond in the antibody with the linker of the compound. The reaction of ipilimumab with the peptide was confirmed by using [Analyzing Conditions E] described in [RP-UPLC-MS Chromatography], and the results are shown in TABLE 3-1 below.

### [Example 3-17]

### Creation Method of Atezolizumab-894_3m_PEG12_dk (Maleimide) Conjugate (Conjugate No. 69) Using Maleimide

6.4 g/L of atezolizumab whose buffer is exchanged by using [Buffer Exchange and Concentration of Atezolizumab] described in Example 3-6 was diluted to 5.2 g/L with 50 mM HEPES pH 7.8 (Nacalai Tesque, Inc., Cat. No. 02443-05). The disulfide bonds in the antibody were cleaved by adding 12.5 mM tris(2-carboxyethyl) phosphine (TCEP, 12 equivalents per one molecule of antibody) to the diluted atezolizumab and incubating at room temperature for 1 hour.

Desalting was performed by adding 70 uL of the above solution per one column to a column packed with 1 mL of swollen Bio-Gel P-30 (Bio-Rad, Cat. No. 1504150). A solution of the compound obtained in Example 1 dissolved to 10 mM with dimethyl sulfoxide (10 equivalents per one antibody molecule) was added, and incubated at 30°C for 30 min to react the disulfide bond in the antibody with the linker of the compound. The reaction of atezolizumab with the peptide was confirmed by using [Analyzing Conditions E] described in [RP-UPLC-MS Chromatography], and the results are shown in TABLE 3-1 below.

### [Example 3-18]

### Creation Method of Pembrolizumab-894_3m_PEG12_dk (Maleimide) Conjugate (Conjugate No. 70) Using Maleimide

6.4 g/L of pembrolizumab whose buffer is exchanged by using [Buffer Exchange and Concentration of Pembrolizumab] described in Example 3-7 was diluted to 5.2 g/L with 50 mM HEPES pH 7.8 (Nacalai Tesque, Inc., Cat. No. 02443-05). The disulfide bonds in the antibody were cleaved by adding 125 mM tris(2-carboxyethyl) phosphine (TCEP, 120 equivalents per one molecule of antibody) to the diluted cetuximab and incubating at 37°C for 1 hour.

Desalting was performed by adding 70 uL of the above solution per one column to a column packed with 1 mL of swollen Bio-Gel P-30 (Bio-Rad, Cat. No. 1504150). This desalting was performed twice in total. A solution of the compound obtained in Example 1 dissolved to 10 mM with dimethyl sulfoxide (10 equivalents per one antibody molecule) was added, and incubated at 30°C for 30 min to react the disulfide bond in the antibody with the linker of the compound. The reaction of pembrolizumab with the peptide was confirmed by using [Analyzing Conditions E] described in [RP-UPLC-MS Chromatography], and the results are shown in TABLE 3-1 below.

### [Example 3-19]

### Creation Method of Trastuzumab-894_3m_PEG12_dk (Maleimide) Conjugate (Conjugate No. 71) Using Maleimide

10.0 g/L of trastuzumab whose buffer is exchanged by using [Buffer Exchange Method B] described in Example 3-2 was diluted to 5.2 g/L with 50 mM HEPES pH 7.8 (Nacalai Tesque, Inc., Cat. No. 02443-05). The disulfide bonds in the antibody were cleaved by adding 12.5 mM tris(2-carboxyethyl) phosphine (TCEP, 12 equivalents per one molecule of antibody) to the diluted cetuximab and incubating at 37°C for 1 hour.

Desalting was performed by adding 70 uL of the above solution per one column to a column packed with 1 mL of swollen Bio-Gel P-30 (Bio-Rad, Cat. No. 1504150). A solution of the compound obtained in Example 1 dissolved to 10 mM with dimethyl sulfoxide (10 equivalents per one antibody molecule) was added, and incubated at 30°C for 30 min to react the disulfide bond in the antibody with the linker of the compound. The reaction of trastuzumab with the peptide was confirmed by using [Analyzing Conditions E] described in [RP-UPLC-MS Chromatography], and the results are shown in TABLE 3-1 below.

### [Example 3-20]

### Creation Method of Nivolumab-894_3m_PEG12_dk (Maleimide) Conjugate (Conjugate No. 72) Using Maleimide

11.1 g/L of nivolumab whose buffer is exchanged by using [Buffer Exchange Method C] described in Example 3-1 was diluted to 5.2 g/L with 50 mM HEPES pH 7.8 (Nacalai Tesque, Inc., Cat. No. 02443-05). The disulfide bonds in the antibody were cleaved by adding 125 mM tris(2-carboxyethyl) phosphine (TCEP, 120 equivalents per one molecule of antibody) to the diluted cetuximab and incubating at 37°C for 1 hour.

Desalting was performed by adding 70 uL of the above solution per one column to a column packed with 1 mL of swollen Bio-Gel P-30 (Bio-Rad, Cat. No. 1504150). This desalting was performed twice in total. A solution of the compound obtained in Example 1 dissolved to 10 mM with dimethyl sulfoxide (10 equivalents per one antibody molecule) was added, and incubated at 30°C for 30 min to react the disulfide bond in the antibody with the linker of the compound. The reaction of nivolumab with the peptide was confirmed by using [Analyzing Conditions E] described in [RP-UPLC-MS Chromatography], and the results are shown in TABLE 3-1 below.

### [Example 3-21]

### Mass Spectrometry of Antibody-peptide Conjugate Prepared with Maleimide

Antibody-peptide conjugates were prepared using the peptides listed in TABLE 3-1 by the conjugation procedures described in Examples 3-8 to 3-10 and 3-14 to 3-20. The prepared antibody-peptide conjugates were analyzed by the method described in the [RP-UPLC-MS Chromatography] section, and the analysis results are shown in TABLE 3-1. The conditions used for the analysis of each antibody-peptide conjugate are shown in the "Analyzing Condition" section of the TABLE. The disulfide bonds of the antibody-peptide conjugates prepared using the thiol-maleimide reaction are cleaved at the peptide-bound portion, and under the conditions used to analyse the antibody-peptide conjugates in the TABLE, it is analyzed as "heavy chain-peptide fragment", "light chain-peptide fragment" or "light and heavy chain complex-peptide fragment". In the table, "L" represents the light chain of the antibody, "H" represents the heavy chain, "P" in "nP" represents the peptide added, and "n" (where n is an integer) represents the number of peptides added. In the TABLE, "LP" represents a fragment with one peptide added to the light chain, "H3P" represents a fragment with three peptides added to the heavy chain, "H4P" represents a fragment with four peptides added to the heavy chain, "LH2P" represents a fragment with two peptides added to the complex of light chain and heavy chains, and "LH3P" represents a fragment with three peptides added to the complex of light chain and heavy chains. In the TABLE, "ND" indicates not detected. The molecular weight (calculated) of each fragment of each antibody-peptide conjugate was calculated by using molecular weights of the light chain and heavy chain obtained by analyzing trastuzumab or nivolumab with the reduction treatments described in the conjugation procedures in Examples 3-3, 3-4 and 3-5 using the analytical methods described in the TABLE and the molecular weights of the peptides indicated by the SEQ ID NOs: in TABLE 3-1.

Each fragment of the antibody-peptide conjugates whose value is described in the "Measured Value" column in the TABLE has an area ratio of 10% or more in the liquid chromatography chromatogram, and fragments with an area ratio lower than 10% are "ND" or undescribed. In addition, the percentage of unreacted antibody in the conjugates listed in the TABLE is less than or equal to 5% of the area ratio in the chromatograms.

### [Example 3-22]

### Mass Spectrometry of Antibody-peptide Conjugate Prepared with Hydrazide

Antibody-peptide conjugates were prepared using the peptides listed in TABLE 3-2 by the conjugation procedures described in Examples 3-11. The prepared antibody-peptide conjugates were analyzed by the method described in the [RP-UPLC-MS Chromatography] section, and the analysis results are shown in TABLE 3-2. The conditions used for the analysis of each antibody-peptide conjugate are shown in the "Analyzing Condition" section of the TABLE. In the table, "P" in "nP" represents the peptide added, and "n" (where n is an integer) represents the number of peptides added. The "Antibody" column in the TABLE indicates the type of antibody used in the conjugation, and "1P" to "9P" indicate the number of peptides added per one molecule of antibody, respectively. In other words, "1P" indicates the structure in which one molecule of peptide is added to one molecule of antibody, "2P" indicates the structure in which two molecules of peptide are added to one molecule of antibody, and others are similar. "ND" in the TABLE indicates not detected.

The values in the TABLE are divided into upper and lower columns for one conjugate, and the upper column is the molecular weight (Calculated Value) of each antibody-peptide conjugate, which is calculated from the molecular weight obtained by analyzing trastuzumab or nivolumab using the analytical method described in the TABLE and the molecular weight of the peptide indicated by the SEQ ID NO: in TABLE 3-2. The molecular weight of G0F/G1F was used to determine the molecular weight (calculated value) of the trastuzumab-peptide conjugate, although there are multiple sugar chains of different types in trastuzumab.

The lower column is "Measured Value," and antibody-peptide conjugates whose values are listed in the Measured Values column have an area ratio of 10% or more in the liquid chromatograms. The ratio of unreacted antibodies in the conjugates listed in the TABLE are all less than or equal to 5% of the area ratio in the chromatograms.

In other words, in the case of Conjugate No. 60 (trastuzumab-hTfR_000894_PEG11_(Hydradine) conjugate), since 150914 in the upper column of 1P which is the structure of trastuzumab with one molecule of peptide added is a calculated value, while the Measured Value of 151031 in the lower column was obtained, it certainly shows that trastuzumab with one molecule of peptide added was obtained.

### [Example 3-23]

### Mass Spectrometry of Antibody-peptide Conjugate Prepared with N-Hydroxysuccinimide (NHS)

Antibody-peptide conjugates were prepared using the peptides listed in TABLE 3-3 by the conjugation procedures described in Examples 3-12 and 3-13. The prepared antibody-peptide conjugates were analyzed by the method described in the [RP-UPLC-MS Chromatography] section, and the analysis results are shown in TABLE 3-3. The conditions used for the analysis of each antibody-peptide conjugate are shown in the "Analyzing Condition" section of the TABLE. In the table, "P" in "nP" represents the peptide added, and "n" (where n is an integer) represents the number of peptides added. The "Antibody" column in the TABLE indicates the type of antibody used in the conjugation, and "1P" to "9P" indicate the number of peptides added per one molecule of antibody, respectively. In other words, "1P" indicates the component in which one molecule of peptide is added to one molecule of antibody, "2P" indicates the component in which two molecules of peptide are added to one molecule of antibody, and others are similar. "ND" in the TABLE indicates not detected.

The values in the TABLE are divided into upper and lower columns for one conjugate, and the upper column is the molecular weight (Calculated Value) of each antibody-peptide conjugate, which is calculated from the molecular weight obtained by analyzing trastuzumab or nivolumab using the analytical method described in the TABLE and the molecular weight of the peptide indicated by the SEQ ID NO: in TABLE 3-3. Although there are multiple types of trastuzumab containing multiple sugar chains of different types, the molecular weight (Calculated Value) of the trastuzumab-peptide conjugate was determined by using molecular weight of G0F/G1F (G0 is the abbreviation for a sugar chain containing no galactose, G1 for one containing one galactose, G2 for one containing two galactoses, and when fucose is contained, they are denoted as G0F, G1F, and G2F. G0F/G1F refers to an antibody with one G0F and one G1F sugar chain).

The lower column is "Measured Value," and antibody-peptide conjugates whose values are listed in the Measured Values column have an area ratio of 10% or more in the liquid chromatograms. The ratio of unreacted antibodies in the conjugates listed in the TABLE are all less than or equal to 5% of the area ratio in the chromatograms.

In other words, in the case of Conjugate No. 63 (trastuzumab-894_PEG12_(NHS) conjugate), since 150887 in the upper column of 1P which is the structure of trastuzumab with one molecule of peptide added is a calculated value, whereas the measured value in the lower column was not obtained, it means that trastuzumab with one molecule of peptide was not obtained, on the other hand, in the 2P which is the structure of trastuzumab with two molecules of peptide added, the calculated value was 153551 while the measured value of 153506 was obtained, it means that trastuzumab with two molecules of peptide was indeed obtained.

**[TABLE 3-1]**

| [TABLE 3-1-1] | | | | | | | | | | |
|---|---|---|---|---|---|---|---|---|---|---|
| Conjugate No | SEQ ID No. | Peptide Name | Antibody | | LP | LH2P | LH3P | K3P | H4P | Analyzing Condition |
| 1 | 1 | hTfR_000894_PEG11_K(Maleimide) | Trastuzumab | Calculated Value | 26472 | 80100 | 83130 | 59688 | 62717 | D |
| | | | | Measured Value | 26472 | 00099 | ND | 59690 | ND | |
| 2 | 2 | hTfR_000894_PEG36_K(Maleimide) | Trastuzumab | Calculated Value | 27529 | 82215 | 86302 | 62860 | 66946 | C |
| | | | | Measured Value | 27526 | 82207 | ND | 62856 | ND | |
| 3 | 3 | hTfR_894_A11PEG11K(Maleimide) | Trastuzumab | Calculated Value | 26386 | 79930 | 82874 | 59432 | 62377 | D |
| | | | | Measured Value | 26385 | 79924 | ND | 59431 | ND | |
| 4 | 20 | 894_3m_10A_GGRGRS_K(Mal) | Nivolumab | Calculated Value | 26342 | 78985 | 81957 | 58567 | 61560 | A |
| | | | | Measured Value | 26343 | 78992 | ND | 58595 | NO | |
| 5 | 21 | 894_3m_6A_GGRGRS_K(Mal) | Nivolumab | Calculated Value | 26341 | 78983 | 81954 | 58584 | 61556 | A |
| | | | | Measured Value | 26342 | 78990 | ND | 58592 | NO | |
| 6 | 22 | 894_variant_03_GGRGRS_K(Mal) | Nivolumab | Calculated Value | 26431 | 79162 | 82223 | 58853 | 61914 | A |
| | | | | Measured Value | 26432 | 79168 | ND | 58860 | ND | |
| 7 | 23 | 894_Bicycle_002_GGRGRS_K(Mal) | Nivolumab | Calculated Value | 26445 | 79190 | 82265 | 58895 | 61970 | A |
| | | | | Measured Value | 26446 | 79197 | ND | 58903 | ND | |
| 8 | 34 | 894_3m_PEG04_K(Mal) | Nivolumab | Calculated Value | 26059 | 78491 | 81183 | 57815 | 60506 | A |
| | | | | Measured Value | 26059 | 78489 | ND | 57814 | ND | |
| 8 | 35 | 894_3m_PEG08_K(Mal) | Nivolumab | Calculated Value | 26235 | 78843 | 81710 | 58342 | 61210 | A |
| | | | | Measured Value | 26235 | 78841 | ND | 58342 | NO | |
| 10 | 36 | 684_3m_PEG12_K(Mal) | Nivolumab | Calculated Value | 26412 | 79195 | 82239 | 58871 | 61914 | A |
| | | | | Measured | 26411 | 79193 | ND | 58871 | ND | |
| 11 | 37 | 894_3m_G4S2_K(Mal) | Nivolumab | Calculated Value | 26215 | 78733 | 81580 | 58212 | 61059 | A |
| | | | | Measured Value | 26214 | 78729 | ND | 58209 | ND | |
| 12 | 38 | 894_5m_PEG04_K(Mal) | Nivolumab | Calculated Value | 26059 | 78491 | 81183 | 57815 | 60506 | A |
| | | | | Measured Value | 26059 | 78490 | ND | 57814 | ND | |
| 13 | 39 | 894_5m_PEG08_K(Mal) | Nivolumab | Calculated Value | 26235 | 78841 | 81711 | 58343 | 61211 | A |
| | | | | Measured Value | 26235 | 78842 | ND | 58343 | ND | |
| **14** | 40 | 894_5m_PEG12_K(Mal) | Nivolumab | Calculated Value | **26412** | 79195 | 82239 | 58871 | 61914 | A |
| | | | | Measured Value | 26411 | 79193 | ND | 58871 | ND | |
| 15 | 41 | 894_5m_G4S2_K(Mal) | Nivolumab | Calculated Value | 26215 | 78804 | 81651 | 58283 | 61130 | A |
| | | | | Measured Value | 26214 | 78800 | 81646 | 58280 | ND | |
| 16 | 42 | 894_3_5m_PEG04_K(Mal) | Nivolumab | Calculated Value | 26073 | 78519 | 81225 | 57857 | 60562 | A |
| | | | | Measured Value | 26072 | 78518 | 81224 | 57857 | ND | |
| 17 | 43 | 894_3_5m_PEG08_K(Mal) | Nivolumab | Calculated Value | 26249 | 76872 | 81753 | 58385 | 61267 | A |
| | | | | Measured Value | 26249 | 78870 | ND | 58386 | ND | |
| 18 | 44 | 894_3 5m_PEG12 K(Mal) | Nivolumab | Calculated Value | 26426 | 79223 | 82281 | 58913 | 61971 | A |
| | | | | Measured Value | 26425 | 79221 | 82279 | 58913 | ND | |
| 19 | 46 | 894_3_5m_G4S2_K(Mal) | Nivolumab | Calculated Value | 26229 | 78832 | 81693 | 58325 | 61186 | A |
| | | | | Measured Value | 26228 | 78828 | 81689 | 58322 | 61182 | |
| 20 | 46 | 894_K11_PEG4c_KTrzMal | Nivolumab | Calculated Value | 25959 | 78291 | 80882 | 57514 | 60105 | A |
| | | | | Measured Value | 25959 | 78289 | 52603 I | 57515 | ND | |

**[TABLE 3-1-2]**

| | | | | | | | | | | |
|---|---|---|---|---|---|---|---|---|---|---|
| 21 | 47 | 894_K11_PEG8c_KTrzMal | Nivolumab | Calculated Value | 26135 | 78643 | 81411 | 58043 | 60810 | A |
| | | | | Measured Value | 26135 | 78643 | ND | 58045 | ND | |
| 22 | 48 | 894_K11_PEG12c_KTrzMal | Nivolumab | Calculated Value | 26311 | 78996 | 81939 | 58571 | 61515 | A |
| | | | | Measured Value | 26311 | 78994 | **ND** | **58576** | **ND** | |
| 23 | 49 | 894_K11_G4S2_KTrzMal | Nivolumab | Calculated Value | 26114 | 78601 | 81347 | 57979 | 60725 | A |
| | | | | Measured Value | **26114** | 78602 | **ND** | **57981** | ND | |
| **24** | 52 | 894_11K_3MeF_PEG12c_KTrzMal | Nivolumab | Calculated Value | 26325 | 79024 | 81981 | 58613 | 61571 | A |
| | | | | Measured Value | 26325 | 79024 | ND | 58617 | ND | |
| 25 | 53 | 894_11K_3MeF_G4S2_KTrzMal | Nivolumab | Calculated Value | 26128 | 78629 | 81389 | 58021 | 60782 | A |
| | | | | Measured Value | 26128 | 78629 | ND | 58024 | ND | |
| 26 | 58 | 894_3m_G2SGSG2SGS_K(Mal) | Nivolumab | Calculated Value | 26677 | 79657 | 82966 | 59598 | 62907 | A |
| | | | | Measured Value | 26676 | 79658 | **ND** | 59597 | ND | |
| 27 | 59 | 894_3m_G2SGSGSS_K(Mal) | Nivolumab | CalculatedValue | 26478 | 79256 | 82363 | 58993 | 62100 | A |
| | | | | Measured Value | 26475 | 79252 | ND | 58993 | **ND** | |
| 28 | 60 | 894_3m_GGSGSS_K(Mal) | Nivolumab | Calculated Value | 26244 | 78791 | 81667 | 58299 | 61175 | A |
| | | | | Measured Value | **26244** | 78789 | 61665 | 58299 | ND | |
| 29 | 62 | 894_3m_GGSSGES_K(Mal) | Nivolumab | Calculated Value | 26286 | 78875 | 81793 | 58425 | 61343 | A |
| | | | | Measured Value | 26286 | 78874 | 81792 | 58426 | ND | |
| 30 | 63 | 894_3m_GGEGES_K(Mal) | Nivolumab | Calculated Value | 26360 | 79021 | 82011 | 58641 | 61632 | A |
| | | | | Measured Value | 26358 | 79018 | ND | 58642 | **ND** | |
| 31 | 64 | 894_3m_GGGSS_K(Mal) | Nivolumab | Calculated Value | 26157 | 78616 | **81406** | 58038 | 60827 | A |
| | | | | Measured Value | 26157 | 78616 | ND | 58039 | ND | |
| 32 | 65 | 894_3m_GGSS_K(Mal) | Nivolumab | Calculated Value | 26100 | 78502 | 81234 | 57866 | 60599 | A |
| | | | | Measured Value | 26100 | 78501 | 81234 | 57866 | ND | |
| 33 | 66 | 894_3m_GGS_K(Mal) | Nivolumab | Calculated Value | 26013 | 78328 | 80973 | 57605 | 60250 | A |
| | | | | Measured Value | 26013 | 78327 | 80973 | 57606 | ND | |
| 34 | 67 | 894_3m_GG_K(Mal) | Nivolumab | Calculated Value | 25926 | **78154** | 80712 | 57344 | 59902 | A |
| | | | | Measured Value | 25926 | 78153 | 80711 | **57345** | 59904 | |
| 35 | **68** | 894_3m_G_K(Mal) | Nivolumab | Calculated Value | 25869 | 78040 | **80541** | 57173 | 59674 | A |
| | | | | Measured Value | 25869 | 78039 | 80540 | 57173 | 59674 | |
| 36 | 69 | 894_3m_K(Mal) | Nivolumab | Calculated Value | 25812 | 77926 | 80370 | 57002 | 59446 | A |
| | | | | Measured Value | 25812 | 77925 | **ND** | 57002 | ND | |
| 37 | **70** | 894_3m_PEG4_PEG4_K(Mal) | Nivolumab | Calculated Value | 26306 | 78917 | 81855 | 58487 | 61426 | A |
| | | | | Measured Value | 26306 | 78915 | 81852 | 58486 | ND | |
| 38 | 71 | 894_3m_PEG4_PEG4_PEG4_K(Mal) | Nivolumab | Calculated Value | **26554** | 79412 | 82597 | 59229 | 62415 | A |
| | | | | Measured Value | 26553 | 79409 | ND | 59228 | ND | |
| 39 | 72 | 894_5m_GSSGSS_K(Mal) | Nivolumab | Calculated Value | 26274 | 78853 | 81759 | 58391 | 61297 | A |
| | | | | Measured Value | 26274 | 78851 | ND | 58390 | NO | |
| 40 | 73 | 894_5m_GGRGRS_K(Mal) | Nivolumab | Calculated Value | 26383 | 79069 | 82084 | 58716 | 61730 | A |
| | | | | Measured Value | 26382 | 79067 | **ND** | 58714 | ND | |
| 41 | 74 | 8964_5m_GGSGES_K(Mal) | Nivolumab | Calculated Value | 26286 | 78877 | 81795 | 58427 | 61345 | A |
| | | | | Measured Value | 26286 | **78877** | **NO** | 58425 | ND | |

**[TABLE 3-1-4]**

| | | | | | | | | | | |
|---|---|---|---|---|---|---|---|---|---|---|
| 42 | 75 | 894_5m_GGRSES_K(Mal) | Nivolumab | Calculated Value | 26385 | 79075 | 82092 | 58724 | 61742 | A |
| | | | | Measured Value | 26385 | 79075 | ND | 58724 | ND | |
| 43 | 82 | 894_3m_GGRGRS_K(Mal) | Nivolumab | Calculated Value | 26384 | 79071 | 82086 | 58717 | 61731 | A |
| | | | | Measured Value | 26384 | 79069 | NΩ | 58716 | ND | |
| 44 | 83 | 894_5m_GG_C(Mal) | Nivolumab | Calculated Value | 25928 | 78156 | 80714 | 57344 | 59902 | A |
| | | | | measured Value | 25927 | 78155 | ND | 57346 | ND | |
| **45** | 84 | 894_5m_GGRGRS_K(Mal) | Nivolumab | Calculated Value | 26385 | 79069 | 82084 | 58714 | 61728 | A |
| | | | | Measured Value | 26383 | 79068 | ND | 58716 | ND | |
| 46 | 85 | 894_3m_5m_GG_K(Mal) | Nivolumab | Calculated Value | 25942 | 78184 | 80756 | 57386 | 59958 | A |
| | | | | Measured Value | 25941 | 78184 | ND | 57388 | ND | |
| 47 | 86 | 894_3m_5m_GGRGRS_K(Mal) | Nivolumab | Calculated Value | 26399 | 79097 | 82126 | 58756 | 61784 | A |
| | | | | Measured Value | 26397 | 79096 | ND | 58758 | ND | |
| 48 | 88 | 36_GGRGRS_K(Mal) | Nivolumab | Calculated Value | 25805 | 77910 | 80344 | 56974 | 59409 | A |
| | | | | Measured Value | 25803 | 77910 | ND | 56979 | ND | |
| 49 | 89 | 36_G4S2_K(Mal) | Nivolumab | Calculated Value | 25637 | 77573 | 79840 | 56470 | 58736 | A |
| | | | | Measured Value | 25835 | 77573 | NO | 56472 | ND | |
| 50 | 90 | 894_3m_1Abu_GG_K(Mal) | Nivolumab | Calculated Value | 25942 | 78184 | 80756 | 57386 | 59958 | A |
| | | | | Measured Value | 25941 | 78184 | ND | 57389 | ND | |
| 51 | 91 | 894_3m_1Abu_GGRGRS_K(Mal) | Nivolumab | Calculated Value | 26399 | 79097 | 82126 | 58756 | 61784 | A |
| | | | | Measured Value | 26397 | 79097 | ND | 58758 | ND | |
| 52 | 92 | 894_3m_1Abu_G4S2_K(Mal) | Nivolumab | Calculated Value | 28230 | 78761 | 81621 | 58251 | 61111 | A |
| | | | | Measured Value | 26229 | 78760 | ND | 58254 | ND | |
| 53 | 93 | 894_3m_8Ahp_GG_K(Mal) | Nivolumab | Calculated Value | 25892 | 78084 | 80606 | 57236 | 59758 | A |
| | | | | Measured Value | 25891 | 78084 | ND | 57239 | ND | |
| 54 | 94 | 894_3m_8Ah_GGRGRS_K(Mal) | Nivolumab | Calculated Value | 26349 | 78997 | 81976 | 58606 | 61584 | A |
| | | | | Measured Value | 26347 | 78999 | ND | 58610 | ND | |
| 55 | 95 | 894_3m_8Ah_G4S2_K(Mal) | Nivolumab | Calculated Value | 26180 | 78661 | 81471 | 58101 | 60911 | A |
| | | | | Measured Value | 26179 | 78660 | ND | 58103 | ND | |
| 56 | 108 | 894_G4S2_K(Mal) | Nivolumab | Calculated Value | 26201.22 | 78774.44 | 81607.66 | 58239.66 | 61072.68 | A |
| | | | | Measured Value | 26199 | 78771 | ND | 58237 | ND | |
| **57** | 109 | 894_PEG8_K(mal) | Nivolumab | Calculated Value | 26221.38 | 78814.76 | 81668.14 | 58300.14 | 61153.52 | A |
| | | | | Measured Value | 26221 | 78813 | 81667 | 38300 | ND | |
| 58 | 1 | 894_PEG12_K(Mal) | Nivolumab | Calculated Value | 26397.59 | 79167.18 | 82196.77 | 58828.77 | 61858.36 | A |
| | | | | Measured Value | 26397 | 79165 | ND | 58828 | ND | |
| 59 | 110 | 894_PEG4_K(Mal) | Nivolumab | Calculated Value | 26045.17 | 78463.34 | 81140.51 | 57772.51 | 60449.68 | A |
| | | | | Measured Value | 26044 | 78462 | 81139 | 57773 | 60450 | |

**[TABLE 3-1-4]**

| | | | | | | | | | | |
|---|---|---|---|---|---|---|---|---|---|---|
| 66 | 36 | 894_3m_PEG12_K(Mal) | Pertuzumab | Calculated Value | 26564 | 80129 | 83173 | 59653 | 62696 | E |
| | | | | Measured Value | 26563 | ND | ND | 59651 | ND | |
| 67 | 36 | 894_3m_PEG12_K(Mal) | Cetuximab | Calculated Value | 26464 | 81612 | 84656 | 61236 | 64279 | E |
| | | | | Measured Value | 26463 | ND | ND | 61234 | ND | |
| 68 | 36 | 894_3m_PEG12_K(Mal) | Ipilimumab | Calculated Value | 26492 | 80025 | 83069 | 59621 | 62664 | E |
| | | | | Measured Value | 26492 | ND | ND | 59618 | ND | |
| 69 | 36 | 894_3m_PEG12_K(Mal) | Atezolizumab | Calculated Value | 26403 | 78263 | 81307 | 57948 | 60991 | E |
| | | | | Measured Value | 26402 | ND | ND | 57947 | 60992 | |
| 70 | 36 | 894_3m_PEG12_K(Mal) | Pembrolizumab | Calculated Value | 26782 | 80533 | 83577 | 56794 | 59838 | E |
| | | | | Measured Value | 26782 | 80530 | ND | ND | 59836 | |
| 71 | 36 | 894_3m_PEG13_K(Mal) | Trastuzumab | Calculated Value | 26481 | 80114 | 83158 | 59721 | 62764 | E |
| | | | | Measured Value | 26480 | ND | ND | 59720 | ND | |
| 72 | 36 | 894_3m_PEG14_K(Mal) | Nivolumab | Calculated Value | 26411 | 82240 | 82240 | 58873 | 61916 | E |
| | | | | Measured Value | 26411 | ND | ND | 58871 | ND | |

### [TABLE 3-2]

**[TABLE 3-2]**

| **Conjugate No.** | **SEQ ID No.** | **Peptide Name** | **Antibody** | **1 P** | **2P** | **3P** | **4P** |
|---|---|---|---|---|---|---|---|
| 60 | 6 | hTfR_000894_PEG11_(Hydradine) | Trastuzumab | 150914 | 153605 | 156297 | 158988 |
| | | | | 151031 | 153756 | 156470 | ND |
| 61 | 7 | hTfR_000894_P E G 36_(H ydrazine) | Trastuzumab | 151971 | 155720 | 159468 | 163717 |
| | | | | 152115 | 155859 | 159608 | ND |
| 62 | 11 | hTfR_894_(GE)PEG12_Hydrazide | Trastuzumab | 151100 | 153978 | 156855 | 159732 |
| | | | | 151192 | 154099 | 156988 | ND |

| 5P | 6P | 7P | 8P | 9P | Analyzing Condition | | |
|---|---|---|---|---|---|---|---|
| 161679 | 164370 | 167061 | 169753 | 172444 | C | | |
| ND | ND | ND | ND | ND | | | |
| 166965 | 170714 | 174462 | 178211 | 181959 | C | | |
| ND | ND | ND | ND | ND | | | |
| 162610 | 165487 | 168365 | 171242 | 174119 | C | | |
| ND | ND | ND | ND | ND | | | |

### [TABLE 3-3]

**[TABLE 3-3]**

| Conjugate No. | SEQ ID No. | Peptide Name | Antibody | 1 P | 2 P | 3P | 4P | 5P |
|---|---|---|---|---|---|---|---|---|
| 63 | 19 | 894_PEG12_(NHS) | Trastuzumab | 150887 | 153551 | 156217 | 158882 | 161547 |
| | | | | ND | 153506 | 156188 | 158859 | 161525 |
| 64 | 19 | 894_PEG12_(NHS) | Nivolumab | 148882 | 151547 | 154213 | 156878 | 159543 |
| | | | | ND | ND | ND | 156890 | 159655 |
| 65 | 4 | JCR_hTfR_000894_PEG36_(NHS) | Nivolumab | 149966 | 153606 | 157245 | 160885 | 164524 |
| | | | | ND | ND | ND | 161150 | 164885 |

| 6P | 7P | 8P | 9P | 10P | | | | |
|---|---|---|---|---|---|---|---|---|
| 164212 | 166878 | 169543 | 172208 | 174873 | | | | |
| 164194 | 166872 | 169543 | 172224 | 174886 | | | | |
| 162208 | 164874 | 167539 | 170204 | 172869 | | | | |
| 162295 | 164935 | 167630 | 170300 | 172960 | | | | |
| 168164 | 171803 | 175443 | 179082 | 182722 | | | | |
| 168610 | 172320 | 176045 | 179780 | 183510 | | | | |

| 11P | 12P | 13P | 14P | 15P | Analyzing Condition | | | |
|---|---|---|---|---|---|---|---|---|
| 177539 | 180204 | 182869 | 185534 | 188199 | C | | | |
| ND | ND | ND | ND | ND | | | | |
| 175535 | 178200 | 180865 | 183530 | 186195 | D | | | |
| 175650 | 178325 | 180995 | 183655 | 186320 | | | | |
| 186361 | 190001 | 193640 | 197279 | 200919 | C | | | |
| 187270 | 191010 | ND | ND | ND | | | | |

### [Example 4-1]

### Bindability Test of PD-1 to Nivolumab-peptide Conjugate Using AlphaLISA

The bindability of anti-PD-1 antibody (nivolumab)-peptide conjugates to the antigen PD-1 was measured by AlphaLISA for various trastuzumab-peptide conjugates. The measurement was performed according to the manual attached with the AlphaLISA Human PD-1 and PD-L1 binding kit (PerkinElmer, AL356F). Specifically, for 2.5 uL of nivolumab-peptide conjugates diluted with the buffer attached to 384 Alpha Plate kit to 5.31E-02 g/L, 1.77E-02 g/L, 5.90E-03 g/L, 1.97E-03 g/L, 6.56E-04 g/L, 2.19E-04 g/L, 7.29E-05 g/L, 2.43E-05 g/L, 8.10E-06 g/L, 2.70E-06 g/L, 20nM 4×His Tagged PD-L1 2.5uL and 20nM 4×Biotinylated PD-1 2.5uL attached to the kit are added, then 2.5 uL of the solution in which 5 mg/mL Anti-6×His Acceptor beads, 5 mg/mL Streptavidin Donar beads and 1×Immunoassay buffer, all of these attached to the kit, are mixed in the ratio of 1:2:122, is added, and the mixture was incubated in the dark at room temperature for 90 min. After incubation, fluorescence was measured with a plate reader (Enspir (trademark), Perkin Elmer). The IC50 of each nivolumab-peptide conjugate was calculated from the obtained measured value and the results were listed in TABLE 4.

### [TABLE 4]

**[TABLE 4]**

| Conjugate No. | PD1-PDL1 IC50 (ng/mL) |
|---|---|
| Nivolumab (control) | 57.5∼195.0 |
| 56 | 489.0 |
| 59 | 126.0 |
| 57 | 437.0 |
| 58 | 91.2 |
| 11 | 347.0 |
| 8 | 148.0 |
| 9 | 389.0 |
| 10 | 355.0 |
| 15 | 97.7 |
| 12 | 128.0 |
| 13 | 112.0 |
| 14 | 417.0 |
| 19 | 123.0 |
| 16 | 191.0 |
| 17 | 407.0 |
| 23 | 162.0 |
| 26 | 74.1 |
| 27 | 135.0 |
| 28 | 20.4 |
| 29 | 23.4 |
| 30 | 61.7 |
| 31 | 20.9 |
| 32 | 43.7 |
| 33 | 63.1 |
| 34 | 70.8 |
| 35 | 81.2 |
| 37 | 81.2 |
| 38 | 79.4 |
| 39 | 148.0 |
| 40 | 93.3 |
| 41 | 91.2 |
| 42 | 91.2 |
| 54 | 89.1 |
| 43 | 177.8 |
| 48 | 120.2 |
| 51 | 190.5 |
| 45 | 239.9 |
| 47 | 128.8 |
| 52 | 229.1 |
| 55 | 177.8 |
| 50 | 144.5 |

These results indicate that the bindability of the antibody to the antigen is not lost when the peptide is conjugated.

### [Example 4-2]

### Evaluation Test of Molecular Interaction between HER-2 and Trastuzumab-Peptide Conjugate by Surface Plasmon Resonance (SPR)

For the various trastuzumab-peptide conjugates, the surface plasmon resonance (SPR) interactions of the anti-HER-2 antibody (trastuzumab)-peptide conjugate against the antigen HER-2 were tested according to the methods described below. Specific test methods are listed below.

NTA sensor chip (Global Life Sciences Technologies Japan K.K.) was inserted into BiacoreT200 (Global Life Sciences Technologies Japan K.K.) and priming operation was performed three times by using running buffer: 10 mM HEPES pH 8.0 (Nacalai Tesque, Inc.), 150 mM NaCl (Nacalai Tesque, Inc.), 0.05% Tween 20 (Nacalai Tesque, Inc.), and 0.1% BSA (SIGMA-ALDRICH), and equilibration is performed at a flow rate of 30 µL/min. 350 mM EDTA solution was reacted at a flow rate of 10 µL/min for 60 seconds, then 0.5 mM NiCl₂ solution (Kishida Chemical Inc.) was reacted at a flow rate of 10 µL/min for 60 seconds, then the NTA sensor chip was washed with 3 mM EDTA solution (Nacalai Tesque, Inc.) at a flow rate of 10 µL/min for60 seconds. 60 mM EDC solution (Global Life Sciences Technologies Japan K.K.) and 650 mM NHS solution (Global Life Sciences Technologies Japan K.K.) were mixed in 50 µL each and reacted at a flow rate of 10 µL/min for 420 seconds. 150 µL of 0.1 µM HER-2 (R&D SYSTEMS) solution was prepared by diluting with running buffer and reacted at a flow rate of 10 µL/min for 60 seconds to immobilize HER-2 on the NTA sensor chip. After immobilization, capping was performed by reacting with 1.0 M ethanolamine solution (Global Life Sciences Technologies Japan K.K.) at a flow rate of 10 µL/min for 420 seconds. Trastuzumab-peptide conjugate (Conjugate No. 1; trastuzumab-hTfR_000894_PEG11_K (Maleimide)) lysate prepared in Example 3-5, prepared at 10 mM in DMSO solution, was diluted with running buffer to a final concentration of 10 µM, and then 100 nM, 50 nM, 25 nM, 10 nM, and 5 nM dilution solutions were prepared. Using the samples described above, the kinetics of the peptide against HER-2 was obtained by SPR measurement.
As the kinetic evaluation model, Single Cycle Kinetics was used, and the binding affinity of the trastuzumab-peptide conjugate of Conjugate No. 1 to HER-2 was evaluated by obtaining the KD value from curve fitting by the least squares method using Biacore T200 Evaluation Software Version 3.0 (Global Life Sciences Technologies Japan K.K.), and the Kd was 1.00E-10 M (0.1 nM).

These results indicate that the bindability of the antibody to the antigen is not lost when the peptide is conjugated.

### [Example 5-1]

### Molecular Interaction Test of HER-2 and Pertuzumab-peptide Conjugate by ELISA

The bindability of pertuzumab-peptide conjugates to the antigen HER-2 was measured by ELISA.

50 µg of HER-2 (R&D SYSTEMS, Cat. No. 10126-ER) was dissolved in PBS (Nacalai Tesque, Inc., Cat. No. 27575-31) to a final concentration of 1 g/L. To the dissolved HER-2, 5 equivalents of 1 mM NHS-PEG4-Biotin (Thermo Scientific, Cat. No. A39259) was added and incubated at 4°C overnight to biotinylate HER-2. To a column packed with 1 mL of Bio-Gel P-30 (Bio-Rad, Cat. No. 1504150), swollen with a solution of polyoxyethylene sorbitan monolaurate (Nacalai Tesque, Inc., Cat. No. 28353-85) added to PBS (Nacalai Tesque, Inc., Cat. No. 27575-31) to a final concentration of 0.1% (PBST), 100 uL of the above solution was added per one column to remove unreacted NHS-PEG4-Biotin.

Random biotinylated HER-2 was added to IMMOBILIZER STREPTAVIDIN F96 clear (NUNC, Cat. No. 436014) at 1 pmol per one well and incubated at 4°C overnight to solidify HER-2. The plate was washed with 900 µL of PBST in a plate washer (TECAN, HydroSpeed). 300 µL of 1% Block Ace (KAC Co., Ltd., Cat. No. UK-B80) was added per one well and incubated at room temperature for 90 minutes to perform blocking treatment. The plate was washed with 900 µL of PBST in the plate washer. 50 µL of pertuzumab-peptide conjugate and pertuzumab diluted to a final concentration of 100 nM by 50 mM HEPES pH 7.8-0.1% Block Ace were added and incubated at room temperature for 60 min. The plate was washed with 900 µL of PBST in the plate washer. 50 µL of Goat Anti-Human IgG H&L (HRP) (Abcam, Cat. No. ab97165) diluted 50,000 times with PBST-0.1% Block Ace solution was added and incubated at room temperature for 45 min. The plate was washed with 900 µL of PBST in the plate washer. 50 µL of TMB Solution (Sara care, Cat. No. 5150-0077) at room temperature was added and color development was performed at room temperature for 10 min in dark place, then 50 µL of TMB Stop Solution (Sara care, Cat. No. 5150-0021) was added to stop the color development. The absorbance at 450 nm was measured in a plate reader (Enspir (trademark), Perkin Elmer). This measurement was repeated twice and the average absorbance values are listed in TABLE 6.

This result indicates that the bindability of the antibody to the antigen is not lost when the peptide is conjugated.

### [Example 5-2]

### Molecular Interaction Test of EGFR and Cetuximab-peptide Conjugate by ELISA

The bindability of cetuximab-peptide conjugates to the antigen EGFR was measured by ELISA.

50 µg of EGFR (R&D SYSTEMS, Cat. No. AVI10493-50) was dissolved in PBS to a final concentration of 1 g/L.

EGFR was added to IMMOBILIZER STREPTAVIDIN F96 clear (NUNC, Cat. No. 436014) at 1 pmol per one well and incubated at 4°C overnight and solidification is performed. The plate was washed with 900 µL of PBST in a plate washer (TECAN, HydroSpeed). 300 µL of 1% Block Ace (KAC Co., Ltd. Cat. No. UK-B80) was added per one well and incubated at room temperature for 90 minutes to perform blocking treatment. The plate was washed with 900 µL of PBST in the plate washer. 50 µL of cetuximab-peptide conjugate and cetuximab diluted to a final concentration of 100 nM by 50 mM HEPES pH 7.8-0.1% Block Ace were added and incubated at room temperature for 60 min. The plate was washed with 900 µL of PBST in the plate washer. 50 µL of Goat Anti-Human IgG H&L (HRP) (Abcam, Cat. No. ab97165) diluted 50,000 times with PBST-0.1% Block Ace solution was added and incubated at room temperature for 45 min. The plate was washed with 900 µL of PBST in the plate washer. 50 µL of TMB Solution (Sara care, Cat. No. 5150-0077) at room temperature was added and color development was performed at room temperature for 10 min in dark place, then 50 µL of TMB Stop Solution (Sara care, Cat. No. 5150-0021) was added to stop the color development. The absorbance at 450 nm was measured in a plate reader (Enspir (trademark), Perkin Elmer). This measurement was repeated twice and the average absorbance values are listed in TABLE 6.

This result indicates that the bindability of the antibody to the antigen is not lost when the peptide is conjugated.

### [Example 5-3]

### Molecular Interaction Test of CTLA-4 and Ipilimumab-peptide Conjugate by ELISA

The bindability of ipilimumab-peptide conjugates to the antigen CTLA-4 was measured by ELISA.

100 µg of CTLA-4 (Abcam, Cat. No. ab167727) was dissolved in PBS (Nacalai Tesque, Inc., Cat. No. 27575-31) to a final concentration of 1 g/L. To the dissolved CTLA-4, 5 equivalents of 10 mM NHS-PEG4-Biotin (Thermo Scientific, Cat. No. A39259) was added and incubated at 4°C overnight to biotinylate CTLA-4. To a column packed with 1 mL of Bio-Gel P-6 (Bio-Rad, Cat. No. 1504130), swollen with a solution of polyoxyethylene sorbitan monolaurate (Nacalai Tesque, Inc., Cat. No. 28353-85) added to PBS to a final concentration of 0.1% (PBST), 100 uL of the above solution was added per one column to remove unreacted NHS-PEG4-Biotin.

Random biotinylated CTLA-4 was added to IMMOBILIZER STREPTAVIDIN F96 clear (NUNC, Cat. No. 436014) at 1 pmol per one well and incubated at 4°C overnight to perform solidification. The plate was washed with 900 µL of PBST in a plate washer (TECAN, HydroSpeed). 300 µL of 1% Block Ace (KAC Co., Ltd., Cat. No. UK-B80) was added per one well and incubated at room temperature for 90 minutes to perform blocking treatment. The plate was washed with 900 µL of PBST in the plate washer. 50 µL of ipilimumab-peptide conjugate and ipilimumab diluted to a final concentration of 100 nM by 50 mM HEPES pH 7.8-0.1% Block Ace were added and incubated at room temperature for 60 min. The plate was washed with 900 µL of PBST in the plate washer. 50 µL of Goat Anti-Human IgG H&L (HRP) (Abcam, Cat. No. ab97165) diluted 50,000 times with PBST-0.1% Block Ace solution was added and incubated at room temperature for 45 min. The plate was washed with 900 µL of PBST in the plate washer. 50 µL of TMB Solution (Sara care, Cat. No. 5150-0077) at room temperature was added and color development was performed at room temperature for 10 min in dark place, then 50 µL of TMB Stop Solution (Sara care, Cat. No. 5150-0021) was added to stop the color development. The absorbance at 450 nm was measured in a plate reader (Enspir (trademark), Perkin Elmer). This measurement was repeated twice and the average absorbance values are listed in TABLE 6.

This result indicates that the bindability of the antibody to the antigen is not lost when the peptide is conjugated.

### [Example 5-4]

### Molecular Interaction Test of PD-L1 and Atezolizumab-peptide Conjugate by ELISA

The bindability of atezolizumab-peptide conjugates to the antigen PD-L1 was measured by ELISA.

100 µg of PD-L1 (R&D SYSTEMS, Cat. No. 9049-B7) was dissolved in PBS (Nacalai Tesque, Inc., Cat. No. 27575-31) to a final concentration of 1 g/L. To the dissolved PD-L1, 5 equivalents of 10 mM NHS-PEG4-Biotin (Thermo Scientific, Cat. No. A39259) was added and incubated at 4°C overnight to biotinylate PD-L1. To a column packed with 1 mL of Bio-Gel P-6 (Bio-Rad, Cat. No. 1504130), swollen with a solution of polyoxyethylene sorbitan monolaurate (Nacalai Tesque, Inc., Cat. No. 28353-85) added to PBS to a final concentration of 0.1% (PBST), 100 uL of the above solution was added per one column to remove unreacted NHS-PEG4-Biotin.

Random biotinylated PD-L1 was added to IMMOBILIZER STREPTAVIDIN F96 clear (NUNC, Cat. No. 436014) at 1 pmol per one well and incubated at 4°C overnight to perform solidification. The plate was washed with 900 µL of PBST in a plate washer (TECAN, HydroSpeed). 300 µL of 1% Block Ace (KAC Co., Ltd., Cat. No. UK-B80) was added per one well and incubated at room temperature for 90 minutes to perform blocking treatment. The plate was washed with 900 µL of PBST in the plate washer. 50 µL of atezolizumab-peptide conjugate and atezolizumab diluted to a final concentration of 100 nM by 50 mM HEPES pH 7.8-0.1% Block Ace were added and incubated at room temperature for 60 min. The plate was washed with 900 µL of PBST in the plate washer. 50 µL of Goat Anti-Human IgG H&L (HRP) (Abcam, Cat. No. ab97165) diluted 50,000 times with PBST-0.1% Block Ace solution was added and incubated at room temperature for 45 min. The plate was washed with 900 µL of PBST in the plate washer. 50 µL of TMB Solution (Sara care, Cat. No. 5150-0077) at room temperature was added and color development was performed at room temperature for 10 min in dark place, then 50 µL of TMB Stop Solution (Sara care, Cat. No. 5150-0021) was added to stop the color development. The absorbance at 450 nm was measured in a plate reader (Enspir (trademark), Perkin Elmer). This measurement was repeated twice and the average absorbance values are listed in TABLE 6.

This result indicates that the bindability of the antibody to the antigen is not lost when the peptide is conjugated.

### [Example 5-5]

### Molecular Interaction Test of PD-1 and Pembrolizumab-peptide Conjugate by ELISA

The bindability of pembrolizumab-peptide conjugates to the antigen PD-1 was measured by ELISA.

100 µg of PD-1 (R&D SYSTEMS, Cat. No. 8986-PD-100) was dissolved in PBS (Nacalai Tesque, Inc., Cat. No. 27575-31) to a final concentration of 1 g/L. To the dissolved PD-1, 5 equivalents of 10 mM NHS-PEG4-Biotin (Thermo Scientific, Cat. No. A39259) was added and incubated at 4°C overnight to biotinylate PD-1. To a column packed with 1 mL of Bio-Gel P-6 (Bio-Rad, Cat. No. 1504130), swollen with a solution of polyoxyethylene sorbitan monolaurate (Nacalai Tesque, Inc., Cat. No. 28353-85) added to PBS to a final concentration of 0.1% (PBST), 100 uL of the above solution was added per one column to remove unreacted NHS-PEG4-Biotin.

Random biotinylated PD-1 was added to IMMOBILIZER STREPTAVIDIN F96 clear (NUNC, Cat. No. 436014) at 1 pmol per one well and incubated at 4°C overnight to perform solidification. The plate was washed with 900 µL of PBST in a plate washer (TECAN, HydroSpeed). 300 µL of 1% Block Ace (KAC Co., Ltd., Cat. No. UK-B80) was added per one well and incubated at room temperature for 90 minutes to perform blocking treatment. The plate was washed with 900 µL of PBST in the plate washer. 50 µL of pembrolizumab-peptide conjugate and pembrolizumab diluted to a final concentration of 100 nM by 50 mM HEPES pH 7.8-0.1% Block Ace were added and incubated at room temperature for 60 min. The plate was washed with 900 µL of PBST in the plate washer. 50 µL of Goat Anti-Human IgG H&L (HRP) (Abcam, Cat. No. ab97165) diluted 50,000 times with PBST-0.1% Block Ace solution was added and incubated at room temperature for 45 min. The plate was washed with 900 µL of PBST in the plate washer. 50 µL of TMB Solution (Sara care, Cat. No. 5150-0077) at room temperature was added and color development was performed at room temperature for 10 min in dark place, then 50 µL of TMB Stop Solution (Sara care, Cat. No. 5150-0021) was added to stop the color development. The absorbance at 450 nm was measured in a plate reader (Enspir (trademark), Perkin Elmer). This measurement was repeated twice and the average absorbance values are listed in TABLE 6.

This result indicates that the bindability of the antibody to the antigen is not lost when the peptide is conjugated.

### [Example 5-6]

### Molecular Interaction Test of HER-2 and Trastuzumab-peptide Conjugate by ELISA

The bindability of trastuzumab-peptide conjugates to the antigen HER-2 was measured by ELISA.

50 µg of HER-2 (R&D SYSTEMS, Cat. No. 10126-ER) was dissolved in PBS (Nacalai Tesque, Inc., Cat. No. 27575-31) to a final concentration of 1 g/L. To the dissolved HER-2, 5 equivalents of 1 mM NHS-PEG4-Biotin (Thermo Scientific, Cat. No. A39259) was added and incubated at 4°C overnight to biotinylate HER-2. To a column packed with 1 mL of Bio-Gel P-30 (Bio-Rad, Cat. No. 1504150), swollen with a solution of polyoxyethylene sorbitan monolaurate (Nacalai Tesque, Inc., Cat. No. 28353-85) added to PBS (Nacalai Tesque, Inc., Cat. No. 27575-31) to a final concentration of 0.1% (PBST), 100 uL of the above solution was added per one column to remove unreacted NHS-PEG4-Biotin.

Random biotinylated HER-2 was added to IMMOBILIZER STREPTAVIDIN F96 clear (NUNC, Cat. No. 436014) at 1 pmol per one well and incubated at 4°C overnight to perform solidification of HER-2. The plate was washed with 900 µL of PBST in a plate washer (TECAN, HydroSpeed). 300 µL of 1% Block Ace (KAC Co., Ltd., Cat. No. UK-B80) was added per one well and incubated at room temperature for 90 minutes to perform blocking treatment. The plate was washed with 900 µL of PBST in the plate washer. 50 µL of trastuzumab-peptide conjugate and trastuzumab diluted to a final concentration of 100 nM by 50 mM HEPES pH 7.8-0.1% Block Ace were added and incubated at room temperature for 60 min. The plate was washed with 900 µL of PBST in the plate washer. 50 µL of Goat Anti-Human IgG H&L (HRP) (Abcam, Cat. No. ab97165) diluted 50,000 times with PBST-0.1% Block Ace solution was added and incubated at room temperature for 45 min. The plate was washed with 900 µL of PBST in the plate washer. 50 µL of TMB Solution (Sara care, Cat. No. 5150-0077) at room temperature was added and color development was performed at room temperature for 10 min in dark place, then 50 µL of TMB Stop Solution (Sara care, Cat. No. 5150-0021) was added to stop the color development. The absorbance at 450 nm was measured in a plate reader (Enspir (trademark), Perkin Elmer). This measurement was repeated twice and the average absorbance values are listed in TABLE 6.

This result indicates that the bindability of the antibody to the antigen is not lost when the peptide is conjugated.

### [Example 5-7]

### Molecular Interaction Test of PD-1 and Nivolumab-peptide Conjugate by ELISA

The bindability of nivolumab-peptide conjugates to the antigen PD-1 was measured by ELISA.

100 µg of PD-1 (R&D SYSTEMS, Cat. No. 8986-PD-100) was dissolved in PBS (Nacalai Tesque, Inc., Cat. No. 27575-31) to a final concentration of 1 g/L. To the dissolved PD-1, 5 equivalents of 10 mM NHS-PEG4-Biotin (Thermo Scientific, Cat. No. A39259) was added and incubated at 4°C overnight to biotinylate PD-1. To a column packed with 1 mL of Bio-Gel P-6 (Bio-Rad, Cat. No. 1504130), swollen with a solution of polyoxyethylene sorbitan monolaurate (Nacalai Tesque, Inc., Cat. No. 28353-85) added to PBS to a final concentration of 0.1% (PBST), 100 uL of the above solution was added per one column to remove unreacted NHS-PEG4-Biotin.

Random biotinylated PD-1 was added to IMMOBILIZER STREPTAVIDIN F96 clear (NUNC, Cat. No. 436014) at 1 pmol per one well and incubated at 4°C overnight to perform solidification. The plate was washed with 900 µL of PBST in a plate washer (TECAN, HydroSpeed). 300 µL of 1% Block Ace (KAC Co., Ltd., Cat. No. UK-B80) was added per one well and incubated at room temperature for 90 minutes to perform blocking treatment. The plate was washed with 900 µL of PBST in the plate washer. 50 µL of nivolumab-peptide conjugate and nivolumab diluted to a final concentration of 100 nM by 50 mM HEPES pH 7.8-0.1% Block Ace were added and incubated at room temperature for 60 min. The plate was washed with 900 µL of PBST in the plate washer. 50 µL of Goat Anti-Human IgG H&L (HRP) (Abcam, Cat. No. ab97165) diluted 50,000 times with PBST-0.1% Block Ace solution was added and incubated at room temperature for 45 min. The plate was washed with 900 µL of PBST in the plate washer. 50 µL of TMB Solution (Sara care, Cat. No. 5150-0077) at room temperature was added and color development was performed at room temperature for 10 min in dark place, then 50 µL of TMB Stop Solution (Sara care, Cat. No. 5150-0021) was added to stop the color development. The absorbance at 450 nm was measured in a plate reader (Enspir (trademark), Perkin Elmer). This measurement was repeated twice and the average absorbance values are listed in TABLE 6.

This result indicates that the bindability of the antibody to the antigen is not lost when the peptide is conjugated.

### [Example 5-8]

### Molecular Interaction Test of TfR and Various Antibody-peptide Conjugate by ELISA

The bindability of various antigen-peptide conjugates to the TfR was measured by ELISA.

10 equivalents of 1 mM NHS-PEG4-Biotin (Thermo Scientific, Cat. No. A39259) was added to 100 µg of human TfR and incubated at 4°C overnight to biotinylate. For human TfR, recombinant hTfR described in Example 2 of WO2018/124121 was used. To a column packed with 1 mL of Bio-Gel P-6 (Bio-Rad, Cat. No. 1504130), swollen with a solution of polyoxyethylene sorbitan monolaurate (Nacalai Tesque, Inc., Cat. No. 28353-85) added to PBS to a final concentration of 0.1% (PBST), 100 uL of the above solution was added per one column to remove unreacted NHS-PEG4-Biotin.

Random biotinylated TfR was added to IMMOBILIZER STREPTAVIDIN F96 clear (NUNC, Cat. No. 436014) at 1 pmol per one well and incubated at 4°C overnight to perform solidification. The plate was washed with 900 µL of PBST in a plate washer (TECAN, HydroSpeed). 300 µL of 1% Block Ace (KAC Co., Ltd., Cat. No. UK-B80) was added per one well and incubated at room temperature for 90 minutes to perform blocking treatment. The plate was washed with 900 µL of PBST in the plate washer. 50 µL of various antibody-peptide conjugate and antibody whose peptide is not modified diluted to a final concentration of 100 nM by 50 mM HEPES pH 7.8-0.1% Block Ace were added and incubated at room temperature for 60 min. The plate was washed with 900 µL of PBST in the plate washer. 50 µL of Goat Anti-Human IgG H&L (HRP) (Abcam, Cat. No. ab97165) diluted 50,000 times with PBST-0.1% Block Ace solution was added and incubated at room temperature for 45 min. The plate was washed with 900 µL of PBST in the plate washer. 50 µL of TMB Solution (Sara care, Cat. No. 5150-0077) at room temperature was added and color development was performed at room temperature for 10 min in dark place, then 50 µL of TMB Stop Solution (Sara care, Cat. No. 5150-0021) was added to stop the color development. The absorbance at 450 nm was measured in a plate reader (Enspir (trademark), Perkin Elmer). This measurement was repeated twice and the average absorbance values are listed in TABLE 6.

This result indicates that the bindability of the TfR binding peptide to the TfR is not lost when the antibody is conjugated.

**[TABLE 6]**

| [TABLE 6-1] | | | |
|---|---|---|---|
| **Sample** | | **Target** | Abs450nm at 100nM |
| Pertuzumab | **Unmodified** | HER2 | 2.0965 |
| | | hTfR | 0.046 |
| | | No-targt | 0.0455 |
| | 894_3m_PEG12_K(Mal) | HER2 | 0.831 |
| | | hTfR | 0.863 |
| | | No-targt | 0.168 |
| Cetuximab | **Unmodified** | EGFR | 2.1625 |
| | | hTfR | 0.0445 |
| | | No-targt | 0.0465 |
| | 894_3m_PEG12_K(Mal) | EGFR | 1.419 |
| | | hTfR | 0.59 |
| | | No-targt | 0.282 |
| Ipilimumab | **Unmodified** | CTLA4 | 1.306 |
| | | hTfR | 0.0435 |
| | | No-targt | 0.044 |
| | 894_3m_PEG12_K(Mal) | CTLA4 | 0.711 |
| | | hTfR | 0.6055 |
| | | No-targt | 0.161 |
| Atezolizumab | **Unmodified** | PD-L1 | 2.237 |
| | | hTfR | 0.0465 |
| | | No-targt | 0.043 |
| | 894_3m_PEG12_K(Mal) | PD-L1 | 1.083 |
| | | hTfR | 0.4715 |
| | | No-targt | 0.3855 |

**[TABLE 6-2]**

| **Sample** | | Target | Abs450nm at 100nM |
|---|---|---|---|
| Pembrolizumab | **Unmodified** | PD-1 | 1.6405 |
| | | hTfR | 0.041 |
| | | No-targt | 0.0415 |
| | 894_3m_PEG12_K(Mal) | PD-1 | 0.916 |
| | | hTfR | 0.6765 |
| | | No-targt | 0.231 |
| Nivolumab | **Unmodified** | PD-1 | 1.599 |
| | | hTfR | 0.049 |
| | | No-targt | 0.049 |
| | 894_3m_PEG12_K(Mal) | PD-1 | 0.8975 |
| | | hTfR | 0.6465 |
| | | No-targt | 0.1365 |
| Trastuzumab | **Unmodified** | HER2 | 2.0715 |
| | | hTfR | 0.0445 |
| | | No-targt | 0.0425 |
| | 894_3m_PEG12_K(Mal) | HER2 | 0.611 |
| | | hTfR | 0.711 |
| | | No-targt | 0.132 |

### [Example 6]

### Cell Permeability Test

The cell permeability of the antibody-peptide conjugates obtained in Examples 1 through 3 was confirmed.

### [Cell Culture]

RPMI-1640 medium containing 10% FBS and 2 mmol/L L-Glutamine was used to culture human breast cancer cells BT-549. Culture was performed under 37°C, 5% CO₂ condition.

### [Seeding of Cell]

Collagen Type I (Corning Incorporated) was diluted with 20 mmol/L acetic acid to 50 µg/mL. One sterile cover glass was placed in each well of a 24-well plate, and after adding diluted Collagen Type I solution, the plate was kept at 37°C for 1 hour. Collagen Type I solution was removed and washed three times with PBS. 1×10⁵ human breast cancer cells BT-549 per well were seeded and cultured at 37°C, 5% CO₂ condition overnight.

### [Preparation of Antibody-peptide Conjugate Solution and Addition to Cell]

Dilution medium (RPMI 1640 medium containing 0.5% bovine serum albumin and 20 µg/mL human transferrin holotype) was used to dilute the antibody-peptide conjugates. The antibody-peptide conjugates were diluted in dilution medium to 2.5, 5, 10 µg/mL or 1, 3, 9 µg/mL.

After confirming that cells BT-549 cultured overnight in 24-well plates had adhered to the cover glass, they were washed twice with RPMI 1640 medium. 500 pL/well of RPMI 1640 medium containing 0.5% bovine serum albumin was added and leaved on ice for 15 min and removed, the 500 pL/well of diluted antibody-peptide conjugate solution was added and leaved under 37°C, 5% CO₂ condition for 3 hours. For negative control, only antibody (nivolumab or trastuzumab) was added instead of antibody-peptide conjugate.

### [Fixation and Permeabilization of Cell]

The antibody-peptide conjugate solution was removed from the 24-well plate, and the cells BT-549 were washed three times with PBS. 500 pL/well of 4% paraformaldehydephosphate buffer (FUJIFILM Wako Pure Chemical Corporation) was added and leaved at room temperature for 15 min, then the cells were washed three times with PBS.

500 pL/well of 0.1% Triton X-100 solution was added, leaved at room temperature for 10 min, and then washed three times with PBS.

### [Immunostaining]

PBS containing 10% donkey serum and 1% bovine serum albumin was added at 250 pL/well, leaved at room temperature for 1 hour, and then washed three times with PBS.

Goat anti-human IgG h+l (Betil Laboratories, Inc.) diluted to 5 µg/mL in PBS-T containing 0.5% bovine serum albumin was used as the primary antibody solution. 200 pL/well of primary antibody solution was added, leaved at room temperature for 1 hour, and then washed three times with PBS.

Donkey anti-goat IgG h+l, DyLight 488 conjugated (Betil Laboratories, Inc.), diluted 300 times in PBS-T containing 0.5% bovine serum albumin was used as the secondary antibody solution. Secondary antibody solution was added 200 pL/well, leaved at room temperature for 1 hour and light-shielded, and then washed three times with PBS.

### [Nuclear Staining and Encapsulation]

Hoechst 33342 (Thermo Fisher Scientific Inc.) diluted to 2 µg/mL with PBS was added at 500 pL/well, leave at room temperature under light-shielded conditions for 10 min, and then washed three times with PBS. The cover glass was removed from the 24-well plate, sealed on a glass slide using Fluorescent Mounting Medium (Agilent Technologies Inc.), and leaved at room temperature under light-shielded condition overnight. An inverted fluorescence microscope DMI6000B (Leica Microsystems) was used for observation at the wavelengths for DyLight 488 and DAPI detection. Results are shown in FIG. 1.

FIG. 1-1 shows the results of adding trastuzumab-894_PEG12_(NHS) conjugate (Conjugate No. 63) in the amounts indicated in the figure. FIG. 1-1 shows that trastuzumab-894_PEG12_(NHS) conjugate has the ability to migrate into cells.

FIG. 1-2 shows the results of adding trastuzumab-hTfR_000894_PEG11_(Hydradine) (Conjugate No. 60) in the amounts indicated in the figure. FIG. 1-2 shows that the conjugate of hTfR_000894_PEG11_(Hydradine) with trastuzumab has the ability to migrate into cells.

FIG. 1-3 show the results of adding 5 µg/mL of trastuzumab-hTfR_000894_PEG11_K (Maleimide) (Conjugate No. 1); a, and trastuzumab-hTfR_000894_PEG36_K (Maleimide) (Conjugate No. 2); b, at 5 µg/mL. Fig. 1-3 show that the conjugate of hTfR_000894_PEG (11/36)_(Hydradine) with trastuzumab has the ability to migrate into cells.

### [Example 7]

### Brain Migration Evaluation Test

Brain migration of the antibody-peptide conjugates obtained in Examples 1 through 3 was confirmed in knock-in mice (KI mice).

### [Example 7-1]

### Evaluation of Brain Migration of Trastuzumab-peptide Conjugate Using hTfR-KI Mouse

### [Preparation of Administering Solution]

Administering solution of trastuzumab: Trastuzumab was diluted in saline to 1 mg/mL.

Administering solution of trastuzumab-hTfR_000894_PEG11_K (Maleimide): Trastuzumab-hTfR_000894_PEG11_K (Maleimide) synthesized in Example 3-5 (Conjugate No. 1) was diluted in saline to 1 mg/mL.

Administering solution of trastuzumab-hTfR_000894_PEG36_K (Maleimide): Trastuzumab-hTfR_000894_PEG36_K (Maleimide) synthesized as in Example 3-5 (Conjugate No. 2) was diluted in saline to 1 mg/mL.

### [Administration to hTfR-KI Mouse and Tissue Collection]

Next, evaluation test of brain migration in hTfR-KI mouse was performed. hTfR-KI mice (male, 13 to 15 weeks old) expressing human TfR were rapidly administered an administering solution of trastuzumab, trastuzumab-hTfR_000894_PEG11_K (Maleimide) or trastuzumab-hTfR_000894_PEG36_K (Maleimide) into the tail vein at a dose of 5 mg/kg (6 mice/group). Blood was drawn from the tail vein after 1 and 3 hours from administration (2 mice/time point). After 6 and 24 hours from administration, mice were anesthetized with isoflurane, blood was drawn from the right ventricle, and then perfused with saline from the left ventricle for 4 to 5 min for blood removing treatment. Blood was collected using an EDTA-2K-treated needle and syringe, and the collected blood was kept on ice until it was separated into plasma. After blood removing treatment, brains were harvested and divided into left and right portions, and the left brain was embedded in a freeze-embedding agent (OCT compound, Sakura Finetek Co., Ltd.) for immunostaining and a frozen block was prepared. The right brain, heart, lung, liver, spleen, kidney, and quadriceps muscle were collected for measurement of test substance concentrations in tissues, weighed, flash-frozen in liquid nitrogen, and stored at -70°C or less.

### [Preparation of Tissue Extract]

Protease Inhibitor Cocktail (P8340, Sigma-Aldrich Co., LLC.) was added to RIPA Buffer (FUJIFILM Wako Pure Chemical Corporation) to make a final concentration of 1% and mixed to make Lysis Buffer. Metal beads, tissue, and 20 times the volume of Lysis Buffer of the tissue weight were added to the tube, and the tissue was crushed using a bead crusher (Taitec Corporation) to make tissue crush solution. The tissue crush solution was centrifuged (11,000 × g, 4°C, 5 min), and the supernatant was collected as tissue extract.

### [Preparation of Test Solution]

Capture antibody solution: Anti-Human Kappa Light Chain Goat IgG Biotin (100 µg/mL, Immuno-Biological Laboratories Co.,Ltd.) was diluted to 1 µg/mL using Blocker Casein in PBS.

SULFO-labeled antibody solution: SULFO-TAG Anti-Human Antibody (Goat) (500 µg/mL, Meso Scale Diagnostics, LLC) was diluted to 1 µg using Blocker Casein in PBS.

2×Read Buffer T: An appropriate amount of MSD Read Buffer T (4×) (Meso Scale Diagnostics, LLC) was diluted with an equal volume of water for injection.

### [Preparation of Calibration Curve Sample]

Trastuzumab, trastuzumab-hTfR_000894_PEG11_K (Maleimide) or trastuzumab-hTfR_000894_PEG36_K (Maleimide) was diluted with blank plasma or tissue extract (from plasma or tissue extract from untreated individuals) to prepare calibration samples at concentrations from 0.195 to 200 ng/mL.

### [Preparation of Measurement Sample]

Each tissue extract was diluted with a blank tissue extract as appropriate and used as the measurement sample.

### [Measurement of Test Substance Concentration in Plasma and Tissue]

To Strept Avidin plate (Meso Scale Diagnostics, LLC), 150 pL/well of Blocker Casein in PBS (Thermo Fisher Scientific, Inc.) was added and shaken (26°C, 500 rpm) for 60 min in a plate shaker (Biosan) to perform blocking treatment. The Blocker Casein in PBS was removed from the Strept Avidin plates, and 200 pL/well of PBST was added to each plate and washed. 25 pL/well of Capture antibody solution was added to each Strept Avidin plate, and the plates were shaken (26°C, 500 rpm) for 60 min in a plate shaker. Capture antibody solution was removed and PBST was added 200 pL/well and washed. This washing operation was performed three times. Calibration curve samples and measurement samples were added to the Strept Avidin plate at 25 pL/well and shaken (26°C, 500 rpm) for 60 min in a plate shaker. Calibration curve samples and measurement samples were removed, and PBST was added 200 µL/well and washed. This washing operation was performed three times. 25 µL/well of SULFO-labeled antibody solution was added to each Strept Avidin plate, and the plates were shaken (26°C, 500 rpm) for 60 min in a plate shaker. SULFO-labeled antibody solution was removed, and PBST was added 200 µL/well and washed. This washing operation was performed three times. The concentration of each test substance in the samples were calculated by adding 150 µL/well of 2×Read Buffer T to each Strept Avidin plate and measuring with a plate reader (Sector S600, Meso Scale Diagnostics, LLC).

### [Result of Measurement of Test Substance Concentration in Plasma]

The results of the trastuzumab-peptide conjugate concentration measurements in plasma are shown in FIG 2-1. Trastuzumab, trastuzumab-hTfR_000894_PEG11_K (Maleimide) and trastuzumab-hTfR_000894_PEG36_K (Maleimide) were detected in plasma at all time points evaluated and showed biphasic changes in plasma concentrations, with relatively fast decay up to 6 hours post-administration and slow decay after 6 hours post-administration. The trastuzumab-peptide conjugates, trastuzumab-hTfR_000894_PEG11_K (Maleimide) and trastuzumab-hTfR_000894_PEG36_K (Maleimide), indicated lower value than trastuzumab at all time points.

FIG. 2-1 shows an alternative graph to the drawing showing concentrations of trastuzumab-hTfR_000894_PEG11_K (Maleimide) conjugate (Conjugate No. 1) and trastuzumab-hTfR_000894_PEG36_K (Maleimide) conjugate (Conjugate No. 2) in plasma.

### [Result of Measurement of Test Substance Concentration in Tissue]

The results of the trastuzumab-peptide conjugate concentration measurements in tissues are shown in FIG 2-2. Trastuzumab, trastuzumab-hTfR_000894_PEG11_K (Maleimide) and trastuzumab-hTfR_000894_PEG36_K (Maleimide) were detected in all tissues. Compared to the concentration of trastuzumab in brain tissue, the concentrations of trastuzumab-hTfR_000894_PEG11_K (Maleimide) and trastuzumab-hTfR_000894_PEG36_K (Maleimide) indicated higher value. This indicated that the trastuzumab-peptide conjugate is brain migratory.

FIG. 2-2 shows an alternative graph to the drawing showing concentrations of trastuzumab-hTfR_000894_PEG11_K (Maleimide) conjugate (Conjugate No. 1) and trastuzumab-hTfR_000894_PEG36_K (Maleimide) conjugate (Conjugate No. 2) in tissue.

### [Example 7-2]

### Evaluation of Brain Migration of Nivolumab-peptide Conjugate Using hTfR-KI Mouse

### [Preparation of Administering Solution]

Administering solution of nivolumab: Nivolumab was diluted in saline to 1 mg/mL.

Administering solution of nivolumab-894_3m_G4S2_K (Mal): Nivolumab-894_3m_G4S2_K (Mal) synthesized in Example 3-3 (Conjugate No. 11) was diluted in saline to 1 mg/mL.

### [Administration to hTfR-KI Mouse and Tissue Collection]

Next, evaluation test of brain migration in hTfR-KI mouse was performed. hTfR-KI mice (male, 15 weeks old) expressing human TfR were rapidly administered an administering solution of nivolumab or nivolumab-894_3m_G4S2_K (Mal) into the tail vein at a dose of 5 mg/kg (6 mice/group). Blood was drawn from the tail vein after 1 and 3 hours from administration (2 mice/time point). After 6 and 24 hours from administration, mice were anesthetized with isoflurane, blood was drawn from the right ventricle, and then perfused with saline from the left ventricle for 4 to 5 min for blood removing treatment. Blood was collected using an EDTA-2K-treated needle and syringe, and the collected blood was kept on ice until it was separated into plasma. After blood removing treatment, brains were harvested and divided into left and right portions, and the left brain was embedded in a freeze-embedding agent (OCT compound, Sakura Finetek Co., Ltd.) for immunostaining and a frozen block was prepared. The right brain, heart, lung, liver, spleen, kidney, and quadriceps muscle were collected for measurement of test substance concentrations in tissues, weighed, flash-frozen in liquid nitrogen, and stored at -70°C or less.

### [Preparation of Tissue Extract]

Protease Inhibitor Cocktail (P8340, Sigma-Aldrich Co., LLC.) was added to RIPA Buffer (FUJIFILM Wako Pure Chemical Corporation) to make a final concentration of 1% and mixed to make Lysis Buffer. Metal beads, tissue, and 20 times the volume of Lysis Buffer of the tissue weight were added to the tube, and the tissue was crushed using a bead crusher (Taitec Corporation) to make tissue crush solution. The tissue crush solution was centrifuged (11,000 × g, 4°C, 5 min), and the supernatant was collected as tissue extract.

### [Preparation of Test Solution]

Capture antibody solution: Anti-Human Kappa Light Chain Goat IgG Biotin (100 µg/mL, Immuno-Biological Laboratories Co.,Ltd.) was diluted to 1 µg/mL using Blocker Casein in PBS.

SULFO-labeled antibody solution: SULFO-TAG Anti-Human Antibody (Goat) (500 µg/mL, Meso Scale Diagnostics, LLC) was diluted to 1 µg/mL using Blocker Casein in PBS.

2×Read Buffer T: An appropriate amount of MSD Read Buffer T (4×) (Meso Scale Diagnostics, LLC) was diluted with an equal volume of water for injection.

### [Preparation of Calibration Curve Sample]

As a control, nivolumab or nivolumab-894_3m_G4S2_K (Mal) (Conjugate No. 11) was diluted with blank plasma or tissue extract (from plasma or tissue extract from untreated individuals) to prepare calibration samples at concentrations from 0.195 to 200 ng/mL.

### [Preparation of Measurement Sample]

Each tissue extract was diluted with a blank tissue extract as appropriate and used as the measurement sample.

### [Measurement of Test Substance Concentration in Plasma and Tissue]

To Strept Avidin plate (Meso Scale Diagnostics, LLC), 150 pL/well of Blocker Casein in PBS (Thermo Fisher Scientific, Inc.) was added and shaken (26°C, 500 rpm) for 60 min in a plate shaker (Biosan) to perform blocking treatment. The Blocker Casein in PBS was removed from the Strept Avidin plates, and 200 pL/well of PBST was added to each plate and washed. 25 pL/well of Capture antibody solution was added to each Strept Avidin plate, and the plates were shaken (26°C, 500 rpm) for 60 min in a plate shaker. Capture antibody solution was removed and PBST was added 200 pL/well and washed. This washing operation was performed three times. Calibration curve samples and measurement samples were added to the Strept Avidin plate at 25 pL/well and shaken (26°C, 500 rpm) for 60 min in a plate shaker. Calibration curve samples and measurement samples were removed, and PBST was added 200 pL/well and washed. This washing operation was performed three times. 25 pL/well of SULFO-labeled antibody solution was added to each Strept Avidin plate, and the plates were shaken (26°C, 500 rpm) for 60 min in a plate shaker. SULFO-labeled antibody solution was removed, and PBST was added 200 pL/well and washed. This washing operation was performed three times. The concentration of each test substance in the samples were calculated by adding 150 pL/well of 2×Read Buffer T to each Strept Avidin plate and measuring with a plate reader (Sector S600, Meso Scale Diagnostics, LLC).

### [Result of Measurement of Test Substance Concentration in Plasma]

The results of the nivolumab-peptide conjugate concentration measurements in plasma are shown in FIG 2-3. Nivolumab and nivolumab-894_3m_G4S2_K (Mal) were detected in plasma at all time points evaluated and showed biphasic changes in plasma concentrations, with relatively fast decay up to 6 hours post-administration and slow decay after 6 hours post-administration. The nivolumab-peptide conjugate, nivolumab-894_3m_G4S2_K (Mal) indicated lower value than nivolumab at all time points.

FIG. 2-3 shows an alternative graph to the drawing showing concentration of nivolumab-894_3m_G4S2_K(Mal) conjugate (Conjugate No. 11) in plasma.

### [Result of Measurement of Test Substance Concentration in Tissue]

The results of the nivolumab-peptide conjugate concentration measurements in tissues are shown in FIG 2-4. Nivolumab and nivolumab-894_3m_G4S2_K(Mal) conjugate were detected in all tissues. Compared to the concentration of nivolumab in brain tissue, the concentration of nivolumab-894_3m_G4S2_K(Mal) conjugate indicated higher value. This indicated that the nivolumab-peptide conjugate is brain migratory.

FIG. 2-4 shows an alternative graph to the drawing showing concentration of nivolumab-894_3m_G4S2_K(Mal) conjugate (Conjugate No. 11) in tissue.

### [Example 7-3]

### [Immunostaining of Trastuzumab in Tissue]

Using a cryostat (Leica Microsystems), 7 µm-thick sections were prepared from the brain freeze block of each individual prepared in Example 7-1, and attached to anti-exfoliation treated glass slides (Matsunami Glass Ind.,Ltd.). After air-drying, tissue sections were fixed by immersion in 4% paraformaldehyde (FUJIFILM Wako Pure Chemical Corporation) for 5 min. After washing with PBST (3 min, 3 times), sections were immersed in 0.3% H₂O₂/MeOH for 20 min for blocking of endogenous peroxidase. After washing of the sections with PBST (3 min, 3 times), SuperBlock Blocking Buffer in PBS was added dropwise to the sections for blocking treatment at room temperature for 100 min. After washing of the sections with PBST (3 min, 3 times), HRP-labelled anti-human IgG antibody (A80-219P, Bethyl Laboratories, Inc.) moderately diluted with CanGet Signal Immunostain solution A (Toyobo Co., Ltd.) was added dropwise, and treated at room temperature for 120 minutes. After washing of the sections with PBST (3 min, 3 times), sensitization was performed with TSA-Plus Fluorescein System (PerkinElmer, Inc.) according to the attached document. After washing of the sections with PBST (3 min, 3 times), Anti-Fluorescein-HRP (Dako) was added dropwise to the sections and treated at room temperature for 15 min. After washing of the sections with PBST (3 min, 3 times), DAB substrate solution (Dako), prepared according to the attached document, was added dropwise to the sections and treated until moderate color development. After washing in running water, counterstaining was performed with Mayer's Hematoxylin stain solution (FUJIFILM Wako Pure Chemical Corporation). After washing in running water, dehydratrion and permeabilization were performed with ethanol ascending series and xylene, Eukitt (ORSAtec GmbH aka Kindler GmbH) was dropped onto the sections as an inclusion agent, and tissue specimens were prepared by covering with a cover glass.

### [Immunostaining of Nivolumab in Tissue]

Using a cryostat (Leica Microsystems), 7 µm-thick sections were prepared from the brain freeze block of each individual prepared in Example 7-2, and attached to anti-exfoliation treated glass slides (Matsunami Glass Ind.,Ltd.). After air-drying, tissue sections were fixed by immersion in 4% paraformaldehyde (FUJIFILM Wako Pure Chemical Corporation) for 5 min. After washing with PBST (3 min, 3 times), sections were immersed in 0.3% H₂O₂/MeOH for 20 min for blocking of endogenous peroxidase. After washing of the sections with PBST (3 min, 3 times), SuperBlock Blocking Buffer in PBS was added dropwise to the sections for blocking treatment at room temperature for 100 min. After washing of the sections with PBST (3 min, 3 times), in the case of 894_3m_G4S2_K(Mal)-nivolumab conjugate (Conjugate No. 11), anti-human IgG antibody moderately diluted in CanGet Signal Immunostain solution A (Toyobo Co., Ltd.) was dropped, and in the case of 894_3m_GGRGRS_K (Mal)-nivolumab (Conjugate No. 43), anti-nivolumab antibody (A01931-40, Genscript Biotech Corporation) moderately diluted in CanGet Signal Immunostain solution A (Toyobo Co., Ltd.) was dropped, and leaved at 4°C overnight. After washing of the sections with PBST (3 min, 3 times), CSAII Rabbit Link (Dako) diluted 20 times with CanGet Signal Immunostain solution A was dropped onto the sections and treated at room temperature for 30 min. After washing of the sections with PBST (3 min, 3 times), sensitization was performed with TSA-Plus Fluorescein System (PerkinElmer, Inc.) according to the attached document. After washing of the sections with PBST (3 min, 3 times), Anti-Fluorescein-HRP (Dako) was added dropwise to the sections and treated at room temperature for 15 min. After washing of the sections with PBST (3 min, 3 times), DAB substrate solution (Dako), prepared according to the attached document, was added dropwise to the sections and treated until moderate color development. After washing the sections in running water, counterstaining was performed with Mayer's Hematoxylin stain solution (FUJIFILM Wako Pure Chemical Corporation). After washing the sections in running water, dehydratrion and permeabilization were performed with ethanol ascending series and xylene, Eukitt (ORSAtec GmbH aka Kindler GmbH) was dropped onto the sections as an inclusion agent, and tissue specimens were prepared by covering with a cover glass.

### [Immunostaining Result]

Result 1: The immunostaining results in the cerebellum 6 hours after treatment with trastuzumab-hTfR_000894_PEG11_K (Maleimide) (Conjugate No. 1) and trastuzumab-hTfR_000894_PEG36_K (Maleimide) (Conjugate No. 2) are shown in FIG. 3-1. The lower figure is an enlargement of rectangle part in the upper figure. These results indicate that the hTfR_000894_PEG11/36_K (Maleimide)-trastuzumab conjugate has brain migratory activity.

Fig. 3-1 is a photograph alternative to the drawing showing trastuzumab-hTfR_000894_PEG11/36_K (Maleimide) (Conjugate No. 1/2) cerebellum 6 hours. In FIG. 3-1,
a-1 indicates trastuzumab only,
b-1 indicates trastuzumab-hTfR_000894_PEG11_K (Maleimide), and
c-1 indicates trastuzumab-hTfR_000894_PEG36_K (Maleimide). The lower figure is a partially enlarged view of the upper figure.

Result 2: The immunostaining results in the cerebellum 6 hours after treatment with nivolumab-894_3m_G4S2_K (Mal) (Conjugate No. 11) are shown in Figure 3-2. The lower figure is an enlargement of rectangle part in the upper figure. These results indicate that the nivolumab-894_3m_G4S2_K(Mal) conjugate has brain migratory activity.

Fig. 3-2 is a photograph alternative to the drawing showing nivolumab-894_3m_G4S2_K (Mal) (Conjugate No. 11) cerebellum 6 hours. In FIG. 3-2,
a-2 indicates nivolumab only, and
b-2 indicates nivolumab-894_3m_G4S2_K (Mal). The lower figure is a partially enlarged view of the upper figure.

Result 3: The immunostaining result in the cerebellum 6 hours after treatment with nivolumab-894_3m_GGRGRS_K (Mal) (Conjugate No. 43) is shown in Figure 3-3, and 24 hours after treatment is shown in Figure 3-4. The lower figure is an enlargement of rectangle part in the upper figure. These results indicate that the nivolumab-894_3m_GGRGRS_K (Mal) conjugate has brain migratory activity.

Fig. 3-3 is a photograph alternative to the drawing showing nivolumab-894_3m_GGRGRS_K (Mal) (Conjugate No. 43) cerebellum 6 hours. In FIG. 3-3,
a-3 indicates nivolumab only, and
b-2 indicates nivolumab-894_3m_GGRGRS_K (Mal). The lower figure is a partially enlarged view of the upper figure.

Fig. 3-4 is a photograph alternative to the drawing showing nivolumab-894_3m_GGRGRS_K (Mal) (Conjugate No. 43) cerebellum 24 hours. In FIG. 3-4,
a-4 indicates nivolumab only, and
b-4 indicates nivolumab-894_3m_GGRGRS_K (Mal). The lower figure is a partially enlarged view of the upper figure.

Result 4: The immunostaining result in the hippocampus 6 hours after treatment with nivolumab-894_3m_GGRGRS_K (Mal) (Conjugate No. 43) is shown in Figure 3-5, and 24 hours after treatment is shown in Figure 3-6. The lower figure is an enlargement of rectangle part in the upper figure. These results indicate that the nivolumab-894_3m_GGRGRS_K (Mal) conjugate has migratory ability also in the hippocampus.

Fig. 3-5 is a photograph alternative to the drawing showing nivolumab-894_3m_GGRGRS_K (Mal) (Conjugate No. 43) hippocampus 6 hours. In FIG. 3-5,
a-5 indicates nivolumab only, and
b-5 indicates nivolumab-894_3m_GGRGRS_K (Mal). The lower figure is a partially enlarged view of the upper figure.

Fig. 3-6 is a photograph alternative to the drawing showing nivolumab-894_3m_GGRGRS_K (Mal) (Conjugate No. 43) hippocampus 24 hours. In FIG. 3-6,
a-6 indicates nivolumab only, and
b-6 indicates nivolumab-894_3m_GGRGRS_K (Mal). The lower figure is a partially enlarged view of the upper figure.

## Claims

1. A conjugate comprising:
(1) a peptide that binds to a transferrin receptor, wherein the peptide is:
(i) a peptide comprising 1st to 15th amino acid sequence (Ala-Val-Phe-Val-Trp-Asn-Tyr-Tyr-Ile-Ile-Arg-Arg-Tyr-MeY-Cys) of an amino acid sequence described in SEQ ID NO: 1;
(ii) a peptide comprising an amino acid sequence having substitution, deletion, addition and/or insertion of 1 to 11 amino acid residues in the 1st to 15th amino acid sequence of the amino acid sequence described in SEQ ID NO: 1;
(iii) a peptide comprising 1st to 12th amino acid sequence (Ala-Val-Phe-Val-Trp-Asn-Tyr-Tyr-Ile-Ile-Ser-Cys) of an amino acid sequence described in SEQ ID NO: 14; or
(iv) a peptide comprising an amino acid sequence having substitution, deletion, addition and/or insertion of 1 to 8 amino acid residues in the 1st to 10th amino acid sequence of the amino acid sequence described in SEQ ID NO: 14,
and
(2) a compound comprising an antibody or an antigen-binding fragment thereof.

2. The conjugate according to claim 1, wherein the antibody or the antigen-binding fragment thereof is IgG or an IgG-derived antigen-binding fragment.

3. The conjugate according to claim 2, wherein the antibody or the antigen-binding fragment thereof is an antibody or an antigen-binding fragment thereof selected from a group consisting of IgG1, IgG2 and IgG4.

4. The conjugate according to claim 1, wherein the peptide is a peptide comprising an amino acid sequence containing one or more substitutions selected from:
(I) substitution of the 1st alanine residue of SEQ ID NO: 1 for an aliphatic amino acid or a methylated aliphatic amino acid;
(II) substitution of the 2nd amino acid residue of SEQ ID NO: 1 for any amino acid residue or any N-methylamino acid;
(III) substitution of the 3rd amino acid residue of SEQ ID NO:1 for an aromatic amino acid residue, a methylated aromatic amino acid residue, or an amino acid residue having an aromatic ring in a side chain;
(IV) substitution of the 5th amino acid residue of SEQ ID NO:1 for an aromatic amino acid residue, a methylated aromatic amino acid residue, or an amino acid residue having an aromatic ring in a side chain;
(V) substitution of the 6th asparagine residue of SEQ ID NO:1 for a hydrophilic amino acid or alanine;
(VI) substitution of the 8th tyrosine residue of SEQ ID NO:1 for an aromatic amino acid residue, a methylated aromatic amino acid residue, or an amino acid residue having an aromatic ring in a side chain;
(VII) substitution of the 10th isoleucine residue of SEQ ID NO:1 for any amino acid;
(VIII) substitution of the 11th arginine residue of SEQ ID NO:1 for any amino acid;
(IX) substitution of the 12th arginine residue of SEQ ID NO:1 for any amino acid; and
(X) substitution of the 13th tyrosine residue of SEQ ID NO:1 for any amino acid;
(XI) substitution of the 14th N-methyltyrosine residue of SEQ ID NO:1 for any amino acid;
or a peptide containing one or more substitutions selected from:
(I) substitution of the 1st alanine residue of SEQ ID NO: 14 for an aliphatic amino acid or a methylated aliphatic amino acid;
(II) substitution of the 2nd amino acid residue of SEQ ID NO: 14 for any amino acid residue or any N-methylamino acid;
(III) substitution of the 3rd amino acid residue of SEQ ID NO: 14 for an aromatic amino acid residue, a methylated aromatic amino acid residue, or an amino acid residue having an aromatic ring in a side chain;
(IV) substitution of the 5th amino acid residue of SEQ ID NO: 14 for an aromatic amino acid residue, a methylated aromatic amino acid residue, or an amino acid residue having an aromatic ring in a side chain;
(V) substitution of the 6th asparagine residue of SEQ ID NO: 14 for a hydrophilic amino acid or alanine;
(VI) substitution of the 8th tyrosine residue of SEQ ID NO: 14 for an aromatic amino acid residue, a methylated aromatic amino acid residue, or an amino acid residue having an aromatic ring in a side chain;
(VII) substitution of the 10th isoleucine residue of SEQ ID NO: 14 for any amino acid; and
(X) substitution of the 11th serine residue of SEQ ID NO: 14 for a hydrophilic amino acid residue.

5. The conjugate according to claim 1, wherein the peptide is a cyclic peptide consisting of any one of the amino acid sequences from the 1st to 15th amino acid sequence of SEQ ID NOs: 1 to 13, 15, 18 to 86, 90 to 110, and from the 1st to 12th amino acid sequence of SEQ ID NOs: 14, 16, 17, 87 to 89.

6. The conjugate according to claim 1, wherein the peptide is bound to the antibody via a linker.

7. The conjugate according to claim 6, wherein the linker is a peptide linker, a chemical linker, or a combination thereof.

8. The conjugate according to claim 6, wherein the linker is a linker comprising a sequence described in any one of SEQ ID NOs: 111 to 161.

9. The conjugate according to claim 6, wherein the peptide is bound to the antibody via a maleimide, hydrazide or NHS part attached to the end of the linker.

10. The conjugate according to claim 1 in which a linker-attached peptide of any one of SEQ ID NOs: 1 to 110 is bound to an antibody or antigen-binding fragment thereof.

11. A composition, containing the conjugate according to any one of claims 1 to 10, for delivering the conjugate into a cell or passing through the blood-brain barrier.

12. A pharmaceutical composition containing the conjugate according to any one of claims 1 to 10 or the salt thereof as an active ingredient.

13. A processing method that allows an antibody or an antigen-binding fragment thereof to be delivered into a cell or to passing through a blood-brain barrier, wherein the method includes a step of conjugating a peptide with the antibody or the antigen-binding fragment thereof, wherein the peptide that binds to a transferrin receptor and the peptide is:
(i) a peptide comprising 1st to 15th amino acid sequence (Ala-Val-Phe-Val-Trp-Asn-Tyr-Tyr-Ile-Ile-Arg-Arg-Tyr-MeY-Cys) of an amino acid sequence described in SEQ ID NO: 1;
(ii) a peptide comprising an amino acid sequence having substitution, deletion, addition and/or insertion of 1 to 11 amino acid residues in the 1st to 15th amino acid sequence of the amino acid sequence described in SEQ ID NO: 1;
(iii) a peptide comprising 1st to 12th amino acid sequence (Ala-Val-Phe-Val-Trp-Asn-Tyr-Tyr-Ile-Ile-Ser-Cys) of an amino acid sequence described in SEQ ID NO: 14; or
(iv) a peptide comprising an amino acid sequence having substitution, deletion, addition and/or insertion of 1 to 8 amino acid residues in the 1st to 10th amino acid sequence of the amino acid sequence described in SEQ ID NO: 14.

14. A method of conjugating the peptide according to claim 1 with an antibody or an antigen-binding fragment thereof, wherein the method comprising the steps of:
(i) reducing disulfide bond possessed by the compound with the antibody or the antigen-binding fragment thereof;
(ii) preparing the peptide to which a linker having a maleimide at its terminal is added; and
(iii) contacting the compound with the antibody or the antigen-binding fragment thereof, in which the disulfide bond is reduced in (i), with the peptide prepared in (ii).
